# EUROPEAN PATENT APPLICATION

(11) **EP 4 729 070 A1**
(43) Date of publication of application: **22.04.2026**
(21) Application number: 24222163.8
(22) Date of filing: 20.12.2024
(51) Int. Cl.: A61K 40/11, A61K 40/30, A61K 40/31, A61K 40/42, C12N 9/22, C12N 15/10, C12N 15/113

(54) **METHOD OF GENE EDITING USING BASE EDITORS FOR TRANSGENE INSERTION AND MULTIPLEX GENE EDITING**

(30) Priority: 17.10.2024 EP 24207313
(71) Applicant: Charité - Universitätsmedizin Berlin Körperschaft des öffentlichen Rechts, 10117 Berlin (DE)
(72) Inventor: WAGNER, Dimitrios Laurin, Houston, 77030 (US); KATH, Jonas Christian, 10435 Berlin (DE); GLASER, Viktor, 10115 Berlin (DE)
(74) Representative: Hertin und Partner Rechts- und Patentanwälte PartG mbB

(57) **Abstract**

The invention relates to an *in vitro* method for modifying double stranded DNA (dsDNA) in a eukaryotic cell. The method comprises: a) introducing a base editor system into the cell, said system comprising i) a base editor, comprising an RNA guided DNA nickase linked to a single-stranded DNA nucleobase modifying enzyme, or a nucleic acid encoding said base editor, and ii) at least two guide RNA (sgRNA) molecules, comprising a first sgRNA that hybridizes to a first target sequence in the dsDNA, and a second sgRNA that hybridizes to a second target sequence in an opposing strand of the dsDNA, b) generating at least two single-strand breaks (SSBs), in opposing strands, of the dsDNA, wherein a first base editor complex, comprising the first sgRNA, introduces a nick cleavage of one strand of the dsDNA in the first target sequence, and a second base editor complex, comprising the second sgRNA, introduces a nick cleavage of the opposite strand of the dsDNA in the second target sequence, thereby producing a 5' or 3' overhang, c) inserting an exogenous DNA repair template sequence in proximity to the two single strand breaks (SSBs). The invention further relates to a genetically modified eukaryotic cell obtained by the method according to the present invention. The invention also relates to a kit for use in the *in vitro* method.

## Description

The invention relates to the field of molecular and cell biology, in particular gene editing and the production and use of therapeutic biological cells, such as cellular immunotherapies.

The invention relates to an *in vitro* method for modifying double stranded DNA (dsDNA) in a eukaryotic cell, the method comprising: a) introducing a base editor system into the cell, said system comprising i) a base editor, comprising an RNA guided DNA nickase linked to a single-stranded DNA nucleobase modifying enzyme, or a nucleic acid encoding said base editor, and ii) at least two guide RNA (sgRNA) molecules, comprising a first sgRNA that hybridizes to a first target sequence in the dsDNA, and a second sgRNA that hybridizes to a second target sequence in an opposing strand of the dsDNA, b) generating at least two single-strand breaks (SSBs), in opposing strands, of the dsDNA, wherein a first base editor complex, comprising the first sgRNA, introduces a nick cleavage of one strand of the dsDNA in the first target sequence, and a second base editor complex, comprising the second sgRNA, introduces a nick cleavage of the opposite strand of the dsDNA in the second target sequence, thereby producing a 5' or 3' overhang, c) inserting an exogenous DNA repair template sequence in proximity to the two single strand breaks (SSB).

The invention further relates to a genetically modified eukaryotic cell obtained by the method according to the present invention.

The invention also relates to a kit for use in the *in vitro* method for modifying double stranded DNA (dsDNA) according to the present invention, comprising a) a base editor, comprising an RNA guided DNA nickase linked to a single-stranded DNA nucleobase modifying enzyme, or a nucleic acid encoding said base editor, and at least two guide RNA (sgRNA) sequences, comprising a first sgRNA that hybridizes to a first target sequence in the dsDNA, and a second sgRNA that hybridizes to a second target sequence in the dsDNA, wherein a first base editor complex, comprising the first sgRNA, is configured to introduce a nick cleavage of one strand of the dsDNA in the first target sequence, and a second base editor complex, comprising the second sgRNA, is configured to introduce a nick cleavage of the opposite strand of the dsDNA in the second target sequence, thereby producing a 5' or 3' overhang, and b) an exogenous DNA repair template that hybridizes to the dsDNA in proximity to one or both single strand breaks (SSB), and c) optionally one or more DNA repair modulators.

### BACKGROUND OF THE INVENTION

Immunotherapies hold significant promise as innovative treatment strategies for cancer and other immune-mediated diseases. Among these, chimeric antigen receptor (CAR) T cell therapies have demonstrated efficacy, particularly in treating hematological malignancies. Since the first CAR T cell therapy was approved in Europe in 2018, six CAR T cell products have received marketing authorization. These approved therapies are autologous, meaning that T cells are collected from the patient's blood, and a new DNA construct encoding a CAR is introduced into these cells ex *vivo.* This receptor enables the modified T cells to recognize and eliminate cancer cells more effectively.

In these approved CAR T cell therapies, gene transfer is performed using viral vectors, which randomly integrate the CAR-encoding DNA into the T cell genome. However, this random integration poses a risk of insertional mutagenesis, potentially leading to the transformation of T cells into cancerous cells. For example, 22 cases of T cell lymphoma were reported following the administration of virally produced CAR T cells. It is important to note that this is a rare side effect, given that over 27,000 patients in the United States have undergone CAR T cell therapy (Verdun and Marks, 2024). Additionally, unintended viral gene transfer into malignant blood cancer cells, rather than T cells, can result in treatment failure (Ruella et al., 2018). Beyond these risks, the clinical application of therapies using retroviral or lentiviral vectors in GMP (Good Manufacturing Practice) quality is associated with substantial cost and complicated manufacturing.

Non-viral gene editing technologies present a promising solution to improve both the safety and cost-effectiveness of CAR T cell therapies. One such alternative is the CRISPR-Cas system, which allows precise gene editing using a simple single guide RNA (sgRNA) to direct the Cas nuclease to a specific DNA sequence within the genome. Upon introducing a double-strand break (DSB) in the DNA, the cell's natural repair mechanisms, such as homology-directed repair (HDR), can be harnessed to integrate new genetic material, such as a CAR transgene. This targeted approach eliminates the need for exogenous promoters, which carry the risk of gene dysregulation and mutagenesis. The precise targeting of defined DNA sequences by CRISPR-Cas provides a significant safety advantage over random viral integration, as off-target effects can be predicted and minimized.

The recent approval of the CRISPR-Cas-based therapy "Exa-cel" for treating sickle cell disease and beta-thalassemia marks a critical milestone, demonstrating CRISPR-Cas's potential not only for gene transfer but also for targeted gene knockout, such as the disruption of genes or regulatory elements. While CRISPR-Cas has already entered clinical practice, the next generation of gene editing technologies is rapidly advancing. Among these, "base editing" allows for the precise modification of individual nucleotide bases within a defined DNA region. Base editing employs deaminase enzymes guided by a modified Cas enzyme to the target site in the genome. Unlike traditional CRISPR-Cas, the Cas enzyme in base editing, known as "Cas-nickase," induces a single-strand break ("nick") instead of a double-strand break, reducing the risks associated with DSB-induced toxicity.

While CAR T cell therapies have demonstrated significant success in treating various blood cancers, their application in other disease areas is still in the early stages. However, there are promising avenues for utilizing gene-edited T cells in the treatment of autoimmune diseases, solid tumors, and organ transplantation. The complexity of these conditions often necessitates multiple genetic modifications within the same T cells. For example, combining viral gene transfer of a CAR with multiple gene knockouts (KOs) can produce T cells that are resistant to lymph-depleting antibodies and immune checkpoints, enabling their use against T cell lymphomas (Georgiadis et al., 2021; Diorio et al., 2022).

In summary, current CAR T cell therapies face several challenges, including the risk of genotoxicity from the random integration of genetic material using viral vectors, which can lead to mutations and the transformation of T cells into cancerous cells. High production costs associated with generating viral vectors in GMP quality and the complexity and expense of performing multiple genetic modifications further complicate these therapies. Additional concerns include the risk of off-target effects, the use of exogenous promoters with mutagenic potential, and the possibility of treatment failure due to unintended gene transfer into non-target cells. While CAR T cells have proven effective in treating blood cancers, their application to autoimmune diseases and solid tumors remains underdeveloped.

Thus, there is an unmet need for developing efficient non-viral gene-editing strategies that are safe and cost-effective, while minimizing genotoxicity and avoiding the use of viral vectors.

### SUMMARY OF THE INVENTION

In light of the prior art, the technical problem underlying the invention was providing alternative or improved *in vitro* means for modifying the genome of a eukaryotic cell, e.g., for the development of CAR T cells. The present invention seeks to provide such means while avoiding the disadvantages known in the prior art.

A further objective of the invention was to develop alternative or improved means for minimizing genotoxicity, particularly to mitigate the risks associated with double-strand breaks and random integration of genetic material.

Another objective of the invention was to develop alternative or improved strategies for non-viral gene editing in therapeutic cells.

A further objective of the invention was to enable complex genetic modifications, including the ability to perform simultaneous genetic alterations in therapeutic cells, which are employed in developing therapies targeting complex diseases such as autoimmune disorders, organ transplantation, and solid tumors.

A further objective of the invention was to enhance the means of production of therapeutic cells, by providing alternative or improved strategies for safer, more cost-effective gene-editing methods, for making multiple genetic alterations to a therapeutic cell.

These problems are solved by the features of the independent claims. Preferred embodiments of the present invention are provided by the dependent claims.

The present invention, in one aspect, relates to an *in vitro* method for modifying double stranded DNA (dsDNA) in a eukaryotic cell, the method comprising:
a) introducing a base editor system into the cell, said system comprising
   a) a base editor, comprising an RNA guided DNA nickase linked to a single-stranded DNA nucleobase modifying enzyme, or a nucleic acid encoding said base editor, and
   b) at least two guide RNA (sgRNA) molecules, comprising a first sgRNA that hybridizes to a first target sequence in the dsDNA, and a second sgRNA that hybridizes to a second target sequence in an opposing strand of the dsDNA,
b) generating at least two single-strand breaks (SSBs), in opposing strands, of the dsDNA,
   a) wherein a first base editor complex, comprising the first sgRNA, introduces a nick cleavage of one strand of the dsDNA in the first target sequence, and a second base editor complex, comprising the second sgRNA, introduces a nick cleavage of the opposite strand of the dsDNA in the second target sequence, thereby producing a 5' or 3' overhang,
c) inserting an exogenous DNA repair template sequence in proximity to the two single strand breaks (SSBs).

The present invention is based on the surprising finding that base editing enzymes may facilitate both transgene knock-in and multiplex gene editing within a single transfection process, achieving efficiencies comparable to or exceeding those of conventional CRISPR-Cas9 methods.

A notable aspect of the invention is its reliance on a non-viral strategy for gene editing. By eliminating the need for viral vectors, this approach significantly reduces genotoxicity by inducing single-strand breaks rather than double-strand breaks, thereby minimizing genomic instability. Additionally, avoiding viral vectors mitigates risks such as insertional mutagenesis, chromosomal translocations, and the costs and risks associated with viral vector production and use.

One aspect of the present invention that represents significant progress over the prior art lies in the strategic combination of base editing with homology-directed repair, which allows for precise and efficient gene editing without the use of viral delivery systems. This approach offers a safer, cost-effective, and highly efficient alternative to traditional gene editing technologies, marking a significant advancement in the field of genetic engineering.

Using viral vectors for gene transfer can result in the random integration of genetic material into the genome, posing a risk of insertional mutagenesis. This random integration can potentially lead to the transformation of T cells into cancerous cells, as evidenced by documented cases of patients developing T cell lymphomas after receiving CAR T cell therapy.

In contrast, base editing presents several advantages over conventional genome editing techniques. Base editors do not induce double-strand breaks, which significantly reduces the risk of generating off-target indel that can occur during the error-prone non-homologous end-joining (NHEJ) repair process. Additionally, base editors function independently of the cell cycle, making them effective in non-dividing cells, which represents a notable limitation of methods that rely on homology-directed repair (HDR).

In embodiments, the method of the present invention comprises generating at least two single-strand breaks (SSBs) in opposing strands of the dsDNA.

In embodiments, the method of the present invention comprises generating at least two single-strand breaks (SSBs) in opposing strands of the dsDNA, wherein a first base editor complex, comprising the first sgRNA, introduces a nick cleavage of one strand of the dsDNA in the first target sequence and a second base editor complex, comprising the second sgRNA, introduces a nick cleavage of the opposite strand of the dsDNA in the second target sequence, thereby producing a 5' or 3' overhang.

In the context of the present invention, the term "overhang" refers to the single-stranded DNA portion that would be produced if the double-stranded DNA fragments, targeted for modification, were separated following the creation of two single-strand breaks. Each resulting dsDNA fragment would possess an overhang, the length of which corresponds to a spacer region between the two SSBs.

The nature of the overhang, whether a 3' or 5' on each end of the resulting dsDNA fragment, depends on the positioning of the guide RNAs responsible for directing the guide RNA/DNA nickase complex to the respective strands. Specifically, the relative alignment of the guide RNAs determines whether the breaks occur so that a 3' or 5' overhang is produced, thereby influencing the structural configuration of the modified DNA fragment and facilitating homology-directed repair.

In embodiments, the method of the invention induces fewer unwanted on- and/or off-target effects, such as micro-insertions and/or deletions (Indels) and/or off-target mutations.

On- and off-target effects can be measured by sequencing-based techniques with subsequent sequence analysis and comparison, as known to a person skilled in the art. For example, on-/off-target effects can be determined by techniques like GUIDE-seq, CAST-Seq or LAM-HTGTS.

On-target mutations (in particular INDELs) are determined by PCR, following PCR amplicons sequenced by Sanger or next-generation sequencing. Frequencies of on-target mutations are analyzed and compared with reference sequences (wild-type). Genome-wide off-target effects induced by CRISPR/Cas are determined by deep sequencing techniques such as GUIDE-seq, CAST-Seq, AAV-seq, and LAM-HTGTS. Quantification of known potential on- and/or off-target effects such as chromosomal translocations can furthermore be quantified by droplet digital PCR (ddPCR).

It is a surprising finding that the method described herein significantly reduces the unwanted on- and off-target gene editing outcomes in the genomic DNA. This surprising finding renders the method of the invention a robust and reliable method for gene correction.

Surprisingly, high-fidelity gene editing in eukaryotic cells has been demonstrated to be effective through single-strand break-mediated homology-directed repair. This method involves the introduction of base editor system comprising a guide RNA-guided DNA nickase linked to a single-stranded DNA nucleobase modifying enzyme into the target cell, in conjunction with at least two guide RNAs and an exogenous DNA donor repair template. The process results in the creation of at least two single-strand breaks within the double-stranded DNA, forming either 5' or 3' overhangs in the region designated for modification. These single-strand breaks, strategically positioned by the base editor complex, promote the precise incorporation of the exogenous DNA template through homology-directed repair mechanisms, enabling highly accurate gene editing outcomes.

A key factor contributing to the high specificity of sequence replacement in the method disclosed by the present invention, with minimal off-target or on-target mutations, is the use of a base editor system. Unlike nucleases that generate double-strand breaks with blunt ends, nickases introduce a single-strand break (nick) in one strand of the double-stranded DNA. This controlled nicking reduces the likelihood of unintended genomic alterations and promotes more accurate homology-directed repair in the targeted region. By avoiding the more disruptive double-strand breaks, the method ensures enhanced precision in sequence modification, thereby minimizing genomic instability and off-target effects.

Preferably, the DNA nickase of the invention is a Cas9 nickase, such as a Cas9 nickase derived from Streptococcus pyogenes Cas9 (SpCas9).

Following the present invention, the design of the two guide RNAs is specifically configured to ensure that the guide RNA/DNA nickase complexes associated with each respective guide RNA are positioned on opposing strands of the double-stranded DNA targeted for modification. Specifically, the first guide base editor system introduces a nick on the plus strand, while the second guide base editor system introduces a nick on the minus strand. As a result, single-strand nicks are introduced on opposite strands of the dsDNA, leading to the generation of overhangs at each end of the resulting DNA fragment. These complementary overhangs facilitate precise and efficient sequence replacement through homology-directed repair, promoting targeted gene modification with high fidelity and minimal off-target effects.

In the context of the present invention, the different components of the method, namely a base editor comprising a guide RNA-guided DNA nickase or a nucleic acid molecule encoding a guide RNA-guided DNA nickase, a first guide RNA, a second guide RNA, and an exogenous DNA donor template (comprising a DNA template sequence), may be added sequentially to a cell or a cell culture comprising the cell, or at the same time, for example using a premixed stock solution comprising all or some of the components.

As used herein, it is understood that replacing a DNA sequence of the dsDNA, positioned in proximity to the two single-strand breaks (SSBs) means replacing a DNA sequence of the dsDNA positioned in proximity to, adjacent to, and/or between the two single-strand breaks (SSBs).

The sequence to be replaced in the context of the present invention can be located between the SSBs induced by the nickase, but it can also span the location of one or both SSBs and, therefore, extend beyond the spacer sequence. It is understood that a DNA sequence positioned in proximity to the SSBs comprises any sequence located between the two SSBs, including the entire spacer sequence, but can also be a sequence extending beyond the spacer on one or both sides.

It is understood that a sequence "adjacent" to an SSB is a sequence that starts with a nucleotide directly next to the SSB/cutting site of the nickase. For example, the spacer sequence located between the two SSBs is on each end "adjacent" to one of the SBBs.

However, a sequence that is "in proximity" to the SSBs can also comprise sequences whose distal ends start up to 1500 bp outside the spacer sequence, meaning that the distal end of the sequence (as regarded from the next SSB induced by the nickase in the context of the method of the invention) starts in a distance of 1500 bp from the SSB. Accordingly, in embodiments, the sequence to be replaced can span the spacer sequence and up to 1500 bp outside from each of the two SBBs. Preferably, a sequence that is "in proximity" to the SSBs can also comprise sequences whose distal ends start about 1400, 1300, 1200, 1100, 1000, 950, 900, 850, 800, 750, 700, 650, 600, 550, 500, 450, 400, 350, 300, 250, 200, 180, 150, 100, 50, 40, 30, 20, 10, 5, 4, 3, 2, 1, 0 (= adjacent to the SSB) bp outside the spacer sequence. Furthermore, sequences that are in proximity to the SSBs comprise all sequences that consist of or are comprised by the spacer region.

In embodiments, the exogenous DNA repair template sequence encodes a recombinant antigen-specific targeting construct, such as a chimeric antigen receptor (CAR), a T cell receptor (TCR), or a construct configured to enhance the persistence and/or potency of cells for therapeutic effect.

In one embodiment, the exogenous DNA repair template sequence is a homology-directed repair (HDR) donor template sequence.

In embodiments, the exogenous DNA repair template sequence encodes a chimeric antigen receptor (CAR), preferably configured for expression in a CAR T cell or a CAR NK cell.

In embodiments, the exogenous DNA repair template sequence encodes a T cell receptor.

In embodiments, the exogenous DNA repair template sequence encodes a construct configured to enhance the persistence and/or potency of a cell for a therapeutic effect.

In embodiments, the exogenous DNA repair template sequence encodes an HLA class I histocompatibility antigen, alpha chain E (HLA-E). In embodiments, the exogenous DNA repair template sequence encodes an HLA class I histocompatibility antigen, alpha chain E (HLA-E) to protect a cell from allogeneic NK cell lysis.

In embodiments, the exogenous DNA repair template sequence encodes a cytokine. In embodiments, the exogenous DNA repair template sequence encodes a cytokine for improved antitumor therapy.

Advantageously, the *in vitro* method of the present invention allows for precise targeting and/or insertion of therapeutic constructs such as CARs, T cell receptors, or other constructs configured to enhance the persistence and/or potency of cells, enhancing the specificity and efficacy of the gene-editing process, e.g., compared to viral methods.

In embodiments, the eukaryotic cell is an effector cell, such as a cytotoxic immune effector cell, preferably a T cell or NK cell. In embodiments, the eukaryotic cell is an effector cell, such as a cytotoxic immune effector cell, preferably a Treg cell.

In another embodiment, the eukaryotic effector cell is an allogeneic eukaryotic cell with respect to a patient. In one embodiment, the effector cell is an allogeneic effector cell with respect to a patient. In one embodiment, the cytotoxic immune effector cell is an allogeneic cytotoxic immune effector cell with respect to a patient. In one embodiment, the immune effector cell is an allogeneic T cell or an allogeneic NK cell with respect to a patient.

In another embodiment, the eukaryotic effector cell is an autologous eukaryotic cell with respect to a patient. In one embodiment, the effector cell is an autologous effector cell with respect to a patient. In one embodiment, the cytotoxic immune effector cell is an autologous cytotoxic immune effector cell with respect to a patient. In one embodiment, the immune effector cell is an autologous T cell or an autologous NK cell with respect to a patient.

In some embodiments, cells can be obtained from a subject different from the intended patient (allogeneic cells). As used herein, a cell is "allogeneic" with respect to a subject if it or one of its progenitor cells is derived from another subject of the same species. As used herein, a cell is "autologous" with respect to a subject if it or its progenitor cells originate from the same subject. The method disclosed in the present invention is advantageously applicable to both autologous and allogeneic cells. Notably, the use of allogeneic cells presents a particularly advantageous approach due to their potential for developing "off-the-sheif" therapeutic products, such as generating CAR T cells. In this context, allogeneic cells offer distinct advantages, including the ability to facilitate large-scale production and pre-emptive storage for immediate clinical deployment.

For the safety of allogeneic therapies, the knockout of the endogenous T cell receptor (TCR) is critical, as it mitigates the risk of graft-versus-host disease (GvHD). GvHD arises when donor-derived CAR-modified T cells, through their endogenous TCR, recognize and attack the recipient's tissues as foreign. The knockout of the endogenous TCR in the allogeneic T cells substantially reduces the risk of GvHD, enhancing the safety profile of such therapies.

The provision of allogeneic cells, as described in the present invention, enables a more rapid and efficient therapeutic response. This is because allogeneic cells can be sourced and prepared in advance, allowing for quicker availability compared to autologous cells, which must be harvested from the patient. Medical pre-treatments of the patients can result in manufacturing failure or cause autologous cells to be functionally limited. Therefore, selection of allogeneic donor cells with an ideal cellular phenotype can enable optimal performance of the therapy. Allogeneic cells can also be engineered to target various tumor-associated antigens, thus offering greater versatility in treating different types of cancer. Furthermore, manufacturing and storing allogeneic CAR T cells for future use enhances the scalability and accessibility of CAR T cell therapies, potentially broadening their clinical application and making them more readily available to a broader patient population.

In embodiments, the eukaryotic cell is a T or NK cell and the first and second target sequences of the dsDNA are in an endogenous genomic dsDNA encoding a T cell receptor component, such as in the *TRAC* locus, *CD3ζ* locus or *CD3ε* locus, or a major histocompatibility complex component, such as in the *B2M* locus.

In one embodiment, the exogenous DNA sequence integrated into the *TRAC* locus encodes a chimeric antigen receptor (CAR), resulting in a disruption of a T cell receptor (TCR)/CD3 complex expression and integration of the CAR-encoding sequence.

In one embodiment, the exogenous DNA sequence integrated into the *CD3ζ* locus encodes a truncated chimeric antigen receptor (truncCAR), resulting in disruption of the *CD3ζ* component of the T cell receptor (TCR)/CD3 complex expression, and integration of the truncated chimeric antigen receptor (truncCAR).

In embodiments, the truncated CAR construct is a CD3 zeta-deficient chimeric antigen receptor (CAR) fragment (transgene) that is then integrated into an endogenous CD3 zeta/CD247 gene for generating a gene fusion and therefore a functional CAR comprising an exogenous CAR fragment fused with an endogenous CD3 zeta domain. The technology employing integrating CD3 zeta deficient CAR constructs into endogenous CD3 zeta genes is described in detail in WO2022/136551.

In embodiments, the truncated CAR construct comprises three nucleic acid sequence regions, wherein a first nucleic acid sequence region encodes a CD30 chimeric antigen receptor (CAR) fragment without a sequence encoding a functional intracellular domain of a CD3 zeta protein, a second nucleic acid sequence region comprises a targeting sequence configured for integrating the construct at an endogenous CD3 zeta gene of a human cell, and a third nucleic acid sequence region encoding and or comprising a protein separation site upstream of the first sequence region.

In embodiments, the targeting sequence of the second nucleic acid sequence region is configured for integrating the construct in-frame with an endogenous CD3 zeta encoding sequence, preferably into an exon of an endogenous CD3 zeta gene. In embodiments, the protein separation site of the third nucleic acid sequence encodes a first self-cleavage peptide, protease cleavage site or an internal ribosomal entry site (IRES).

In embodiments, the target site of the CD3 zeta gene, into which the first sequence region is integrated, is an exon and/or an intron, preferably exon 2, and is positioned upstream of an endogenous CD3 zeta sequence encoding an intracellular domain or fragment thereof, said intracellular domain or fragment thereof comprising one or more immunoreceptor tyrosine-based activation motifs (ITAMs).

In embodiments, the CAR, when integrated as a truncated CAR into the CD3 zeta locus, as provided by the invention, can be expressed under the control of the endogenous CD3 zeta promoter.

In one embodiment, the exogenous DNA sequence integrated into the *CD3ε* locus encodes a chimeric antigen receptor (CAR) or other therapeutic construct, resulting in the disruption of the CD3ε component of the T cell receptor (TCR)/CD3 complex expression, and integration of the CAR-encoding sequence.

In one embodiment, the DNA template for CD3e encodes an antigen binding domain (such as an antibody fragment - scFv) resulting in the incorporation of the CD3e-binding domain into the TCR. Thus, the CD3e component and TCR are not disrupted.

In one embodiment, the exogenous DNA sequence integrated into the *B2M* locus encodes an HLA-E fusion protein, resulting in the disruption of the major histocompatibility complex (MHC) class I molecule expression.

The method of the present invention advantageously provides a targeted approach by directing gene editing to specific loci within the genome. This precise targeting results in more consistent expression and stable functionality of the inserted genes, in contrast to random integration methods, which can lead to variable gene expression and unpredictable outcomes. By ensuring that transgenes are integrated at predetermined sites, the invention enhances control over gene expression and reduces the risk of insertional mutagenesis. Furthermore, targeted insertion into an open reading frame (ORF) eliminates the need for a promoter, further enhancing the approach's safety.

In embodiments, the exogenous DNA repair template sequence comprises at least one sequence (homology arm) of at least 45 bp, preferably 400 bp, with at least 90%, preferably 95%, more preferably 100%, sequence identity to a region of the dsDNA sequence, wherein said homology arm is positioned to hybridize to the dsDNA in proximity to the two single strand breaks (SSBs).

In embodiments, the sequence to be replaced is defined by the design of the DNA template sequence. To induce HDR mediated sequence replacement, the DNA template sequence requires at least one homology arm, which is understood to be a sequence of at least 45, 50, 100, 150, 200, 250, 300, 350, and 400 bp with sequence identity to a region within the DNA sequence sufficient to mediated HDR, such as as at least 90%, preferably 95%, 96%, 97%, 98%, 99%, most preferably 100% sequence identity to a region within the DNA sequence to be replaced. In embodiments, the homology arm has a sequence identity sufficient to enable the hybridization of the homology arm to the corresponding region of the sequence to be replaced. Preferably, the homology arms share this sequence identity with a region located at the end of the sequence to be replaced.

Preferably, the DNA template has two homology arms, each homologous/sufficiently identical to enable HDR-mediated sequence replacement. In embodiments, the DNA template sequence comprises a sequence unrelated to the replacement sequence located between (flanked by) two homology arms with sufficient sequence identity to a region of the sequence to be replaced to enable HDR-mediated sequence replacement.

As used herein, the terms sequence identity and sequence homology are used interchangeably. Herein, it is understood that a "homologous sequence" has a sufficient sequence identity to a region of the sequence to be replaced to enable replacement through HDR.

In some embodiment, the DNA template sequence comprises at least one sequence (homology arm) of at least 100 bp, preferably 500 bp, with at least 90%, preferably 95%, more preferably 100%, sequence identity to a region within the DNA sequence to be replaced, wherein said homology arm is positioned to hybridize to the dsDNA molecule to be modified in proximity to the two single-strand breaks (SSBs), preferably wherein the DNA template comprises at least two homology arms with at least 90%, preferably 95%, more preferably 100%, sequence identity to a region within the DNA sequence to be replaced.

It is a great advantage of the method of the invention that the DNA template sequence can be adjusted as necessary and suited, which can be individually judged by a skilled person depending on the DNA sequence to be replaced and its location within the dsDNA molecule to be modified. There is no strict rule with respect to the designs of the homology arms, which may enable hybridization of a region of the DNA sequence to be replaced, which can be located outside or inside the spacer region or which can comprise the location of one or both SSBs induced in the context of the present invention.

In some embodiments, the one or two homology arms are positioned to hybridize to the dsDNA molecule to be modified in proximity to, adjacent to, and/or between the two single-strand breaks (SSBs).

In embodiments, the homology arm includes regulatory elements. In embodiments, the homology arm includes regulatory elements to drive controlled expression of the integrated transgene.

In embodiments, the method comprises in step a) introducing one or more additional sgRNA sequences, comprising a third sgRNA that hybridizes to a third target sequence in the dsDNA and forming a third base editor complex that introduces a nick cleavage of one strand of the dsDNA and a nucleobase modification in the third target sequence.

In certain embodiments, the nucleobase modification introduced by the third base editor complex results in a precise alteration of the DNA sequence at the third target site, leading to functional alterations in the gene or regulatory element being modified.

In embodiments, the third sgRNA targets a splice site to modulate alternative splicing patterns and/or gene expression.

In embodiments, the third sgRNA results in a precise alteration of the DNA sequence at the third target site from encoding an amino acid to a stop codon to modulate gene expression.

In embodiments, the third sgRNA introduces a modification to confer resistance to viral infections in the edited cells.

An advantageous aspect of the present invention is using multiple sgRNA sequences to enable multiplex gene editing. This approach allows for the simultaneous modification of multiple genetic targets, significantly increasing efficiency compared to sequential editing methods. To the best of the inventors' knowledge, no prior study has demonstrated simultaneous editing, such as knock-in (KI) and knock-out (KO), using base editors within a single transfection event. This novel method simplifies the clinical development of T-cell products that require multiple genetic modifications. For example, a single base editor may be employed with multiple guide RNAs targeting different sites in the genome of the therapeutic cell to be modified, thereby enabling multiplex gene editing with a single base editor needing to be transformed into the cells.

The invention's development of a fully non-viral gene editing strategy for simultaneous knock-in (e.g., a chimeric antigen receptor, CAR) and knock-out of multiple genes is of particular importance. Previous work by the inventors (Glaser et al., 2023) demonstrated the use of different nucleases for knock-in and base editing to prevent chromosomal translocations. However, integrating viral transduction or using multiple nucleases in conjunction with base editing introduces considerable complexity and significantly increases the cost of GMP-grade reagents. While it is possible to clinically translate complex gene-editing strategies, as shown by Chiesa et al. (2023), reducing the complexity of multiplex gene-edited cell products would directly enhance their clinical feasibility and significantly lower associated costs. The present invention addresses this need by simplifying the process, thereby offering a more streamlined, cost-effective approach for clinical applications.

In embodiments, the third target sequence of the dsDNA is a component of a gene, such as a regulatory sequence, splice donor, splice acceptor, or other sequence associated with expression of said gene, and wherein said modification of the third target sequence leads to disruption of expression of said gene.

In embodiments, the third target sequence of the dsDNA is located in the open reading frame (ORF) of a gene associated with a functional domain of said gene and wherein said modification of the third target sequence leads to an amino acid change leading to a functional alteration of said gene.

In embodiments, the third target sequence of the dsDNA is located in the open reading frame (ORF) of a gene wherein said modification of the third target sequence leads to an amino acid change leading to alteration of an epitope. The edited epitope alters the interaction with antigen binding proteins compared to the antigen on non-edited cells. The altered epitope can mask an epitope that was previously detectable on non-edited cells or generate a novel binding site that can be specifically detected.

In embodiments, the expression of multiple genes is disrupted (multiple gene knockouts) with multiple additional sgRNA sequences, simultaneously with the insertion of an exogenous DNA repair template sequence.

In embodiments, the method comprises the use of adenine base editors and/or cytosine base editors to achieve targeted nucleobase modifications.

In embodiments, simultaneous knockout and/or knock-in involves the use of adenine base editors and/or cytosine base editors to achieve targeted nucleobase modifications.

In embodiments, the disruption of one or more genes involves the use of adenine base editors and/or cytosine base editors. In embodiments, the insertion of an exogenous DNA repair template sequence involves the use of adenine base editors and/or cytosine base editors. In embodiments, the disruption of one or more genes with simultaneous insertion of an exogenous DNA repair template sequence involves the use of adenine base editors and/or cytosine base editors.

In embodiments, the disruption of one or more genes is achieved through introducing adenine base editors and/or cytosine base editors into the cell. In embodiments, the insertion of an exogenous DNA repair template sequence is achieved is achieved through introducing adenine base editors and/or cytosine base editors into the cell. In embodiments, the disruption of one or more genes with simultaneous insertion of an exogenous DNA repair template sequence is achieved through introducing of adenine base editors and/or cytosine base editors into the cell.

In embodiments, the disruption of one or more genes using the methods of the present invention leads to lower levels of chromosomal translocation. In embodiments, the insertion of an exogenous DNA repair template sequence using the methods of the present invention leads to reduced chromosomal translocation. In embodiments, the disruption of one or more genes with simultaneous insertion of an exogenous DNA repair template sequence leads to lower levels of chromosomal translocation in comparison to using nuclease-mediated methods.

In embodiments, reduced translocations occur with the present invention in comparison to methods using nuclease-mediated gene editing. In general, knock-ins and multiplexed editing are possible with minimal translocations, when using the approach of the present invention.

In embodiments, one or more exogenous DNA repair template sequences are inserted into the cell.

In embodiments, one or more exogenous DNA repair template sequences are inserted into the cell in combination with multiple nucleobase modifications.

In embodiments, one sgRNA guides the base editing machinery to a specific locus for modification, and the second sgRNA directs the insertion of the exogenous template.

In embodiments, the method of the present invention further comprises the use of specific sgRNA combinations to optimize editing efficiency and minimize off-target effects.

Selecting appropriate sgRNAs is crucial for creating optimal conditions for intended genetic modifications, such as knock-ins (Kls) or knock-outs (KOs). The choice of sgRNAs is informed by the necessity of positioning the editing window effectively, ensuring that the targeted bases are accessible for efficient modification by the base editors. Specifically, minimizing certain nucleobases within the editing window enhances editing efficiency. For instance, reducing adenine bases for Adenine Base Editors (ABEs) or cytosine bases for Cytosine Base Editors (CBEs) can lead to more precise genetic edits. Furthermore, the spacing between DNA nicks created by different sgRNAs is a critical factor that influences the success of the editing process. Maintaining an optimal distance between these nicks facilitates favorable conditions for homology-directed repair, the preferred pathway for the high-fidelity integration of exogenous DNA. The careful selection and combination of sgRNAs, therefore, not only enhance KI and KO efficiencies but also reduce off-target effects, resulting in a higher overall success rate in achieving the desired genetic modifications.

The method described in the present invention advantageously facilitates a wide array of genetic modifications within a single editing event. By enabling the disruption of gene expression, the introduction of functional alterations, and the simultaneous editing of multiple genes, this invention provides a versatile strategy for comprehensive gene manipulation.

The capacity for multiplex gene editing reduces the reliance on sequential editing processes, thereby enhancing overall efficiency. This simplification can result in shorter development timelines, reduced costs, and diminished risks of errors or unintended consequences during the editing process.

Moreover, the ability to execute multiplex editing, along with targeted gene disruption and functional modification, supports the advancement of innovative therapeutic strategies. For example, creating cell therapies that necessitate both the insertion of therapeutic genes and the knockout of inhibitory genes can significantly enhance treatment efficacy for diseases such as cancer and genetic disorders.

Overall, the present invention offers a flexible and adaptable approach that can be tailored to various cell types and genetic contexts. This adaptability is particularly advantageous for addressing a wide range of diseases and conditions, thereby broadening the applicability of the gene editing platform.

In embodiments, the gene is selected from *FKBP12, NR3C1, PPIA, CD38, CD45, CD52, DNMT3A, Regnase-1, NR4A1, NR4A2, NR4A3, RASA2, PIC3CD, PDCD1, CTLA4, CISH, PTPN2, KLRC1, SOCS1, TIM3, LAG3, CD5, CD7, CD30, SUV39H1, TGFBR2, CD3d, CD3e, CD3g, CD3z, TRBC1, TRBC2, 62M, CIITA, HLA-A, HLA-B, HLA-C, CD54, CD58, CD74, CD16,* or a non-coding RNA relevant to T cell function, such as a microRNA or a long non-coding RNA.

In embodiments, the target gene is a T cell receptor complex component.

In embodiments, the target gene is a co-receptor modulating T cell activation.

In embodiments, the target gene is in involved T cell activation.

In embodiments, the target gene is involved in T cell signaling.

In embodiments, the target gene is associated with the MHC class I complex. In embodiments, the target gene is an MHC class I molecule. In embodiments, the target gene presents antigens to cytotoxic T cells. In embodiments, the target gene is involved in antigen presentation and/or T cell-APC interactions.

In embodiments, the target gene is a cytokine.

In embodiments, the target gene is involved in intracellular signaling. In embodiments, the target gene is involved in protein folding.

In embodiments, the target gene is involved in immune regulation.

In embodiments, the single-stranded DNA nucleobase modifying enzyme is an adenosine deaminase, a cytidine deaminase, cytosine-DNA glycolase, thymidine-DNA glycolase, or fusion constructs containing deaminases and glycolases, such as a cytodine deaminase with a uracil glycolase.

In embodiments, the base editor is a Cytosine Base Editor (CBE) and/or an Adenine Base Editor (ABE) and/or a Glycosylase Base Editor (GBE).

In embodiments, the base editor is an Adenine Base Editor (ABE). In embodiments, the ABE substitutes adenine (A) nucleotides with guanine (G) nucleotides. In embodiments, the ABE substitutes adenine-threonine (A-T) base pairs with guanine-cytosine (G-C) base pairs.

In embodiments, the base editor is a Cytosine Base Editor (CBE). In embodiments, the CBE substitutes cytosine (C) nucleotides with threonine (T) nucleotides. In embodiments, the CBE substitutes cytosine-guanine (C-T) base pairs substitutes threonine-adenine (T-A) base pairs.

In embodiments, the base editor is a Glycosylate Base Editor (GBE). In embodiments, the GBE substitutes guanine (G) nucleotides with adenine (A) nucleotides and/or cytosine (C) nucleotides.

In one embodiment, the method of the present invention combines several base editors. In embodiments, the base editor is an Adenine Base Editor (ABE) combined with a Cytosine Base Editor (CBE). In embodiments, the base editor is an Adenine Base Editor (ABE) combined with a Glycosylase Base Editor (GBE). In embodiments, the base editor is a Cytosine Base Editor (CBE) combined with a Glycosylase Base Editor (GBE). In embodiments, the base editor is a Cytosine Base Editor (CBE) and an Adenine Base Editor (ABE) and a Glycosylase Base Editor (GBE).

In embodiments, the first and/or second sgRNAs, and optionally additional sgRNAs, are configured such that an editing window within the target DNA sequence is located within positions 3-15 of the 20 base pair protospacer region of the sgRNA.

In embodiments, the first and/or second sgRNAs, and optionally additional sgRNAs, are configured such that the specific target nucleobase within the target DNA sequence is located within the editing window at the positions 3, 4, 5, 6, 7, 8, 9, 10, 11, 12, 13, 14, 15 of the 20 base pair protospacer region of the sgRNA.

In embodiments, the editing window is located at positions 3 to 20, preferably 4 to 10, most preferably 4 to 8 of the 20 base pair protospacer region of the sgRNA.

In embodiments, the editing window overlaps with the target nucleobases within positions 3-15 of the 20 base pair protospacer region of the sgRNA.

In embodiments, the sgRNA configuration is adjusted based on the specific base composition (adenine or cytosine) within the editing window and available PAMs surrounding the target sequence.

In embodiments, the sgRNA combinations lack adenine bases in the editing window within the target DNA sequence when using adenine base editors. In embodiments, the editing window lacks adenine bases within the target DNA sequence when using adenine base editors.

In embodiments, the sgRNA combination comprises 1, 2, 3, 4, 5, 6, 7, 8 adenine bases in the editing window within the target DNA sequence when using adenine base editors.

In embodiments, the sgRNA combinations lack cytosine bases in the editing window within the target DNA sequence when using cytosine base editors. In embodiments, the editing window lacks cytosine bases within the target DNA sequence when using cytosine base editors.

In another embodiment, the editing window aligns with regulatory regions or functional domains of the target gene.

The present invention advantageously optimizes the design of the editing window to minimize bases that could lead to unintended modifications. For instance, reducing the number of adenines in the target region when employing an adenine base editor (ABE) significantly decreases on-target undesired A-to-G conversions, enhancing specificity and precision. Additionally, the size and placement of the editing window have been strategically optimized to achieve maximum editing efficiency. For example, without being bound by theory, certain sgRNA combinations that promote high knock-in (KI) efficiency exhibited specific characteristics, notably reducing adenines within the editing window when using ABE. Low levels of adenines yield efficient knock ins. For example, up to 4 adenines within the combined editing window of the 2 sgRNAs still yielded efficient KI. Additionally, sgRNAs lacking adenines in this region have demonstrated significantly higher KI frequencies, highlighting the critical role of precise editing window design in achieving optimal gene-editing outcomes.

A person skilled in the art is able to select an appropriate base editing system and the sgRNA to adjust the position of the editing window without undue effort.

In embodiments, the base editor is an ABE, and the first and/or second sgRNA lacks adenine bases in the editing window and/or wherein the base editor is a CBE, and the first and/or second sgRNA lacks cytosine bases in the editing window.

In embodiments, the base editor is an ABE, and the first and second sgRNA together have a total of less than 6 adenine bases in the editing window and/or wherein the base editor is a CBE, and the first and second sgRNA together have a total of less than 6 cytosine bases in the editing window.

In embodiments, the base editor is an ABE, and the first and second sgRNA together have a total of less than 6, 5, 4, 3, 2 adenine bases in the editing window.

In embodiments, the base editor is a CBE, and the first and second sgRNA together have a total of less than 6, 5, 4, 3, 2 cytosine bases in the editing window.

Surprisingly, the present invention demonstrates that minimizing the presence of specific target nucleobases within the editing window significantly enhances both the specificity and efficiency of the base editing process. It was unexpectedly found that a reduced concentration of adenine bases correlates with increased knock-in (KI) rates when employing adenine base editors (ABEs). Similarly, a decrease in cytosine bases leads to enhanced editing efficiency with cytosine base editors (CBEs). Thus, the advantage of the method disclosed in the present invention lies in its ability to achieve higher editing precision and efficiency by constraining the number of target nucleobases within the editing window.

This method significantly reduces the likelihood of unwanted on-target effects and unintended modifications by strategically configuring sgRNAs to limit the presence of adenines or cytosines in the editing window. This increased precision is critical for applications that necessitate high fidelity and safety in gene editing, particularly in therapeutic contexts.

This method significantly reduces the likelihood of off-target effects and unintended modifications by utilizing in-silico prediction of potential off target sites during the sgRNAs design process. Furthermore, off-target DSB are reduced when using base editors relying on SSBs compared to nuclease mediated KI strategies. This reduced the risk for genotoxic events, which is particularly crucial for therapeutic applications.

In embodiments, the method optionally comprises introducing to said cell one or more DNA repair modulators.

In embodiments, the method comprises introducing to said cell one or more HDR enhancers. Non-limiting examples of HDR enhancers include Wortmannin, SCR7, L755507, EPZ5676, Rucaparib, Pevonedistat/MLN4924, Brefeldin A, Alt-R HDR Enhancer (V1), XL413, NU7441, Trichostatin A, CRISPY mix, Romidepsin, Nedisertib/M3814, and Alt-R HDR Enhancer V2.

In embodiments, the method comprises contacting said cell with one or more DNA-PK inhibitors and/or DNA PolQ inhibitors. Non-limiting examples of DNA-PK inhibitors are ART558, NU7026, NU7441, M3814, AZD7648, and KU-0060648.

Any DNA-PK inhibitor, DNA PolQ inhibitor, or HDR enhancer known in the art is suitable for the method of the present invention. A skilled person is capable of selecting a suitable inhibitor or enhancer without undue effort.

The introduction of additional DNA repair modulators can enhance homology-directed repair (HDR), thereby improving the integration efficiency of exogenous DNA sequences during knock-in processes. By strategically modifying the DNA repair pathways, it is possible to achieve more efficient and precise gene edits. This modulation increases the likelihood of desired repair outcomes, favoring HDR over non-homologous end joining (NHEJ), often associated with unwanted genetic alterations. Consequently, employing DNA repair modulators can significantly improve the efficacy and specificity of base editing techniques, facilitating the accurate incorporation of therapeutic genes or sequences into targeted genomic loci.

Various DNA repair modulators are known in the art and a person skilled in the art is able to select a modulator according to the specific need.

In embodiments, one or more PAM regions on the dsDNA targeted by said sgRNAs are oriented facing outwards.

In the context of the present invention, the term "facing outwards" refers to the protospacer adjacent motif (PAM) being oriented outward, or external, to the region located between the target sequences of the two guide RNAs used in the method.

In the context of the present invention, the term "facing outwards" refers to the PAM being oriented outward, or external, to the region located between the target sequences of the two guide RNAs used in the method.

For example, when utilizing a Cas9, the PAM sequences of the two target sites are positioned outside the spacer region between the two single-strand breaks. This is because the PAM is located at the 3' end of the target sequence, and the nicking occurs three bases upstream of the PAM site.

Thus, in a preferred embodiment where a "PAM-out" configuration is employed, the guide RNAs are designed such that the PAM sequences are external to the modification region, leading to the generation of 5' overhangs at the ends of the resulting DNA fragments. This strategic configuration optimizes the generation of overhangs and facilitates precise homology-directed repair, contributing to the high fidelity of the gene editing process.

A person skilled in the art is able to modify these configurations to suit alternative nickases, such as Cas9 H840A or other variants, which may require different guide RNA arrangements or spacer design. The specific positioning of the guide RNAs, the PAM orientation and the resulting overhangs can be adjusted based on the cleavage properties of the particular nickase being used. For instance, different nickases may generate single-strand breaks at alternative positions relative to the PAM, necessitating changes in the guide RNA design and the configuration of the target region to optimize the resulting 5' or 3' overhangs. These modifications ensure efficient and precise gene editing through homology-directed repair in accordance with the method of the invention.

In embodiments, the distance between the two single strand breaks on the dsDNA is more than 29 bases.

In embodiments, the distance between the two single strand breaks on the dsDNA is more than 25 bases, preferably more than 50 bases, more preferably more than 100 bases.

In embodiments, the distance between the two single strand breaks on the dsDNA is less than 250 bases, preferably less than 200 bases.

In embodiments, the distance between two single strand breaks on the dsDNA is between 30 and 250 bases, more preferably between 50 and 200 bases. In embodiments, the distance between two single strand breaks on the dsDNA is 30, 40, 50, 60, 70, 80, 90, 100, 110, 120, 130, 140, 150, 160, 170, 180, 190, 200, 210, 220, 230, 240, 250 bases. The distance between two single strand breaks on the dsDNA may also fall within the range formed between any to endpoints mentioned in the list.

Optimizing the distance between DNA nicks is essential for facilitating effective base editing and the integration of exogenous DNA sequences. The specified distance between two single-strand breaks (SSBs) in double-stranded DNA (dsDNA) is particularly beneficial for achieving efficient homology-directed repair (HDR). Maintaining a maximum distance of less than 250 bases ensures that the repair machinery can effectively bridge the gaps between the breaks. This optimization stabilizes the repair intermediates and aligns with the physical constraints imposed by the cellular repair machinery, enhancing the likelihood of successful repair outcomes. Furthermore, this constraint helps maintain the structural integrity of the repair template and minimizes the potential for erroneous repair processes, thereby improving the overall efficiency and precision of base editing as well as the successful incorporation of therapeutic sequences into the genome.

In one aspect, the present invention relates to a genetically modified eukaryotic cell obtained by the method according to present invention.

Advantageously, the method of the present invention produces genetically modified cells featuring one or more precise genetic alterations. These cells are characterized by targeted genetic modifications attained through the precise application of base editing techniques and the strategic utilization of sgRNAs and DNA repair modulators. As a result, the genetically modified eukaryotic cells generated by this method exhibit specific and intended genetic and functional changes, enhancing their potential for therapeutic applications.

In embodiments, the genetically modified eukaryotic cell comprises at least one insertion of an exogenous DNA donor template sequence and at least one base edit in the dsDNA produced by the base editor system in proximity to said exogenous DNA donor template sequence.

In embodiments, the genetically modified eukaryotic cell comprises 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 insertions of an exogenous DNA donor template sequence. In embodiments, the genetically modified eukaryotic cell comprises at least one insertion of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 exogenous DNA donor template sequences.

In embodiments, the genetically modified eukaryotic cell comprises 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 insertions of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 exogenous DNA donor template sequences.

In further embodiments, the genetically modified eukaryotic cell comprises 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 base edits in the dsDNA produced by the base editor system.

In embodiments, the integration of exogenous DNA sequences comprises the integration of a chimeric antigen receptor (CAR) into a specific genomic locus, e.g., in the *TRAC* locus.

The combination of exogenous DNA insertion and concurrent base editing enables the creation of cells with enhanced or novel functionalities, which is one important objective of the described gene editing methods.

In embodiments, the base editors mediate a knockout of the cell population that did not integrate the DNA template into the target gene.

The present invention provides a novel method for gene editing that utilizes base editing techniques to achieve efficient knockout (KO) of cell populations that fail to integrate the DNA template into the target gene. An embodiment of this method involves using two sgRNAs in conjunction with base editing to mediate targeted gene modifications. For instance, as shown below, in the case of the β2-microglobulin (B2M) gene, one of the sgRNAs employed in the knockout process was designed to disrupt a critical splice site within the gene. This unexpected outcome not only enhances the overall efficiency of the gene editing process but also broadens the potential applications of this method, providing a significant advantage over traditional gene editing techniques.

In a further aspect, the invention relates to a kit for use in the *in vitro* method for modifying double stranded DNA (dsDNA) according to any one of claims 1 to 16, the kit comprising:
a) a base editor system, said system comprising
   i. a base editor, comprising an RNA guided DNA nickase linked to a single-stranded DNA nucleobase modifying enzyme, or a nucleic acid encoding said base editor, and
   ii. at least two guide RNA (sgRNA) sequences, comprising a first sgRNA that hybridizes to a first target sequence in the dsDNA, and a second sgRNA that hybridizes to a second target sequence in the dsDNA,
   wherein a first base editor complex, comprising the first sgRNA, is configured to introduce a nick cleavage of one strand of the dsDNA in the first target sequence, and a second base editor complex, comprising the second sgRNA, is configured to introduce a nick cleavage of the opposite strand of the dsDNA in the second target sequence, thereby producing a 5' or 3' overhang, and
b) an exogenous DNA repair template that hybridizes to the dsDNA in proximity to one or both single strand breaks (SSBs).
c) and optionally one or more DNA repair modulators.

Further embodiments of the invention employ specific gRNA sequences and/or template repair sequences in order to carry out the method of the present invention.

The invention therefore also relates to isolated nucleic acid molecules, including for example gRNA or repair templates (also referred to as HDRT (homology directed repair templates), knock-in templates, knock-ins, and the like) as such. These molecules may represent an embodiment of the invention, to be employed in the inventive method, or an independent aspect of the invention directed to the molecules themselves, preferably defined by the sequences presented below.

**Table 1. Preferred nucleotide sequences of the present invention:**

| **SEQ ID No.** | **Sequence** | **Description** |
|---|---|---|
| 1 | AGAGTCTCTCAGCTGGTACA | TRAC_sense T1 |
| 2 | GAGAATCAAAATCGGTGAAT | TRAC_sense T2 |
| 3 | aaagtcagatttgttgctccagg | TRAC_sense T3 |
| 4 | cttacctgggctggggaagaagg | TRAC_sense T4 |
| 5 | agctgcccttacctgggctgggg | TRAC_sense T5 |
| 6 | caccaaagctgcccttacctggg | TRAC_sense T6 |
| 7 | AGCCCAGGTAAGGGCAGCTT | TRAC_antisense T7 |
| 8 | TGTGCTAGACATGAGGTCTA | TRAC_antisense T8 |
| 9 | AGAGCAACAGT GCTGTGGCC | TRAC_antisense T9 |
| 10 | aacaaatgtgtcacaaagta | TRAC_antisense T10 |
| 11 | gacaccttcttccccagccc | TRAC_antisense T11 |
| 12 | ttcttccccagcccaggtaaggg | TRAC_antisense T12 |
| 13 | acatccccattaccccaggg | CD3z_sense Z1 |
| 14 | tctgtgccaagagataaagc | CD3z_sense Z2 |
| 15 | tccagcaggtagcagagttt | CD3z_sense Z3 |
| 16 | gaatgacaccatagatgaag | CD3z_sense Z4 |
| 17 | tgccttgttcctgagagtga | CD3z_antisense Z5 |
| 18 | gatggaatcctcttcatcta | CD3z_antisense Z6 |
| 19 | aggtgggtaccactgggctt | CD3z_antisense Z7 |
| 20 | gagtgaaggtgggtaccact | CD3z_antisense Z8 |
| 21 | ACAAAGTCACATGGTTCACA | B2M_sense B1 |
| 22 | CTTACCCCACTTAACTATCT | B2M_sense B2 |
| 23 | CTTGCCCCACTTAACTATCT | B2M_sense B3 |
| 24 | TGGGCTGTGACAAAGTCACA | B2M_sense B4 |
| 25 | AGT CACAT GGT T CACAC GGC | B2M_sense B5 |
| 26 | AAGCTGCTTTGATATAAAAA | B2M_antisense B6 |
| 27 | ATCTGCATATTGGGATTGTC | B2M_antisense B7 |
| 28 | ACAGCCCAAGATAGTTAAGT | B2M_antisense B8 |
| 29 | TTTGATATAAAAAAGGTCTA | B2M_antisense B9 |
| 30 | AGAGTCTCTCAGCTGGTACA | TRAC Cas9 |
| 31 | gagtctctcagctggtacacggc | TRAC Cas12a |
| 32 | GCTTACAGATCTTGCCCCGC | RASA2 sgRNA BE #1 |
| 33 | ATTTACCTGAACCTCTGAAT | RASA2 sgRNA BE #2 |
| 34 | CCCTTACCAGGCTTGATGAG | RASA2 sgRNA BE #3 |
| 35 | CTCACCGTCTCCTGGGGAGA | FKBP12 sgRNA BE #1 |
| 36 | CTCACCGGTGTAGTGCACCA | FKBP12 sgRNA BE #2 |
| 37 | CTACTCACCGTCTCCTGGGG | FKBP12 sgRNA BE #3 |
| 38 | TTTCAGGTTTCCTTGAGTGGCAGGCCAGGCCTGGCCGTGAACGTTCACTGAAATCATG GCCTCTTGGCCAAGATTGATAGCTTGTGCCTGTCCCTGAGTCCCAGTCCATCACGAGC AGCTGGTTTCTAAGATGCTATTTCCCGTATAAAGCATGAGACCGTGACTTGCCAGCCC CACAGAGCCCCGCCCTTGTCCATCACTGGCATCTGGACTCCAGCCTGGGTTGGGGCAA AGAGGGAAATGAGATCATGTCCTAACCCTGATCCTCTTGTCCCACAGATATCCAGAAC CCTGACCCTGCCTCCGGATCCGGAGCCACCAACTTCAGCCTGCTGAAGCAGGCCGGCG ACGTGGAAGAAAATCCTGGGCCCATGGCTCTTCCTGTGACTGCCCTTCTGCTGCCCCT GGCTCTCCTGCTCCATGCTGCCCGGCCAGACATCCAGATGACACAGACAACCAGCAGC CTCAGCGCCAGCCTGGGGGACAGAGTGACCATTAGCTGCCGGGCCTCTCAGGACATCA GCAAATACCTGAACTGGTACCAGCAGAAACCAGATGGCACTGTCAAGCTGCTGATTTA CCACACCTCCAGGCTCCACAGCGGCGTGCCCAGTCGCTTCAGCGGCAGTGGGAGCGGG ACAGATTATTCCCTCACAATCTCCAACCTGGAGCAGGAAGATATTGCCACATACTTCT GCCAGCAAGGCAACACCCTGCCATACACATTTGGAGGCGGCACCAAATTGGAGATCAC CGGCGGTGGTGGATCTGGAGGAGGGGGCAGCGGAGGTGGCGGCTCTGAGGTGAAACTG CAGGAGAGTGGCCCTGGCCTGGTGGCTCCCAGCCAGAGCCTTTCTGTCACCTGCACCG TGTCTGGGGTGTCCCTGCCTGACTATGGAGTCTCCTGGATCCGGCAGCCTCCAAGAAA AGGACTGGAATGGCTGGGCGTCATCTGGGGAAGTGAGACCACCTACTATAATTCAGCC CTCAAGTCCCGGCTCACCATCATTAAGGACAACTCCAAATCCCAGGTGTTCCTGAAGA TGAATTCTCTCCAGACTGATGACACAGCCATCTACTACTGTGCCAAGCATTATTATTA CGGCGGGTCCTATGCCATGGACTACTGGGGCCAGGGGACCAGTGTCACTGTTTCTTCT GAAAGCAAGTATGGCCCCCCATGCCCTCCCTGCCCCGGACAGCCGCGGGAGCCCCAGG TCTACACCCTCCCACCCTCCAGAGATGAGCTGACCAAGAACCAGGTTTCACTGACATG CCTGGTGAAGGGCTTCTACCCCTCTGACATTGCTGTGGAGTGGGAGAGCAATGGGCAG CCAGAGAACAACTACAAGACCACACCTCCTGTCCTGGACAGCGACGGCTCCTTCTTCC TCTACAGTAAGCTCACTGTGGACAAGAGCCGCTGGCAGCAGGGCAATGTCTTCTCCTG CAGCGTGATGCACGAGGCCCTGCACAATGCCTATACCCAGAAGTCACTCTCTCTGAGC CCTGGAAAGAAGGATCCCAAGTTTTGGGTCCTCGTGGTGGTCGGGGGTGTGCTGGCCT GCTACAGCTTGCTGGTCACAGTGGCCTTCATCATCTTCTGGGTGCGCTCCAAGAGGAG CCGGCTGCTTCACAGTGATTACATGAACATGACCCCCAGGAGGCCAGGACCCACCAGG AAGCACTACCAGCCCTACGCTCCCCCGCGGGACTTTGCTGCTTACCGCAGCAGGGTCA AATTTTCTAGATCTGCAGATGCGCCGGCCTATCAAcaaggccagaaccagctcTATAA CGAGCTCAATCTAGGACGAAGAGAGGAGTACGATGTTTTGGACAAGAGACGTGGCCGG GACCCTGAGATGGGGGGAAAGCCGAGAAGGAAGAACCCTCAGGAAGGCCTGTACAATG AACTGCAGAAAGATAAGATGGCGGAGGCCTACAGTGAGATTGGGATGAAAGGCGAGCG CCGGAGGGGCAAGGGGCACGATGGCCTTTACCAGGGTCTCAGTACAGCCACCAAGGAC ACCTACGACGCCCTTCACATGCAGGCCCTGCCCCCTCGCTAAtaagaattctaactag agctcgctgatcagcctcgactgtgccttctagttgccagccatctgttgtttgcccc tcccccgtgccttccttgaccctggaaggtgccactcccactgtcctttcctaataaa atgaggaaattgcatcgcattgtctgagtaggtgtcattctattctggggggtggggt ggggcaggacagcaagggggaggattgggaagagaatagcaggcatgctggggacttt ggtgccttcgcaggctgtttccttgcttcaggaatggccaggttctgcccagagctct ggtcaatgatgtctaaaactcctctgattggtggtctcggccttatccattgccacca | HDRT1 (T1/T7) |
| | aaaccctctttttactaagaaacagtgagccttgttctggcagtccagagaatgacac gggaaaaaagcagatgaagagaaggtggcaggagagggcacgtggcccagcctcagtc tctccaactgagttcctgcctgcctgcctttgctcagactgtttgccccttactgctc ttctaggcctcattctaagccccttctccaagttgcctctccttatttctccctgtct gccaaaaaatctttcccagctcactaagtcagtctcacgcagtcactc | |
| 39 | TTTCAGGTTTCCTTGAGTGGCAGGCCAGGCCTGGCCGTGAACGTTCACTGAAATCATG GCCTCTTGGCCAAGATTGATAGCTTGTGCCTGTCCCTGAGTCCCAGTCCATCACGAGC AGCTGGTTTCTAAGATGCTATTTCCCGTATAAAGCATGAGACCGTGACTTGCCAGCCC CACAGAGCCCCGCCCTTGTCCATCACTGGCATCTGGACTCCAGCCTGGGTTGGGGCAA AGAGGGAAATGAGATCATGTCCTAACCCTGATCCTCTTGTCCCACAGATATCCAGAAC CCTGACCCTGCCTCCGGATCCGGAGCCACCAACTTCAGCCTGCTGAAGCAGGCCGGCG ACGTGGAAGAAAATCCTGGGCCCATGGCTCTTCCTGTGACTGCCCTTCTGCTGCCCCT GGCTCTCCTGCTCCATGCTGCCCGGCCAGACATCCAGATGACACAGACAACCAGCAGC CTCAGCGCCAGCCTGGGGGACAGAGTGACCATTAGCTGCCGGGCCTCTCAGGACATCA GCAAATACCTGAACTGGTACCAGCAGAAACCAGATGGCACTGTCAAGCTGCTGATTTA CCACACCTCCAGGCTCCACAGCGGCGTGCCCAGTCGCTTCAGCGGCAGTGGGAGCGGG ACAGATTATTCCCTCACAATCTCCAACCTGGAGCAGGAAGATATTGCCACATACTTCT GCCAGCAAGGCAACACCCTGCCATACACATTTGGAGGCGGCACCAAATTGGAGATCAC CGGCGGTGGTGGATCTGGAGGAGGGGGCAGCGGAGGTGGCGGCTCTGAGGTGAAACTG CAGGAGAGTGGCCCTGGCCTGGTGGCTCCCAGCCAGAGCCTTTCTGTCACCTGCACCG TGTCTGGGGTGTCCCTGCCTGACTATGGAGTCTCCTGGATCCGGCAGCCTCCAAGAAA AGGACTGGAATGGCTGGGCGTCATCTGGGGAAGTGAGACCACCTACTATAATTCAGCC CTCAAGTCCCGGCTCACCATCATTAAGGACAACTCCAAATCCCAGGTGTTCCTGAAGA TGAATTCTCTCCAGACTGATGACACAGCCATCTACTACTGTGCCAAGCATTATTATTA CGGCGGGTCCTATGCCATGGACTACTGGGGCCAGGGGACCAGTGTCACTGTTTCTTCT GAAAGCAAGTATGGCCCCCCATGCCCTCCCTGCCCCGGACAGCCGCGGGAGCCCCAGG TCTACACCCTCCCACCCTCCAGAGATGAGCTGACCAAGAACCAGGTTTCACTGACATG CCTGGTGAAGGGCTTCTACCCCTCTGACATTGCTGTGGAGTGGGAGAGCAATGGGCAG CCAGAGAACAACTACAAGACCACACCTCCTGTCCTGGACAGCGACGGCTCCTTCTTCC TCTACAGTAAGCTCACTGTGGACAAGAGCCGCTGGCAGCAGGGCAATGTCTTCTCCTG CAGCGTGATGCACGAGGCCCTGCACAATGCCTATACCCAGAAGTCACTCTCTCTGAGC CCTGGAAAGAAGGATCCCAAGTTTTGGGTCCTCGTGGTGGTCGGGGGTGTGCTGGCCT GCTACAGCTTGCTGGTCACAGTGGCCTTCATCATCTTCTGGGTGCGCTCCAAGAGGAG CCGGCTGCTTCACAGTGATTACATGAACATGACCCCCAGGAGGCCAGGACCCACCAGG AAGCACTACCAGCCCTACGCTCCCCCGCGGGACTTTGCTGCTTACCGCAGCAGGGTCA AATTTTCTAGATCTGCAGATGCGCCGGCCTATCAAcaaggccagaaccagctcTATAA CGAGCTCAATCTAGGACGAAGAGAGGAGTACGATGTTTTGGACAAGAGACGTGGCCGG GACCCTGAGATGGGGGGAAAGCCGAGAAGGAAGAACCCTCAGGAAGGCCTGTACAATG AACTGCAGAAAGATAAGATGGCGGAGGCCTACAGTGAGATTGGGATGAAAGGCGAGCG CCGGAGGGGCAAGGGGCACGATGGCCTTTACCAGGGTCTCAGTACAGCCACCAAGGAC ACCTACGACGCCCTTCACATGCAGGCCCTGCCCCCTCGCTAAtaagaattctaactag agctcgctgatcagcctcgactgtgccttctagttgccagccatctgttgtttgcccc tcccccgtgccttccttgaccctggaaggtgccactcccactgtcctttcctaataaa atgaggaaattgcatcgcattgtctgagtaggtgtcattctattctggggggtggggt ggggcaggacagcaagggggaggattgggaagagaatagcaggcatgctggggactat ggacttcaagagcaacagtgctgtggcctggagcaacaaatctgactttgcatgtgca aacgccttcaacaacagcattattccagaagacaccttcttccccagcccaggtaagg gcagctttggtgccttcgcaggctgtttccttgcttcaggaatggccaggttctgccc agagctctggtcaatgatgtctaaaactcctctgattggtggtctcggccttatccat tgccaccaaaaccctctttttactaagaaacagtgagccttgttctggcagtccagag aatgacacgggaaaaaagcagatgaagagaaggtggcaggagagggcacgtggcccag cctcagtctctccaactgagttcctgcctgcctgcctttgctcagact | HDRT2 (T1/T8) |
| 40 | TTTCAGGTTTCCTTGAGTGGCAGGCCAGGCCTGGCCGTGAACGTTCACTGAAATCATG GCCTCTTGGCCAAGATTGATAGCTTGTGCCTGTCCCTGAGTCCCAGTCCATCACGAGC AGCTGGTTTCTAAGATGCTATTTCCCGTATAAAGCATGAGACCGTGACTTGCCAGCCC CACAGAGCCCCGCCCTTGTCCATCACTGGCATCTGGACTCCAGCCTGGGTTGGGGCAA AGAGGGAAATGAGATCATGTCCTAACCCTGATCCTCTTGTCCCACAGATATCCAGAAC CCTGACCCTGCCTCCGGATCCGGAGCCACCAACTTCAGCCTGCTGAAGCAGGCCGGCG ACGTGGAAGAAAATCCTGGGCCCATGGCTCTTCCTGTGACTGCCCTTCTGCTGCCCCT GGCTCTCCTGCTCCATGCTGCCCGGCCAGACATCCAGATGACACAGACAACCAGCAGC CTCAGCGCCAGCCTGGGGGACAGAGTGACCATTAGCTGCCGGGCCTCTCAGGACATCA GCAAATACCTGAACTGGTACCAGCAGAAACCAGATGGCACTGTCAAGCTGCTGATTTA | HDRT3 (T1/T9) |
| | CCACACCTCCAGGCTCCACAGCGGCGTGCCCAGTCGCTTCAGCGGCAGTGGGAGCGGG ACAGATTATTCCCTCACAATCTCCAACCTGGAGCAGGAAGATATTGCCACATACTTCT GCCAGCAAGGCAACACCCTGCCATACACATTTGGAGGCGGCACCAAATTGGAGATCAC CGGCGGTGGTGGATCTGGAGGAGGGGGCAGCGGAGGTGGCGGCTCTGAGGTGAAACTG CAGGAGAGTGGCCCTGGCCTGGTGGCTCCCAGCCAGAGCCTTTCTGTCACCTGCACCG TGTCTGGGGTGTCCCTGCCTGACTATGGAGTCTCCTGGATCCGGCAGCCTCCAAGAAA AGGACTGGAATGGCTGGGCGTCATCTGGGGAAGTGAGACCACCTACTATAATTCAGCC CTCAAGTCCCGGCTCACCATCATTAAGGACAACTCCAAATCCCAGGTGTTCCTGAAGA TGAATTCTCTCCAGACTGATGACACAGCCATCTACTACTGTGCCAAGCATTATTATTA CGGCGGGTCCTATGCCATGGACTACTGGGGCCAGGGGACCAGTGTCACTGTTTCTTCT GAAAGCAAGTATGGCCCCCCATGCCCTCCCTGCCCCGGACAGCCGCGGGAGCCCCAGG TCTACACCCTCCCACCCTCCAGAGATGAGCTGACCAAGAACCAGGTTTCACTGACATG CCTGGTGAAGGGCTTCTACCCCTCTGACATTGCTGTGGAGTGGGAGAGCAATGGGCAG CCAGAGAACAACTACAAGACCACACCTCCTGTCCTGGACAGCGACGGCTCCTTCTTCC TCTACAGTAAGCTCACTGTGGACAAGAGCCGCTGGCAGCAGGGCAATGTCTTCTCCTG CAGCGTGATGCACGAGGCCCTGCACAATGCCTATACCCAGAAGTCACTCTCTCTGAGC CCTGGAAAGAAGGATCCCAAGTTTTGGGTCCTCGTGGTGGTCGGGGGTGTGCTGGCCT GCTACAGCTTGCTGGTCACAGTGGCCTTCATCATCTTCTGGGTGCGCTCCAAGAGGAG CCGGCTGCTTCACAGTGATTACATGAACATGACCCCCAGGAGGCCAGGACCCACCAGG AAGCACTACCAGCCCTACGCTCCCCCGCGGGACTTTGCTGCTTACCGCAGCAGGGTCA AATTTTCTAGATCTGCAGATGCGCCGGCCTATCAAcaaggccagaaccagctcTATAA CGAGCTCAATCTAGGACGAAGAGAGGAGTACGATGTTTTGGACAAGAGACGTGGCCGG GACCCTGAGATGGGGGGAAAGCCGAGAAGGAAGAACCCTCAGGAAGGCCTGTACAATG AACTGCAGAAAGATAAGATGGCGGAGGCCTACAGTGAGATTGGGATGAAAGGCGAGCG CCGGAGGGGCAAGGGGCACGATGGCCTTTACCAGGGTCTCAGTACAGCCACCAAGGAC ACCTACGACGCCCTTCACATGCAGGCCCTGCCCCCTCGCTAAtaagaattctaactag agctcgctgatcagcctcgactgtgccttctagttgccagccatctgttgtttgcccc tcccccgtgccttccttgaccctggaaggtgccactcccactgtcctttcctaataaa atgaggaaattgcatcgcattgtctgagtaggtgtcattctattctggggggtggggt ggggcaggacagcaagggggaggattgggaagagaatagcaggcatgctggggagcct ggagcaacaaatctgactttgcatgtgcaaacgccttcaacaacagcattattccaga agacaccttcttccccagcccaggtaagggcagctttggtgccttcgcaggctgtttc cttgcttcaggaatggccaggttctgcccagagctctggtcaatgatgtctaaaactc ctctgattggtggtctcggccttatccattgccaccaaaaccctctttttactaagaa acagtgagccttgttctggcagtccagagaatgacacgggaaaaaagcagatgaagag aaggtggcaggagagggcacgtggcccagcctcagtctctccaactgagttcctgcct gcctgcctttgctcagactgtttgccccttactgctcttctaggcctc | |
| 41 | TTTCAGGTTTCCTTGAGTGGCAGGCCAGGCCTGGCCGTGAACGTTCACTGAAATCATG GCCTCTTGGCCAAGATTGATAGCTTGTGCCTGTCCCTGAGTCCCAGTCCATCACGAGC AGCTGGTTTCTAAGATGCTATTTCCCGTATAAAGCATGAGACCGTGACTTGCCAGCCC CACAGAGCCCCGCCCTTGTCCATCACTGGCATCTGGACTCCAGCCTGGGTTGGGGCAA AGAGGGAAATGAGATCATGTCCTAACCCTGATCCTCTTGTCCCACAGATATCCAGAAC CCTGACCCTGCCTCCGGATCCGGAGCCACCAACTTCAGCCTGCTGAAGCAGGCCGGCG ACGTGGAAGAAAATCCTGGGCCCATGGCTCTTCCTGTGACTGCCCTTCTGCTGCCCCT GGCTCTCCTGCTCCATGCTGCCCGGCCAGACATCCAGATGACACAGACAACCAGCAGC CTCAGCGCCAGCCTGGGGGACAGAGTGACCATTAGCTGCCGGGCCTCTCAGGACATCA GCAAATACCTGAACTGGTACCAGCAGAAACCAGATGGCACTGTCAAGCTGCTGATTTA CCACACCTCCAGGCTCCACAGCGGCGTGCCCAGTCGCTTCAGCGGCAGTGGGAGCGGG ACAGATTATTCCCTCACAATCTCCAACCTGGAGCAGGAAGATATTGCCACATACTTCT GCCAGCAAGGCAACACCCTGCCATACACATTTGGAGGCGGCACCAAATTGGAGATCAC CGGCGGTGGTGGATCTGGAGGAGGGGGCAGCGGAGGTGGCGGCTCTGAGGTGAAACTG CAGGAGAGTGGCCCTGGCCTGGTGGCTCCCAGCCAGAGCCTTTCTGTCACCTGCACCG TGTCTGGGGTGTCCCTGCCTGACTATGGAGTCTCCTGGATCCGGCAGCCTCCAAGAAA AGGACTGGAATGGCTGGGCGTCATCTGGGGAAGTGAGACCACCTACTATAATTCAGCC CTCAAGTCCCGGCTCACCATCATTAAGGACAACTCCAAATCCCAGGTGTTCCTGAAGA TGAATTCTCTCCAGACTGATGACACAGCCATCTACTACTGTGCCAAGCATTATTATTA CGGCGGGTCCTATGCCATGGACTACTGGGGCCAGGGGACCAGTGTCACTGTTTCTTCT GAAAGCAAGTATGGCCCCCCATGCCCTCCCTGCCCCGGACAGCCGCGGGAGCCCCAGG TCTACACCCTCCCACCCTCCAGAGATGAGCTGACCAAGAACCAGGTTTCACTGACATG CCTGGTGAAGGGCTTCTACCCCTCTGACATTGCTGTGGAGTGGGAGAGCAATGGGCAG CCAGAGAACAACTACAAGACCACACCTCCTGTCCTGGACAGCGACGGCTCCTTCTTCC TCTACAGTAAGCTCACTGTGGACAAGAGCCGCTGGCAGCAGGGCAATGTCTTCTCCTG | HDRT4 (T1/T10) |
| | CAGCGTGATGCACGAGGCCCTGCACAATGCCTATACCCAGAAGTCACTCTCTCTGAGC CCTGGAAAGAAGGATCCCAAGTTTTGGGTCCTCGTGGTGGTCGGGGGTGTGCTGGCCT GCTACAGCTTGCTGGTCACAGTGGCCTTCATCATCTTCTGGGTGCGCTCCAAGAGGAG CCGGCTGCTTCACAGTGATTACATGAACATGACCCCCAGGAGGCCAGGACCCACCAGG AAGCACTACCAGCCCTACGCTCCCCCGCGGGACTTTGCTGCTTACCGCAGCAGGGTCA AATTTTCTAGATCTGCAGATGCGCCGGCCTATCAAcaaggccagaaccagctcTATAA CGAGCTCAATCTAGGACGAAGAGAGGAGTACGATGTTTTGGACAAGAGACGTGGCCGG GACCCTGAGATGGGGGGAAAGCCGAGAAGGAAGAACCCTCAGGAAGGCCTGTACAATG AACTGCAGAAAGATAAGATGGCGGAGGCCTACAGTGAGATTGGGATGAAAGGCGAGCG CCGGAGGGGCAAGGGGCACGATGGCCTTTACCAGGGTCTCAGTACAGCCACCAAGGAC ACCTACGACGCCCTTCACATGCAGGCCCTGCCCCCTCGCTAAtaagaattctaactag agctcgctgatcagcctcgactgtgccttctagttgccagccatctgttgtttgcccc tcccccgtgccttccttgaccctggaaggtgccactcccactgtcctttcctaataaa atgaggaaattgcatcgcattgtctgagtaggtgtcattctattctggggggtggggt ggggcaggacagcaagggggaggattgggaagagaatagcaggcatgctggggaaagg attctgatgtgtatatcacagacaaaactgtgctagacatgaggtctatggacttcaa gagcaacagtgctgtggcctggagcaacaaatctgactttgcatgtgcaaacgccttc aacaacagcattattccagaagacaccttcttccccagcccaggtaagggcagctttg gtgccttcgcaggctgtttccttgcttcaggaatggccaggttctgcccagagctctg gtcaatgatgtctaaaactcctctgattggtggtctcggccttatccattgccaccaa aaccctctttttactaagaaacagtgagccttgttctggcagtccagagaatgacacg ggaaaaaagcagatg | |
| 42 | TTTCAGGTTTCCTTGAGTGGCAGGCCAGGCCTGGCCGTGAACGTTCACTGAAATCATG GCCTCTTGGCCAAGATTGATAGCTTGTGCCTGTCCCTGAGTCCCAGTCCATCACGAGC AGCTGGTTTCTAAGATGCTATTTCCCGTATAAAGCATGAGACCGTGACTTGCCAGCCC CACAGAGCCCCGCCCTTGTCCATCACTGGCATCTGGACTCCAGCCTGGGTTGGGGCAA AGAGGGAAATGAGATCATGTCCTAACCCTGATCCTCTTGTCCCACAGATATCCAGAAC CCTGACCCTGCCTCCGGATCCGGAGCCACCAACTTCAGCCTGCTGAAGCAGGCCGGCG ACGTGGAAGAAAATCCTGGGCCCATGGCTCTTCCTGTGACTGCCCTTCTGCTGCCCCT GGCTCTCCTGCTCCATGCTGCCCGGCCAGACATCCAGATGACACAGACAACCAGCAGC CTCAGCGCCAGCCTGGGGGACAGAGTGACCATTAGCTGCCGGGCCTCTCAGGACATCA GCAAATACCTGAACTGGTACCAGCAGAAACCAGATGGCACTGTCAAGCTGCTGATTTA CCACACCTCCAGGCTCCACAGCGGCGTGCCCAGTCGCTTCAGCGGCAGTGGGAGCGGG ACAGATTATTCCCTCACAATCTCCAACCTGGAGCAGGAAGATATTGCCACATACTTCT GCCAGCAAGGCAACACCCTGCCATACACATTTGGAGGCGGCACCAAATTGGAGATCAC CGGCGGTGGTGGATCTGGAGGAGGGGGCAGCGGAGGTGGCGGCTCTGAGGTGAAACTG CAGGAGAGTGGCCCTGGCCTGGTGGCTCCCAGCCAGAGCCTTTCTGTCACCTGCACCG TGTCTGGGGTGTCCCTGCCTGACTATGGAGTCTCCTGGATCCGGCAGCCTCCAAGAAA AGGACTGGAATGGCTGGGCGTCATCTGGGGAAGTGAGACCACCTACTATAATTCAGCC CTCAAGTCCCGGCTCACCATCATTAAGGACAACTCCAAATCCCAGGTGTTCCTGAAGA TGAATTCTCTCCAGACTGATGACACAGCCATCTACTACTGTGCCAAGCATTATTATTA CGGCGGGTCCTATGCCATGGACTACTGGGGCCAGGGGACCAGTGTCACTGTTTCTTCT GAAAGCAAGTATGGCCCCCCATGCCCTCCCTGCCCCGGACAGCCGCGGGAGCCCCAGG TCTACACCCTCCCACCCTCCAGAGATGAGCTGACCAAGAACCAGGTTTCACTGACATG CCTGGTGAAGGGCTTCTACCCCTCTGACATTGCTGTGGAGTGGGAGAGCAATGGGCAG CCAGAGAACAACTACAAGACCACACCTCCTGTCCTGGACAGCGACGGCTCCTTCTTCC TCTACAGTAAGCTCACTGTGGACAAGAGCCGCTGGCAGCAGGGCAATGTCTTCTCCTG CAGCGTGATGCACGAGGCCCTGCACAATGCCTATACCCAGAAGTCACTCTCTCTGAGC CCTGGAAAGAAGGATCCCAAGTTTTGGGTCCTCGTGGTGGTCGGGGGTGTGCTGGCCT GCTACAGCTTGCTGGTCACAGTGGCCTTCATCATCTTCTGGGTGCGCTCCAAGAGGAG CCGGCTGCTTCACAGTGATTACATGAACATGACCCCCAGGAGGCCAGGACCCACCAGG AAGCACTACCAGCCCTACGCTCCCCCGCGGGACTTTGCTGCTTACCGCAGCAGGGTCA AATTTTCTAGATCTGCAGATGCGCCGGCCTATCAAcaaggccagaaccagctcTATAA CGAGCTCAATCTAGGACGAAGAGAGGAGTACGATGTTTTGGACAAGAGACGTGGCCGG GACCCTGAGATGGGGGGAAAGCCGAGAAGGAAGAACCCTCAGGAAGGCCTGTACAATG AACTGCAGAAAGATAAGATGGCGGAGGCCTACAGTGAGATTGGGATGAAAGGCGAGCG CCGGAGGGGCAAGGGGCACGATGGCCTTTACCAGGGTCTCAGTACAGCCACCAAGGAC ACCTACGACGCCCTTCACATGCAGGCCCTGCCCCCTCGCTAAtaagaattctaactag agctcgctgatcagcctcgactgtgccttctagttgccagccatctgttgtttgcccc tcccccgtgccttccttgaccctggaaggtgccactcccactgtcctttcctaataaa atgaggaaattgcatcgcattgtctgagtaggtgtcattctattctggggggtggggt ggggcaggacagcaagggggaggattgggaagagaatagcaggcatgctggggacttt | HDRT1 (T1/T11) |
| | ggtgccttcgcaggctgtttccttgcttcaggaatggccaggttctgcccagagctct ggtcaatgatgtctaaaactcctctgattggtggtctcggccttatccattgccacca aaaccctctttttactaagaaacagtgagccttgttctggcagtccagagaatgacac gggaaaaaagcagatgaagagaaggtggcaggagagggcacgtggcccagcctcagtc tctccaactgagttcctgcctgcctgcctttgctcagactgtttgccccttactgctc ttctaggcctcattctaagccccttctccaagttgcctctccttatttctccctgtct gccaaaaaatctttcccagctcactaagtcagtctcacgcagtcactc | |
| 43 | TTTCAGGTTTCCTTGAGTGGCAGGCCAGGCCTGGCCGTGAACGTTCACTGAAATCATG GCCTCTTGGCCAAGATTGATAGCTTGTGCCTGTCCCTGAGTCCCAGTCCATCACGAGC AGCTGGTTTCTAAGATGCTATTTCCCGTATAAAGCATGAGACCGTGACTTGCCAGCCC CACAGAGCCCCGCCCTTGTCCATCACTGGCATCTGGACTCCAGCCTGGGTTGGGGCAA AGAGGGAAATGAGATCATGTCCTAACCCTGATCCTCTTGTCCCACAGATATCCAGAAC CCTGACCCTGCCTCCGGATCCGGAGCCACCAACTTCAGCCTGCTGAAGCAGGCCGGCG ACGTGGAAGAAAATCCTGGGCCCATGGCTCTTCCTGTGACTGCCCTTCTGCTGCCCCT GGCTCTCCTGCTCCATGCTGCCCGGCCAGACATCCAGATGACACAGACAACCAGCAGC CTCAGCGCCAGCCTGGGGGACAGAGTGACCATTAGCTGCCGGGCCTCTCAGGACATCA GCAAATACCTGAACTGGTACCAGCAGAAACCAGATGGCACTGTCAAGCTGCTGATTTA CCACACCTCCAGGCTCCACAGCGGCGTGCCCAGTCGCTTCAGCGGCAGTGGGAGCGGG ACAGATTATTCCCTCACAATCTCCAACCTGGAGCAGGAAGATATTGCCACATACTTCT GCCAGCAAGGCAACACCCTGCCATACACATTTGGAGGCGGCACCAAATTGGAGATCAC CGGCGGTGGTGGATCTGGAGGAGGGGGCAGCGGAGGTGGCGGCTCTGAGGTGAAACTG CAGGAGAGTGGCCCTGGCCTGGTGGCTCCCAGCCAGAGCCTTTCTGTCACCTGCACCG TGTCTGGGGTGTCCCTGCCTGACTATGGAGTCTCCTGGATCCGGCAGCCTCCAAGAAA AGGACTGGAATGGCTGGGCGTCATCTGGGGAAGTGAGACCACCTACTATAATTCAGCC CTCAAGTCCCGGCTCACCATCATTAAGGACAACTCCAAATCCCAGGTGTTCCTGAAGA TGAATTCTCTCCAGACTGATGACACAGCCATCTACTACTGTGCCAAGCATTATTATTA CGGCGGGTCCTATGCCATGGACTACTGGGGCCAGGGGACCAGTGTCACTGTTTCTTCT GAAAGCAAGTATGGCCCCCCATGCCCTCCCTGCCCCGGACAGCCGCGGGAGCCCCAGG TCTACACCCTCCCACCCTCCAGAGATGAGCTGACCAAGAACCAGGTTTCACTGACATG CCTGGTGAAGGGCTTCTACCCCTCTGACATTGCTGTGGAGTGGGAGAGCAATGGGCAG CCAGAGAACAACTACAAGACCACACCTCCTGTCCTGGACAGCGACGGCTCCTTCTTCC TCTACAGTAAGCTCACTGTGGACAAGAGCCGCTGGCAGCAGGGCAATGTCTTCTCCTG CAGCGTGATGCACGAGGCCCTGCACAATGCCTATACCCAGAAGTCACTCTCTCTGAGC CCTGGAAAGAAGGATCCCAAGTTTTGGGTCCTCGTGGTGGTCGGGGGTGTGCTGGCCT GCTACAGCTTGCTGGTCACAGTGGCCTTCATCATCTTCTGGGTGCGCTCCAAGAGGAG CCGGCTGCTTCACAGTGATTACATGAACATGACCCCCAGGAGGCCAGGACCCACCAGG AAGCACTACCAGCCCTACGCTCCCCCGCGGGACTTTGCTGCTTACCGCAGCAGGGTCA AATTTTCTAGATCTGCAGATGCGCCGGCCTATCAAcaaggccagaaccagctcTATAA CGAGCTCAATCTAGGACGAAGAGAGGAGTACGATGTTTTGGACAAGAGACGTGGCCGG GACCCTGAGATGGGGGGAAAGCCGAGAAGGAAGAACCCTCAGGAAGGCCTGTACAATG AACTGCAGAAAGATAAGATGGCGGAGGCCTACAGTGAGATTGGGATGAAAGGCGAGCG CCGGAGGGGCAAGGGGCACGATGGCCTTTACCAGGGTCTCAGTACAGCCACCAAGGAC ACCTACGACGCCCTTCACATGCAGGCCCTGCCCCCTCGCTAAtaagaattctaactag agctcgctgatcagcctcgactgtgccttctagttgccagccatctgttgtttgcccc tcccccgtgccttccttgaccctggaaggtgccactcccactgtcctttcctaataaa atgaggaaattgcatcgcattgtctgagtaggtgtcattctattctggggggtggggt ggggcaggacagcaagggggaggattgggaagagaatagcaggcatgctggggacttt ggtgccttcgcaggctgtttccttgcttcaggaatggccaggttctgcccagagctct ggtcaatgatgtctaaaactcctctgattggtggtctcggccttatccattgccacca aaaccctctttttactaagaaacagtgagccttgttctggcagtccagagaatgacac gggaaaaaagcagatgaagagaaggtggcaggagagggcacgtggcccagcctcagtc tctccaactgagttcctgcctgcctgcctttgctcagactgtttgccccttactgctc ttctaggcctcattctaagccccttctccaagttgcctctccttatttctccctgtct gccaaaaaatctttcccagctcactaagtcagtctcacgcagtcactc | HDRT1 (T1/T12) |
| 44 | cctattaaataaaagaataagcagtattattaagtagccctgcatttcaggtttcctt gagtggcaggccaggcctggccgtgaacgttcactgaaatcatggcctcttggccaag attgatagcttgtgcctgtccctgagtcccagtccatcacgagcagctggtttctaag atgctatttcccgtataaagcatgagaccgtgacttgccagccccacagagccccgcc cttgtccatcactggcatctggactccagcctgggttggggcaaagagggaaatgaga tcatgtcctaaccctgatcctcttgtcccacagatatccagaaccctgaccctgccgt gtaccagctgagagactctaaatccagtgacaagtctgtctgcctattcaccGCCACC AACTTCTCTCTTTTGAAGCAGGCCGGAGATGTGGAAGAAAATCCTGGGCCCATGGCTC | HDRT6 (T2/T8) |
| | TTCCTGTGACTGCCCTTCTGCTGCCCCTGGCTCTCCTGCTCCATGCTGCCCGGCCAGA CATCCAGATGACACAGACAACCAGCAGCCTCAGCGCCAGCCTGGGGGACAGAGTGACC ATTAGCTGCCGGGCCTCTCAGGACATCAGCAAATACCTGAACTGGTACCAGCAGAAAC CAGATGGCACTGTCAAGCTGCTGATTTACCACACCTCCAGGCTCCACAGCGGCGTGCC CAGTCGCTTCAGCGGCAGTGGGAGCGGGACAGATTATTCCCTCACAATCTCCAACCTG GAGCAGGAAGATATTGCCACATACTTCTGCCAGCAAGGCAACACCCTGCCATACACAT TTGGAGGCGGCACCAAATTGGAGATCACCGGCGGTGGTGGATCTGGAGGAGGGGGCAG CGGAGGTGGCGGCTCTGAGGTGAAACTGCAGGAGAGTGGCCCTGGCCTGGTGGCTCCC AGCCAGAGCCTTTCTGTCACCTGCACCGTGTCTGGGGTGTCCCTGCCTGACTATGGAG TCTCCTGGATCCGGCAGCCTCCAAGAAAAGGACTGGAATGGCTGGGCGTCATCTGGGG AAGTGAGACCACCTACTATAATTCAGCCCTCAAGTCCCGGCTCACCATCATTAAGGAC AACTCCAAATCCCAGGTGTTCCTGAAGATGAATTCTCTCCAGACTGATGACACAGCCA TCTACTACTGTGCCAAGCATTATTATTACGGCGGGTCCTATGCCATGGACTACTGGGG CCAGGGGACCAGTGTCACTGTTTCTTCTGAAAGCAAGTATGGCCCCCCATGCCCTCCC TGCCCCGGACAGCCGCGGGAGCCCCAGGTCTACACCCTCCCACCCTCCAGAGATGAGC TGACCAAGAACCAGGTTTCACTGACATGCCTGGTGAAGGGCTTCTACCCCTCTGACAT TGCTGTGGAGTGGGAGAGCAATGGGCAGCCAGAGAACAACTACAAGACCACACCTCCT GTCCTGGACAGCGACGGCTCCTTCTTCCTCTACAGTAAGCTCACTGTGGACAAGAGCC GCTGGCAGCAGGGCAATGTCTTCTCCTGCAGCGTGATGCACGAGGCCCTGCACAATGC CTATACCCAGAAGTCACTCTCTCTGAGCCCTGGAAAGAAGGATCCCAAGTTTTGGGTC CTCGTGGTGGTCGGGGGTGTGCTGGCCTGCTACAGCTTGCTGGTCACAGTGGCCTTCA TCATCTTCTGGGTGCGCTCCAAGAGGAGCCGGCTGCTTCACAGTGATTACATGAACAT GACCCCCAGGAGGCCAGGACCCACCAGGAAGCACTACCAGCCCTACGCTCCCCCGCGG GACTTTGCTGCTTACCGCAGCAGGGTCAAATTTTCTAGATCTGCAGATGCGCCGGCCT ATCAAcaaggccagaaccagctcTATAACGAGCTCAATCTAGGACGAAGAGAGGAGTA CGATGTTTTGGACAAGAGACGTGGCCGGGACCCTGAGATGGGGGGAAAGCCGAGAAGG AAGAACCCTCAGGAAGGCCTGTACAATGAACTGCAGAAAGATAAGATGGCGGAGGCCT ACAGTGAGATTGGGATGAAAGGCGAGCGCCGGAGGGGCAAGGGGCACGATGGCCTTTA CCAGGGTCTCAGTACAGCCACCAAGGACACCTACGACGCCCTTCACATGCAGGCCCTG CCCCCTCGCTAAtaagaattctaactagagctcgctgatcagcctcgactgtgccttc tagttgccagccatctgttgtttgcccctcccccgtgccttccttgaccctggaaggt gccactcccactgtcctttcctaataaaatgaggaaattgcatcgcattgtctgagta ggtgtcattctattctggggggtggggtggggcaggacagcaagggggaggattggga agagaatagcaggcatgctggggactatggacttcaagagcaacagtgctgtggcctg gagcaacaaatctgactttgcatgtgcaaacgccttcaacaacagcattattccagaa gacaccttcttccccagcccaggtaagggcagctttggtgccttcgcaggctgtttcc ttgcttcaggaatggccaggttctgcccagagctctggtcaatgatgtctaaaactcc tctgattggtggtctcggccttatccattgccaccaaaaccctctttttactaagaaa cagtgagccttgttctggcagtccagagaatgacacgggaaaaaagcagatgaagaga aggtggcaggagagggcacgtggcccagcctcagtctctccaactgagttcctgcctg cctgcctttgctcagact | |
| 45 | cctattaaataaaagaataagcagtattattaagtagccctgcatttcaggtttcctt gagtggcaggccaggcctggccgtgaacgttcactgaaatcatggcctcttggccaag attgatagcttgtgcctgtccctgagtcccagtccatcacgagcagctggtttctaag atgctatttcccgtataaagcatgagaccgtgacttgccagccccacagagccccgcc cttgtccatcactggcatctggactccagcctgggttggggcaaagagggaaatgaga tcatgtcctaaccctgatcctcttgtcccacagatatccagaaccctgaccctgccgt gtaccagctgagagactctaaatccagtgacaagtctgtctgcctattcaccGCCACC AACTTCTCTCTTTTGAAGCAGGCCGGAGATGTGGAAGAAAATCCTGGGCCCATGGCTC TTCCTGTGACTGCCCTTCTGCTGCCCCTGGCTCTCCTGCTCCATGCTGCCCGGCCAGA CATCCAGATGACACAGACAACCAGCAGCCTCAGCGCCAGCCTGGGGGACAGAGTGACC ATTAGCTGCCGGGCCTCTCAGGACATCAGCAAATACCTGAACTGGTACCAGCAGAAAC CAGATGGCACTGTCAAGCTGCTGATTTACCACACCTCCAGGCTCCACAGCGGCGTGCC CAGTCGCTTCAGCGGCAGTGGGAGCGGGACAGATTATTCCCTCACAATCTCCAACCTG GAGCAGGAAGATATTGCCACATACTTCTGCCAGCAAGGCAACACCCTGCCATACACAT TTGGAGGCGGCACCAAATTGGAGATCACCGGCGGTGGTGGATCTGGAGGAGGGGGCAG CGGAGGTGGCGGCTCTGAGGTGAAACTGCAGGAGAGTGGCCCTGGCCTGGTGGCTCCC AGCCAGAGCCTTTCTGTCACCTGCACCGTGTCTGGGGTGTCCCTGCCTGACTATGGAG TCTCCTGGATCCGGCAGCCTCCAAGAAAAGGACTGGAATGGCTGGGCGTCATCTGGGG AAGTGAGACCACCTACTATAATTCAGCCCTCAAGTCCCGGCTCACCATCATTAAGGAC AACTCCAAATCCCAGGTGTTCCTGAAGATGAATTCTCTCCAGACTGATGACACAGCCA TCTACTACTGTGCCAAGCATTATTATTACGGCGGGTCCTATGCCATGGACTACTGGGG | HDRT7 (T2/T9) |
| | CCAGGGGACCAGTGTCACTGTTTCTTCTGAAAGCAAGTATGGCCCCCCATGCCCTCCC TGCCCCGGACAGCCGCGGGAGCCCCAGGTCTACACCCTCCCACCCTCCAGAGATGAGC TGACCAAGAACCAGGTTTCACTGACATGCCTGGTGAAGGGCTTCTACCCCTCTGACAT TGCTGTGGAGTGGGAGAGCAATGGGCAGCCAGAGAACAACTACAAGACCACACCTCCT GTCCTGGACAGCGACGGCTCCTTCTTCCTCTACAGTAAGCTCACTGTGGACAAGAGCC GCTGGCAGCAGGGCAATGTCTTCTCCTGCAGCGTGATGCACGAGGCCCTGCACAATGC CTATACCCAGAAGTCACTCTCTCTGAGCCCTGGAAAGAAGGATCCCAAGTTTTGGGTC CTCGTGGTGGTCGGGGGTGTGCTGGCCTGCTACAGCTTGCTGGTCACAGTGGCCTTCA TCATCTTCTGGGTGCGCTCCAAGAGGAGCCGGCTGCTTCACAGTGATTACATGAACAT GACCCCCAGGAGGCCAGGACCCACCAGGAAGCACTACCAGCCCTACGCTCCCCCGCGG GACTTTGCTGCTTACCGCAGCAGGGTCAAATTTTCTAGATCTGCAGATGCGCCGGCCT ATCAAcaaggccagaaccagctcTATAACGAGCTCAATCTAGGACGAAGAGAGGAGTA CGATGTTTTGGACAAGAGACGTGGCCGGGACCCTGAGATGGGGGGAAAGCCGAGAAGG AAGAACCCTCAGGAAGGCCTGTACAATGAACTGCAGAAAGATAAGATGGCGGAGGCCT ACAGTGAGATTGGGATGAAAGGCGAGCGCCGGAGGGGCAAGGGGCACGATGGCCTTTA CCAGGGTCTCAGTACAGCCACCAAGGACACCTACGACGCCCTTCACATGCAGGCCCTG CCCCCTCGCTAAtaagaattctaactagagctcgctgatcagcctcgactgtgccttc tagttgccagccatctgttgtttgcccctcccccgtgccttccttgaccctggaaggt gccactcccactgtcctttcctaataaaatgaggaaattgcatcgcattgtctgagta ggtgtcattctattctggggggtggggtggggcaggacagcaagggggaggattggga agagaatagcaggcatgctggggagcctggagcaacaaatctgactttgcatgtgcaa acgccttcaacaacagcattattccagaagacaccttcttccccagcccaggtaaggg cagctttggtgccttcgcaggctgtttccttgcttcaggaatggccaggttctgccca gagctctggtcaatgatgtctaaaactcctctgattggtggtctcggccttatccatt gccaccaaaaccctctttttactaagaaacagtgagccttgttctggcagtccagaga atgacacgggaaaaaagcagatgaagagaaggtggcaggagagggcacgtggcccagc ctcagtctctccaactgagttcctgcctgcctgcctttgctcagactgtttgcccctt actgctcttctaggcctc | |
| 46 | cctattaaataaaagaataagcagtattattaagtagccctgcatttcaggtttcctt gagtggcaggccaggcctggccgtgaacgttcactgaaatcatggcctcttggccaag attgatagcttgtgcctgtccctgagtcccagtccatcacgagcagctggtttctaag atgctatttcccgtataaagcatgagaccgtgacttgccagccccacagagccccgcc cttgtccatcactggcatctggactccagcctgggttggggcaaagagggaaatgaga tcatgtcctaaccctgatcctcttgtcccacagatatccagaaccctgaccctgccgt gtaccagctgagagactctaaatccagtgacaagtctgtctgcctattcaccGCCACC AACTTCTCTCTTTTGAAGCAGGCCGGAGATGTGGAAGAAAATCCTGGGCCCATGGCTC TTCCTGTGACTGCCCTTCTGCTGCCCCTGGCTCTCCTGCTCCATGCTGCCCGGCCAGA CATCCAGATGACACAGACAACCAGCAGCCTCAGCGCCAGCCTGGGGGACAGAGTGACC ATTAGCTGCCGGGCCTCTCAGGACATCAGCAAATACCTGAACTGGTACCAGCAGAAAC CAGATGGCACTGTCAAGCTGCTGATTTACCACACCTCCAGGCTCCACAGCGGCGTGCC CAGTCGCTTCAGCGGCAGTGGGAGCGGGACAGATTATTCCCTCACAATCTCCAACCTG GAGCAGGAAGATATTGCCACATACTTCTGCCAGCAAGGCAACACCCTGCCATACACAT TTGGAGGCGGCACCAAATTGGAGATCACCGGCGGTGGTGGATCTGGAGGAGGGGGCAG CGGAGGTGGCGGCTCTGAGGTGAAACTGCAGGAGAGTGGCCCTGGCCTGGTGGCTCCC AGCCAGAGCCTTTCTGTCACCTGCACCGTGTCTGGGGTGTCCCTGCCTGACTATGGAG TCTCCTGGATCCGGCAGCCTCCAAGAAAAGGACTGGAATGGCTGGGCGTCATCTGGGG AAGTGAGACCACCTACTATAATTCAGCCCTCAAGTCCCGGCTCACCATCATTAAGGAC AACTCCAAATCCCAGGTGTTCCTGAAGATGAATTCTCTCCAGACTGATGACACAGCCA TCTACTACTGTGCCAAGCATTATTATTACGGCGGGTCCTATGCCATGGACTACTGGGG CCAGGGGACCAGTGTCACTGTTTCTTCTGAAAGCAAGTATGGCCCCCCATGCCCTCCC TGCCCCGGACAGCCGCGGGAGCCCCAGGTCTACACCCTCCCACCCTCCAGAGATGAGC TGACCAAGAACCAGGTTTCACTGACATGCCTGGTGAAGGGCTTCTACCCCTCTGACAT TGCTGTGGAGTGGGAGAGCAATGGGCAGCCAGAGAACAACTACAAGACCACACCTCCT GTCCTGGACAGCGACGGCTCCTTCTTCCTCTACAGTAAGCTCACTGTGGACAAGAGCC GCTGGCAGCAGGGCAATGTCTTCTCCTGCAGCGTGATGCACGAGGCCCTGCACAATGC CTATACCCAGAAGTCACTCTCTCTGAGCCCTGGAAAGAAGGATCCCAAGTTTTGGGTC CTCGTGGTGGTCGGGGGTGTGCTGGCCTGCTACAGCTTGCTGGTCACAGTGGCCTTCA TCATCTTCTGGGTGCGCTCCAAGAGGAGCCGGCTGCTTCACAGTGATTACATGAACAT GACCCCCAGGAGGCCAGGACCCACCAGGAAGCACTACCAGCCCTACGCTCCCCCGCGG GACTTTGCTGCTTACCGCAGCAGGGTCAAATTTTCTAGATCTGCAGATGCGCCGGCCT ATCAAcaaggccagaaccagctcTATAACGAGCTCAATCTAGGACGAAGAGAGGAGTA CGATGTTTTGGACAAGAGACGTGGCCGGGACCCTGAGATGGGGGGAAAGCCGAGAAGG | HDRT8 (T2/T10) |
| | AAGAACCCTCAGGAAGGCCTGTACAATGAACTGCAGAAAGATAAGATGGCGGAGGCCT ACAGTGAGATTGGGATGAAAGGCGAGCGCCGGAGGGGCAAGGGGCACGATGGCCTTTA CCAGGGTCTCAGTACAGCCACCAAGGACACCTACGACGCCCTTCACATGCAGGCCCTG CCCCCTCGCTAAtaagaattctaactagagctcgctgatcagcctcgactgtgccttc tagttgccagccatctgttgtttgcccctcccccgtgccttccttgaccctggaaggt gccactcccactgtcctttcctaataaaatgaggaaattgcatcgcattgtctgagta ggtgtcattctattctggggggtggggtggggcaggacagcaagggggaggattggga agagaatagcaggcatgctggggaaaggattctgatgtgtatatcacagacaaaactg tgctagacatgaggtctatggacttcaagagcaacagtgctgtggcctggagcaacaa atctgactttgcatgtgcaaacgccttcaacaacagcattattccagaagacaccttc ttccccagcccaggtaagggcagctttggtgccttcgcaggctgtttccttgcttcag gaatggccaggttctgcccagagctctggtcaatgatgtctaaaactcctctgattgg tggtctcggccttatccattgccaccaaaaccctctttttactaagaaacagtgagcc ttgttctggcagtccagagaatgacacgggaaaaaagcagatg | |
| 47 | tgatagcttgtgcctgtccctgagtcccagtccatcacgagcagctggtttctaagat gctatttcccgtataaagcatgagaccgtgacttgccagccccacagagccccgccct tgtccatcactggcatctggactccagcctgggttggggcaaagagggaaatgagatc atgtcctaaccctgatcctcttgtcccacagatatccagaaccctgaccctgccgtgt accagctgagagactctaaatccagtgacaagtctgtctgcctattcaccgattttga ttctcaaacaaatgtgtcacaaagtaaggattctgatgtgtatatcacagacaaaact gtgctagacatgaggtctatggacttcaagagcaacagtgctgtggcctggagcGGAT CCGGAGCCACCAACTTCAGCCTGCTGAAGCAGGCCGGCGACGTGGAAGAAAATCCTGG GCCCATGGCTCTTCCTGTGACTGCCCTTCTGCTGCCCCTGGCTCTCCTGCTCCATGCT GCCCGGCCAGACATCCAGATGACACAGACAACCAGCAGCCTCAGCGCCAGCCTGGGGG ACAGAGTGACCATTAGCTGCCGGGCCTCTCAGGACATCAGCAAATACCTGAACTGGTA CCAGCAGAAACCAGATGGCACTGTCAAGCTGCTGATTTACCACACCTCCAGGCTCCAC AGCGGCGTGCCCAGTCGCTTCAGCGGCAGTGGGAGCGGGACAGATTATTCCCTCACAA TCTCCAACCTGGAGCAGGAAGATATTGCCACATACTTCTGCCAGCAAGGCAACACCCT GCCATACACATTTGGAGGCGGCACCAAATTGGAGATCACCGGCGGTGGTGGATCTGGA GGAGGGGGCAGCGGAGGTGGCGGCTCTGAGGTGAAACTGCAGGAGAGTGGCCCTGGCC TGGTGGCTCCCAGCCAGAGCCTTTCTGTCACCTGCACCGTGTCTGGGGTGTCCCTGCC TGACTATGGAGTCTCCTGGATCCGGCAGCCTCCAAGAAAAGGACTGGAATGGCTGGGC GTCATCTGGGGAAGTGAGACCACCTACTATAATTCAGCCCTCAAGTCCCGGCTCACCA TCATTAAGGACAACTCCAAATCCCAGGTGTTCCTGAAGATGAATTCTCTCCAGACTGA TGACACAGCCATCTACTACTGTGCCAAGCATTATTATTACGGCGGGTCCTATGCCATG GACTACTGGGGCCAGGGGACCAGTGTCACTGTTTCTTCTGAAAGCAAGTATGGCCCCC CATGCCCTCCCTGCCCCGGACAGCCGCGGGAGCCCCAGGTCTACACCCTCCCACCCTC CAGAGATGAGCTGACCAAGAACCAGGTTTCACTGACATGCCTGGTGAAGGGCTTCTAC CCCTCTGACATTGCTGTGGAGTGGGAGAGCAATGGGCAGCCAGAGAACAACTACAAGA CCACACCTCCTGTCCTGGACAGCGACGGCTCCTTCTTCCTCTACAGTAAGCTCACTGT GGACAAGAGCCGCTGGCAGCAGGGCAATGTCTTCTCCTGCAGCGTGATGCACGAGGCC CTGCACAATGCCTATACCCAGAAGTCACTCTCTCTGAGCCCTGGAAAGAAGGATCCCA AGTTTTGGGTCCTCGTGGTGGTCGGGGGTGTGCTGGCCTGCTACAGCTTGCTGGTCAC AGTGGCCTTCATCATCTTCTGGGTGCGCTCCAAGAGGAGCCGGCTGCTTCACAGTGAT TACATGAACATGACCCCCAGGAGGCCAGGACCCACCAGGAAGCACTACCAGCCCTACG CTCCCCCGCGGGACTTTGCTGCTTACCGCAGCAGGGTCAAATTTTCTAGATCTGCAGA TGCGCCGGCCTATCAAcaaggccagaaccagctcTATAACGAGCTCAATCTAGGACGA AGAGAGGAGTACGATGTTTTGGACAAGAGACGTGGCCGGGACCCTGAGATGGGGGGAA AGCCGAGAAGGAAGAACCCTCAGGAAGGCCTGTACAATGAACTGCAGAAAGATAAGAT GGCGGAGGCCTACAGTGAGATTGGGATGAAAGGCGAGCGCCGGAGGGGCAAGGGGCAC GATGGCCTTTACCAGGGTCTCAGTACAGCCACCAAGGACACCTACGACGCCCTTCACA TGCAGGCCCTGCCCCCTCGCTAAtaagaattctaactagagctcgctgatcagcctcg actgtgccttctagttgccagccatctgttgtttgcccctcccccgtgccttccttga ccctggaaggtgccactcccactgtcctttcctaataaaatgaggaaattgcatcgca ttgtctgagtaggtgtcattctattctggggggtggggtggggcaggacagcaagggg gaggattgggaagagaatagcaggcatgctggggataagggcagctttggtgccttcg caggctgtttccttgcttcaggaatggccaggttctgcccagagctctggtcaatgat gtctaaaactcctctgattggtggtctcggccttatccattgccaccaaaaccctctt tttactaagaaacagtgagccttgttctggcagtccagagaatgacacgggaaaaaag cagatgaagagaaggtggcaggagagggcacgtggcccagcctcagtctctccaactg agttcctgcctgcctgcctttgctcagactgtttgccccttactgctcttctaggcct | HDRT9 (T3/T12) |
| | cattctaagccccttctccaagttgcctctccttatttctccctgtctgccaaaaaat ctttcccagctcactaagtcagtctcacgcagtcactc | |
| 48 | atctcatttgaccatcatttgacctattgtctccctgtgggtaggcctcagagccaca cacctcaggccaggagtaccattcatccagacgtgaacatcttcccgaggcttccaga gttcttggttcacaccggggctaacatggctgggcttctgctgcagtggcaggagctc tgtgcacagagaacagcctcatctgctcgccttgtttccacctcccctcccattgccc caggttctttggccccacagcggccacatctgccgttggtgccaataggttttccagg agctggttgaggtgggagggagggagagggttgtgatcaggctgaggcatggggattg gatatagtctccgtgtcatgatttatttggtcagtcagtcctagtgcGaccctggggt aatggggatgtgttctcgtcacGttgggcctggctgaccagctttatctcttggcaca gaggcaggcagcggcGCTACCAATTTTTCTTTGTTGAAGCAGGCCGGGGATGTGGAGG AAAATCCGGGGCCAATGGCTCTTCCTGTGACTGCCCTTCTGCTGCCCCTGGCTCTCCT GCTCCATGCTGCCCGGCCAGACATCCAGATGACACAGACAACCAGCAGCCTCAGCGCC AGCCTGGGGGACAGAGTGACCATTAGCTGCCGGGCCTCTCAGGACATCAGCAAATACC TGAACTGGTACCAGCAGAAACCAGATGGCACTGTCAAGCTGCTGATTTACCACACCTC CAGGCTCCACAGCGGCGTGCCCAGTCGCTTCAGCGGCAGTGGGAGCGGGACAGATTAT TCCCTCACAATCTCCAACCTGGAGCAGGAAGATATTGCCACATACTTCTGCCAGCAAG GCAACACCCTGCCATACACATTTGGAGGCGGCACCAAATTGGAGATCACCGGCGGTGG TGGATCTGGAGGAGGGGGCAGCGGAGGTGGCGGCTCTGAGGTGAAACTGCAGGAGAGT GGCCCTGGCCTGGTGGCTCCCAGCCAGAGCCTTTCTGTCACCTGCACCGTGTCTGGGG TGTCCCTGCCTGACTATGGAGTCTCCTGGATCCGGCAGCCTCCAAGAAAAGGACTGGA ATGGCTGGGCGTCATCTGGGGAAGTGAGACCACCTACTATAATTCAGCCCTCAAGTCC CGGCTCACCATCATTAAGGACAACTCCAAATCCCAGGTGTTCCTGAAGATGAATTCTC TCCAGACTGATGACACAGCCATCTACTACTGTGCCAAGCATTATTATTACGGCGGGTC CTATGCCATGGACTACTGGGGCCAGGGGACCAGTGTCACTGTTTCTTCTGAAAGCAAG TATGGCCCCCCATGCCCTCCCTGCCCCGGACAGCCGCGGGAGCCCCAGGTCTACACCC TCCCACCCTCCAGAGATGAGCTGACCAAGAACCAGGTTTCACTGACATGCCTGGTGAA GGGCTTCTACCCCTCTGACATTGCTGTGGAGTGGGAGAGCAATGGGCAGCCAGAGAAC AACTACAAGACCACACCTCCTGTCCTGGACAGCGACGGCTCCTTCTTCCTCTACAGTA AGCTCACTGTGGACAAGAGCCGCTGGCAGCAGGGCAATGTCTTCTCCTGCAGCGTGAT GCACGAGGCCCTGCACAATGCCTATACCCAGAAGTCACTCTCTCTGAGCCCTGGAAAG AAGGATCCCAAGTTTTGGGTCCTCGTGGTGGTCGGGGGTGTGCTGGCCTGCTACAGCT TGCTGGTCACAGTGGCCTTCATCATCTTCTGGGTGCGCTCCAAGAGGAGCCGGCTGCT TCACAGTGATTACATGAACATGACCCCCAGGAGGCCAGGACCCACCAGGAAGCACTAC CAGCCCTACGCTCCCCCGCGGGACTTTGCTGCTTACCGCAGCagagtgaaggtgggta ccactgggctttgggaggagggcacggggtcccccacttgatggatgttcagaggggc cttggtcttggaaggtctcaagctcgggtggtgcctggggcttggtatccaggagcaa agcaaggaccagccaagtgtgtgccttgagtgggctgaggaggaggtggcagtgtctg gctgagatggacagggtaggagggagagcctggtgctaggcacctccatgacaagccg tacaaatgtgtgcacatcagagtgtcccagggaaggcgatgctactggtgacaaaggg gcttacactcaggcagaggtccttctttccaagtgtgaatgaaggccatgttagcctt tctcttgaaaaggccctttcctcatctgtaactggggaG | HDRT17 (Z2/Z5) |
| 49 | atctcatttgaccatcatttgacctattgtctccctgtgggtaggcctcagagccaca cacctcaggccaggagtaccattcatccagacgtgaacatcttcccgaggcttccaga gttcttggttcacaccggggctaacatggctgggcttctgctgcagtggcaggagctc tgtgcacagagaacagcctcatctgctcgccttgtttccacctcccctcccattgccc caggttctttggccccacagcggccacatctgccgttggtgccaataggttttccagg agctggttgaggtgggagggagggagagggttgtgatcaggctgaggcatggggattg gatatagtctccgtgtcatgatttatttggtcagtcagtcctagtgcGaccctggggt aatggggatgtgttctcgtcacGttgggcctggctgaccagctttatctcttggcaca gaggcaggcagcggcGCTACCAATTTTTCTTTGTTGAAGCAGGCCGGGGATGTGGAGG AAAATCCGGGGCCAATGGCTCTTCCTGTGACTGCCCTTCTGCTGCCCCTGGCTCTCCT GCTCCATGCTGCCCGGCCAGACATCCAGATGACACAGACAACCAGCAGCCTCAGCGCC AGCCTGGGGGACAGAGTGACCATTAGCTGCCGGGCCTCTCAGGACATCAGCAAATACC TGAACTGGTACCAGCAGAAACCAGATGGCACTGTCAAGCTGCTGATTTACCACACCTC CAGGCTCCACAGCGGCGTGCCCAGTCGCTTCAGCGGCAGTGGGAGCGGGACAGATTAT TCCCTCACAATCTCCAACCTGGAGCAGGAAGATATTGCCACATACTTCTGCCAGCAAG GCAACACCCTGCCATACACATTTGGAGGCGGCACCAAATTGGAGATCACCGGCGGTGG TGGATCTGGAGGAGGGGGCAGCGGAGGTGGCGGCTCTGAGGTGAAACTGCAGGAGAGT GGCCCTGGCCTGGTGGCTCCCAGCCAGAGCCTTTCTGTCACCTGCACCGTGTCTGGGG TGTCCCTGCCTGACTATGGAGTCTCCTGGATCCGGCAGCCTCCAAGAAAAGGACTGGA ATGGCTGGGCGTCATCTGGGGAAGTGAGACCACCTACTATAATTCAGCCCTCAAGTCC | HDRT17 (Z2/Z6) |
| | CGGCTCACCATCATTAAGGACAACTCCAAATCCCAGGTGTTCCTGAAGATGAATTCTC TCCAGACTGATGACACAGCCATCTACTACTGTGCCAAGCATTATTATTACGGCGGGTC CTATGCCATGGACTACTGGGGCCAGGGGACCAGTGTCACTGTTTCTTCTGAAAGCAAG TATGGCCCCCCATGCCCTCCCTGCCCCGGACAGCCGCGGGAGCCCCAGGTCTACACCC TCCCACCCTCCAGAGATGAGCTGACCAAGAACCAGGTTTCACTGACATGCCTGGTGAA GGGCTTCTACCCCTCTGACATTGCTGTGGAGTGGGAGAGCAATGGGCAGCCAGAGAAC AACTACAAGACCACACCTCCTGTCCTGGACAGCGACGGCTCCTTCTTCCTCTACAGTA AGCTCACTGTGGACAAGAGCCGCTGGCAGCAGGGCAATGTCTTCTCCTGCAGCGTGAT GCACGAGGCCCTGCACAATGCCTATACCCAGAAGTCACTCTCTCTGAGCCCTGGAAAG AAGGATCCCAAGTTTTGGGTCCTCGTGGTGGTCGGGGGTGTGCTGGCCTGCTACAGCT TGCTGGTCACAGTGGCCTTCATCATCTTCTGGGTGCGCTCCAAGAGGAGCCGGCTGCT TCACAGTGATTACATGAACATGACCCCCAGGAGGCCAGGACCCACCAGGAAGCACTAC CAGCCCTACGCTCCCCCGCGGGACTTTGCTGCTTACCGCAGCagagtgaaggtgggta ccactgggctttgggaggagggcacggggtcccccacttgatggatgttcagaggggc cttggtcttggaaggtctcaagctcgggtggtgcctggggcttggtatccaggagcaa agcaaggaccagccaagtgtgtgccttgagtgggctgaggaggaggtggcagtgtctg gctgagatggacagggtaggagggagagcctggtgctaggcacctccatgacaagccg tacaaatgtgtgcacatcagagtgtcccagggaaggcgatgctactggtgacaaaggg gcttacactcaggcagaggtccttctttccaagtgtgaatgaaggccatgttagcctt tctcttgaaaaggccctttcctcatctgtaactggggaG | |
| 50 | tcttcccgaggcttccagagttcttggttcacaccggggctaacatggctgggcttct gctgcagtggcaggagctctgtgcacagagaacagcctcatctgctcgccttgtttcc acctcccctcccattgccccaggttctttggccccacagcggccacatctgccgttgg tgccaataggttttccaggagctggttgaggtgggagggagggagagggttgtgatca ggctgaggcatggggattggatatagtctccgtgtcatgatttatttggtcagtcagt cctagtgccaccctggggtaatggggatgtgttctcgtcaccttgggcctggctgacc agctttatctcttggcacagaggcacagagctttggcctgctggatcccaaaggcagc ggcGCTACCAATTTTTCTTTGTTGAAGCAGGCCGGGGATGTGGAGGAAAATCCGGGGC CAATGGCTCTTCCTGTGACTGCCCTTCTGCTGCCCCTGGCTCTCCTGCTCCATGCTGC CCGGCCAGACATCCAGATGACACAGACAACCAGCAGCCTCAGCGCCAGCCTGGGGGAC AGAGTGACCATTAGCTGCCGGGCCTCTCAGGACATCAGCAAATACCTGAACTGGTACC AGCAGAAACCAGATGGCACTGTCAAGCTGCTGATTTACCACACCTCCAGGCTCCACAG CGGCGTGCCCAGTCGCTTCAGCGGCAGTGGGAGCGGGACAGATTATTCCCTCACAATC TCCAACCTGGAGCAGGAAGATATTGCCACATACTTCTGCCAGCAAGGCAACACCCTGC CATACACATTTGGAGGCGGCACCAAATTGGAGATCACCGGCGGTGGTGGATCTGGAGG AGGGGGCAGCGGAGGTGGCGGCTCTGAGGTGAAACTGCAGGAGAGTGGCCCTGGCCTG GTGGCTCCCAGCCAGAGCCTTTCTGTCACCTGCACCGTGTCTGGGGTGTCCCTGCCTG ACTATGGAGTCTCCTGGATCCGGCAGCCTCCAAGAAAAGGACTGGAATGGCTGGGCGT CATCTGGGGAAGTGAGACCACCTACTATAATTCAGCCCTCAAGTCCCGGCTCACCATC ATTAAGGACAACTCCAAATCCCAGGTGTTCCTGAAGATGAATTCTCTCCAGACTGATG ACACAGCCATCTACTACTGTGCCAAGCATTATTATTACGGCGGGTCCTATGCCATGGA CTACTGGGGCCAGGGGACCAGTGTCACTGTTTCTTCTGAAAGCAAGTATGGCCCCCCA TGCCCTCCCTGCCCCGGACAGCCGCGGGAGCCCCAGGTCTACACCCTCCCACCCTCCA GAGATGAGCTGACCAAGAACCAGGTTTCACTGACATGCCTGGTGAAGGGCTTCTACCC CTCTGACATTGCTGTGGAGTGGGAGAGCAATGGGCAGCCAGAGAACAACTACAAGACC ACACCTCCTGTCCTGGACAGCGACGGCTCCTTCTTCCTCTACAGTAAGCTCACTGTGG ACAAGAGCCGCTGGCAGCAGGGCAATGTCTTCTCCTGCAGCGTGATGCACGAGGCCCT GCACAATGCCTATACCCAGAAGTCACTCTCTCTGAGCCCTGGAAAGAAGGATCCCAAG TTTTGGGTCCTCGTGGTGGTCGGGGGTGTGCTGGCCTGCTACAGCTTGCTGGTCACAG TGGCCTTCATCATCTTCTGGGTGCGCTCCAAGAGGAGCCGGCTGCTTCACAGTGATTA CATGAACATGACCCCCAGGAGGCCAGGACCCACCAGGAAGCACTACCAGCCCTACGCT CCCCCGCGGGACTTTGCTGCTTACCGCAGCagagtgaaggtgggtaccactgggcttt gggaggagggcacggggtcccccacttgatggatgttcagaggggccttggtcttgga aggtctcaagctcgggtggtgcctggggcttggtatccaggagcaaagcaaggaccag ccaagtgtgtgccttgagtgggctgaggaggaggtggcagtgtctggctgagatggac agggtaggagggagagcctggtgctaggcacctccatgacaagccgtacaaatgtgtg cacatcagagtgtcccagggaaggcgatgctactggtgacaaaggggcttacactcag gcagaggtccttctttccaagtgtgaatgaaggccatgttagcctttctcttgaaaag gccctttcctcatctgtaactggggaG | HDRT20 (Z3/Z5) |
| 51 | tcttcccgaggcttccagagttcttggttcacaccggggctaacatggctgggcttct gctgcagtggcaggagctctgtgcacagagaacagcctcatctgctcgccttgtttcc acctcccctcccattgccccaggttctttggccccacagcggccacatctgccgttgg | HDRT20 (Z3/Z6) |
| | tgccaataggttttccaggagctggttgaggtgggagggagggagagggttgtgatca ggctgaggcatggggattggatatagtctccgtgtcatgatttatttggtcagtcagt cctagtgccaccctggggtaatggggatgtgttctcgtcaccttgggcctggctgacc agctttatctcttggcacagaggcacagagctttggcctgctggatcccaaaggcagc ggcGCTACCAATTTTTCTTTGTTGAAGCAGGCCGGGGATGTGGAGGAAAATCCGGGGC CAATGGCTCTTCCTGTGACTGCCCTTCTGCTGCCCCTGGCTCTCCTGCTCCATGCTGC CCGGCCAGACATCCAGATGACACAGACAACCAGCAGCCTCAGCGCCAGCCTGGGGGAC AGAGTGACCATTAGCTGCCGGGCCTCTCAGGACATCAGCAAATACCTGAACTGGTACC AGCAGAAACCAGATGGCACTGTCAAGCTGCTGATTTACCACACCTCCAGGCTCCACAG CGGCGTGCCCAGTCGCTTCAGCGGCAGTGGGAGCGGGACAGATTATTCCCTCACAATC TCCAACCTGGAGCAGGAAGATATTGCCACATACTTCTGCCAGCAAGGCAACACCCTGC CATACACATTTGGAGGCGGCACCAAATTGGAGATCACCGGCGGTGGTGGATCTGGAGG AGGGGGCAGCGGAGGTGGCGGCTCTGAGGTGAAACTGCAGGAGAGTGGCCCTGGCCTG GTGGCTCCCAGCCAGAGCCTTTCTGTCACCTGCACCGTGTCTGGGGTGTCCCTGCCTG ACTATGGAGTCTCCTGGATCCGGCAGCCTCCAAGAAAAGGACTGGAATGGCTGGGCGT CATCTGGGGAAGTGAGACCACCTACTATAATTCAGCCCTCAAGTCCCGGCTCACCATC ATTAAGGACAACTCCAAATCCCAGGTGTTCCTGAAGATGAATTCTCTCCAGACTGATG ACACAGCCATCTACTACTGTGCCAAGCATTATTATTACGGCGGGTCCTATGCCATGGA CTACTGGGGCCAGGGGACCAGTGTCACTGTTTCTTCTGAAAGCAAGTATGGCCCCCCA TGCCCTCCCTGCCCCGGACAGCCGCGGGAGCCCCAGGTCTACACCCTCCCACCCTCCA GAGATGAGCTGACCAAGAACCAGGTTTCACTGACATGCCTGGTGAAGGGCTTCTACCC CTCTGACATTGCTGTGGAGTGGGAGAGCAATGGGCAGCCAGAGAACAACTACAAGACC ACACCTCCTGTCCTGGACAGCGACGGCTCCTTCTTCCTCTACAGTAAGCTCACTGTGG ACAAGAGCCGCTGGCAGCAGGGCAATGTCTTCTCCTGCAGCGTGATGCACGAGGCCCT GCACAATGCCTATACCCAGAAGTCACTCTCTCTGAGCCCTGGAAAGAAGGATCCCAAG TTTTGGGTCCTCGTGGTGGTCGGGGGTGTGCTGGCCTGCTACAGCTTGCTGGTCACAG TGGCCTTCATCATCTTCTGGGTGCGCTCCAAGAGGAGCCGGCTGCTTCACAGTGATTA CATGAACATGACCCCCAGGAGGCCAGGACCCACCAGGAAGCACTACCAGCCCTACGCT CCCCCGCGGGACTTTGCTGCTTACCGCAGCagagtgaaggtgggtaccactgggcttt gggaggagggcacggggtcccccacttgatggatgttcagaggggccttggtcttgga aggtctcaagctcgggtggtgcctggggcttggtatccaggagcaaagcaaggaccag ccaagtgtgtgccttgagtgggctgaggaggaggtggcagtgtctggctgagatggac agggtaggagggagagcctggtgctaggcacctccatgacaagccgtacaaatgtgtg cacatcagagtgtcccagggaaggcgatgctactggtgacaaaggggcttacactcag gcagaggtccttctttccaagtgtgaatgaaggccatgttagcctttctcttgaaaag gccctttcctcatctgtaactggggaG | |
| 52 | tcttcccgaggcttccagagttcttggttcacaccggggctaacatggctgggcttct gctgcagtggcaggagctctgtgcacagagaacagcctcatctgctcgccttgtttcc acctcccctcccattgccccaggttctttggccccacagcggccacatctgccgttgg tgccaataggttttccaggagctggttgaggtgggagggagggagagggttgtgatca ggctgaggcatggggattggatatagtctccgtgtcatgatttatttggtcagtcagt cctagtgccaccctggggtaatggggatgtgttctcgtcaccttgggcctggctgacc agctttatctcttggcacagaggcacagagctttggcctgctggatcccaaaggcagc ggcGCTACCAATTTTTCTTTGTTGAAGCAGGCCGGGGATGTGGAGGAAAATCCGGGGC CAATGGCTCTTCCTGTGACTGCCCTTCTGCTGCCCCTGGCTCTCCTGCTCCATGCTGC CCGGCCAGACATCCAGATGACACAGACAACCAGCAGCCTCAGCGCCAGCCTGGGGGAC AGAGTGACCATTAGCTGCCGGGCCTCTCAGGACATCAGCAAATACCTGAACTGGTACC AGCAGAAACCAGATGGCACTGTCAAGCTGCTGATTTACCACACCTCCAGGCTCCACAG CGGCGTGCCCAGTCGCTTCAGCGGCAGTGGGAGCGGGACAGATTATTCCCTCACAATC TCCAACCTGGAGCAGGAAGATATTGCCACATACTTCTGCCAGCAAGGCAACACCCTGC CATACACATTTGGAGGCGGCACCAAATTGGAGATCACCGGCGGTGGTGGATCTGGAGG AGGGGGCAGCGGAGGTGGCGGCTCTGAGGTGAAACTGCAGGAGAGTGGCCCTGGCCTG GTGGCTCCCAGCCAGAGCCTTTCTGTCACCTGCACCGTGTCTGGGGTGTCCCTGCCTG ACTATGGAGTCTCCTGGATCCGGCAGCCTCCAAGAAAAGGACTGGAATGGCTGGGCGT CATCTGGGGAAGTGAGACCACCTACTATAATTCAGCCCTCAAGTCCCGGCTCACCATC ATTAAGGACAACTCCAAATCCCAGGTGTTCCTGAAGATGAATTCTCTCCAGACTGATG ACACAGCCATCTACTACTGTGCCAAGCATTATTATTACGGCGGGTCCTATGCCATGGA CTACTGGGGCCAGGGGACCAGTGTCACTGTTTCTTCTGAAAGCAAGTATGGCCCCCCA TGCCCTCCCTGCCCCGGACAGCCGCGGGAGCCCCAGGTCTACACCCTCCCACCCTCCA GAGATGAGCTGACCAAGAACCAGGTTTCACTGACATGCCTGGTGAAGGGCTTCTACCC CTCTGACATTGCTGTGGAGTGGGAGAGCAATGGGCAGCCAGAGAACAACTACAAGACC ACACCTCCTGTCCTGGACAGCGACGGCTCCTTCTTCCTCTACAGTAAGCTCACTGTGG | HDRT21 (Z3/Z7) |
| | ACAAGAGCCGCTGGCAGCAGGGCAATGTCTTCTCCTGCAGCGTGATGCACGAGGCCCT GCACAATGCCTATACCCAGAAGTCACTCTCTCTGAGCCCTGGAAAGAAGGATCCCAAG TTTTGGGTCCTCGTGGTGGTCGGGGGTGTGCTGGCCTGCTACAGCTTGCTGGTCACAG TGGCCTTCATCATCTTCTGGGTGCGCTCCAAGAGGAGCCGGCTGCTTCACAGTGATTA CATGAACATGACCCCCAGGAGGCCAGGACCCACCAGGAAGCACTACCAGCCCTACGCT CCCCCGCGGGACTTTGCTGCTTACCGCAGCagagtgaaggtgggtaccactgCgcttt gCgaggagggcacggggtcccccacttgatggatgttcagaggggccttggtcttgga aggtctcaagctcgggtggtgcctggggcttggtatccaggagcaaagcaaggaccag ccaagtgtgtgccttgagtgggctgaggaggaggtggcagtgtctggctgagatggac agggtaggagggagagcctggtgctaggcacctccatgacaagccgtacaaatgtgtg cacatcagagtgtcccagggaaggcgatgctactggtgacaaaggggcttacactcag gcagaggtccttctttccaagtgtgaatgaaggccatgttagcctttctcttgaaaag gccctttcctcatctgtaactggggaG | |
| 53 | tcttcccgaggcttccagagttcttggttcacaccggggctaacatggctgggcttct gctgcagtggcaggagctctgtgcacagagaacagcctcatctgctcgccttgtttcc acctcccctcccattgccccaggttctttggccccacagcggccacatctgccgttgg tgccaataggttttccaggagctggttgaggtgggagggagggagagggttgtgatca ggctgaggcatggggattggatatagtctccgtgtcatgatttatttggtcagtcagt cctagtgccaccctggggtaatggggatgtgttctcgtcaccttgggcctggctgacc agctttatctcttggcacagaggcacagagctttggcctgctggatcccaaaggcagc ggcGCTACCAATTTTTCTTTGTTGAAGCAGGCCGGGGATGTGGAGGAAAATCCGGGGC CAATGGCTCTTCCTGTGACTGCCCTTCTGCTGCCCCTGGCTCTCCTGCTCCATGCTGC CCGGCCAGACATCCAGATGACACAGACAACCAGCAGCCTCAGCGCCAGCCTGGGGGAC AGAGTGACCATTAGCTGCCGGGCCTCTCAGGACATCAGCAAATACCTGAACTGGTACC AGCAGAAACCAGATGGCACTGTCAAGCTGCTGATTTACCACACCTCCAGGCTCCACAG CGGCGTGCCCAGTCGCTTCAGCGGCAGTGGGAGCGGGACAGATTATTCCCTCACAATC TCCAACCTGGAGCAGGAAGATATTGCCACATACTTCTGCCAGCAAGGCAACACCCTGC CATACACATTTGGAGGCGGCACCAAATTGGAGATCACCGGCGGTGGTGGATCTGGAGG AGGGGGCAGCGGAGGTGGCGGCTCTGAGGTGAAACTGCAGGAGAGTGGCCCTGGCCTG GTGGCTCCCAGCCAGAGCCTTTCTGTCACCTGCACCGTGTCTGGGGTGTCCCTGCCTG ACTATGGAGTCTCCTGGATCCGGCAGCCTCCAAGAAAAGGACTGGAATGGCTGGGCGT CATCTGGGGAAGTGAGACCACCTACTATAATTCAGCCCTCAAGTCCCGGCTCACCATC ATTAAGGACAACTCCAAATCCCAGGTGTTCCTGAAGATGAATTCTCTCCAGACTGATG ACACAGCCATCTACTACTGTGCCAAGCATTATTATTACGGCGGGTCCTATGCCATGGA CTACTGGGGCCAGGGGACCAGTGTCACTGTTTCTTCTGAAAGCAAGTATGGCCCCCCA TGCCCTCCCTGCCCCGGACAGCCGCGGGAGCCCCAGGTCTACACCCTCCCACCCTCCA GAGATGAGCTGACCAAGAACCAGGTTTCACTGACATGCCTGGTGAAGGGCTTCTACCC CTCTGACATTGCTGTGGAGTGGGAGAGCAATGGGCAGCCAGAGAACAACTACAAGACC ACACCTCCTGTCCTGGACAGCGACGGCTCCTTCTTCCTCTACAGTAAGCTCACTGTGG ACAAGAGCCGCTGGCAGCAGGGCAATGTCTTCTCCTGCAGCGTGATGCACGAGGCCCT GCACAATGCCTATACCCAGAAGTCACTCTCTCTGAGCCCTGGAAAGAAGGATCCCAAG TTTTGGGTCCTCGTGGTGGTCGGGGGTGTGCTGGCCTGCTACAGCTTGCTGGTCACAG TGGCCTTCATCATCTTCTGGGTGCGCTCCAAGAGGAGCCGGCTGCTTCACAGTGATTA CATGAACATGACCCCCAGGAGGCCAGGACCCACCAGGAAGCACTACCAGCCCTACGCT CCCCCGCGGGACTTTGCTGCTTACCGCAGCagagtgaaggtgggtaccactgCgcttt gCgaggagggcacggggtcccccacttgatggatgttcagaggggccttggtcttgga aggtctcaagctcgggtggtgcctggggcttggtatccaggagcaaagcaaggaccag ccaagtgtgtgccttgagtgggctgaggaggaggtggcagtgtctggctgagatggac agggtaggagggagagcctggtgctaggcacctccatgacaagccgtacaaatgtgtg cacatcagagtgtcccagggaaggcgatgctactggtgacaaaggggcttacactcag gcagaggtccttctttccaagtgtgaatgaaggccatgttagcctttctcttgaaaag gccctttcctcatctgtaactggggaG | HDRT21 (Z3/Z8) |
| 54 | acaccggggctaacatggctgggcttctgctgcagtggcaggagctctgtgcacagag aacagcctcatctgctcgccttgtttccacctcccctcccattgccccaggttctttg gccccacagcggccacatctgccgttggtgccaataggttttccaggagctggttgag gtgggagggagggagagggttgtgatcaggctgaggcatggggattggatatagtctc cgtgtcatgatttatttggtcagtcagtcctagtgccaccctggggtaatggggatgt gttctcgtcaccttgggcctggctgaccagctttatctcttggcacagaggcacagag ctttggcctgctggatcTcaaactctgctacctgctggatggaatcctcttcggcagc ggcGCTACCAATTTTTCTTTGTTGAAGCAGGCCGGGGATGTGGAGGAAAATCCGGGGC CAATGGCTCTTCCTGTGACTGCCCTTCTGCTGCCCCTGGCTCTCCTGCTCCATGCTGC CCGGCCAGACATCCAGATGACACAGACAACCAGCAGCCTCAGCGCCAGCCTGGGGGAC | HDRT22 (Z3/Z5) |
| | AGAGTGACCATTAGCTGCCGGGCCTCTCAGGACATCAGCAAATACCTGAACTGGTACC AGCAGAAACCAGATGGCACTGTCAAGCTGCTGATTTACCACACCTCCAGGCTCCACAG CGGCGTGCCCAGTCGCTTCAGCGGCAGTGGGAGCGGGACAGATTATTCCCTCACAATC TCCAACCTGGAGCAGGAAGATATTGCCACATACTTCTGCCAGCAAGGCAACACCCTGC CATACACATTTGGAGGCGGCACCAAATTGGAGATCACCGGCGGTGGTGGATCTGGAGG AGGGGGCAGCGGAGGTGGCGGCTCTGAGGTGAAACTGCAGGAGAGTGGCCCTGGCCTG GTGGCTCCCAGCCAGAGCCTTTCTGTCACCTGCACCGTGTCTGGGGTGTCCCTGCCTG ACTATGGAGTCTCCTGGATCCGGCAGCCTCCAAGAAAAGGACTGGAATGGCTGGGCGT CATCTGGGGAAGTGAGACCACCTACTATAATTCAGCCCTCAAGTCCCGGCTCACCATC ATTAAGGACAACTCCAAATCCCAGGTGTTCCTGAAGATGAATTCTCTCCAGACTGATG ACACAGCCATCTACTACTGTGCCAAGCATTATTATTACGGCGGGTCCTATGCCATGGA CTACTGGGGCCAGGGGACCAGTGTCACTGTTTCTTCTGAAAGCAAGTATGGCCCCCCA TGCCCTCCCTGCCCCGGACAGCCGCGGGAGCCCCAGGTCTACACCCTCCCACCCTCCA GAGATGAGCTGACCAAGAACCAGGTTTCACTGACATGCCTGGTGAAGGGCTTCTACCC CTCTGACATTGCTGTGGAGTGGGAGAGCAATGGGCAGCCAGAGAACAACTACAAGACC ACACCTCCTGTCCTGGACAGCGACGGCTCCTTCTTCCTCTACAGTAAGCTCACTGTGG ACAAGAGCCGCTGGCAGCAGGGCAATGTCTTCTCCTGCAGCGTGATGCACGAGGCCCT GCACAATGCCTATACCCAGAAGTCACTCTCTCTGAGCCCTGGAAAGAAGGATCCCAAG TTTTGGGTCCTCGTGGTGGTCGGGGGTGTGCTGGCCTGCTACAGCTTGCTGGTCACAG TGGCCTTCATCATCTTCTGGGTGCGCTCCAAGAGGAGCCGGCTGCTTCACAGTGATTA CATGAACATGACCCCCAGGAGGCCAGGACCCACCAGGAAGCACTACCAGCCCTACGCT CCCCCGCGGGACTTTGCTGCTTACCGCAGCagagtgaaggtgggtaccactgggcttt gggaggagggcacggggtcccccacttgatggatgttcagaggggccttggtcttgga aggtctcaagctcgggtggtgcctggggcttggtatccaggagcaaagcaaggaccag ccaagtgtgtgccttgagtgggctgaggaggaggtggcagtgtctggctgagatggac agggtaggagggagagcctggtgctaggcacctccatgacaagccgtacaaatgtgtg cacatcagagtgtcccagggaaggcgatgctactggtgacaaaggggcttacactcag gcagaggtccttctttccaagtgtgaatgaaggccatgttagcctttctcttgaaaag gccctttcctcatctgtaactggggaG | |
| 55 | acaccggggctaacatggctgggcttctgctgcagtggcaggagctctgtgcacagag aacagcctcatctgctcgccttgtttccacctcccctcccattgccccaggttctttg gccccacagcggccacatctgccgttggtgccaataggttttccaggagctggttgag gtgggagggagggagagggttgtgatcaggctgaggcatggggattggatatagtctc cgtgtcatgatttatttggtcagtcagtcctagtgccaccctggggtaatggggatgt gttctcgtcaccttgggcctggctgaccagctttatctcttggcacagaggcacagag ctttggcctgctggatcTcaaactctgctacctgctggatggaatcctcttcggcagc ggcGCTACCAATTTTTCTTTGTTGAAGCAGGCCGGGGATGTGGAGGAAAATCCGGGGC CAATGGCTCTTCCTGTGACTGCCCTTCTGCTGCCCCTGGCTCTCCTGCTCCATGCTGC CCGGCCAGACATCCAGATGACACAGACAACCAGCAGCCTCAGCGCCAGCCTGGGGGAC AGAGTGACCATTAGCTGCCGGGCCTCTCAGGACATCAGCAAATACCTGAACTGGTACC AGCAGAAACCAGATGGCACTGTCAAGCTGCTGATTTACCACACCTCCAGGCTCCACAG CGGCGTGCCCAGTCGCTTCAGCGGCAGTGGGAGCGGGACAGATTATTCCCTCACAATC TCCAACCTGGAGCAGGAAGATATTGCCACATACTTCTGCCAGCAAGGCAACACCCTGC CATACACATTTGGAGGCGGCACCAAATTGGAGATCACCGGCGGTGGTGGATCTGGAGG AGGGGGCAGCGGAGGTGGCGGCTCTGAGGTGAAACTGCAGGAGAGTGGCCCTGGCCTG GTGGCTCCCAGCCAGAGCCTTTCTGTCACCTGCACCGTGTCTGGGGTGTCCCTGCCTG ACTATGGAGTCTCCTGGATCCGGCAGCCTCCAAGAAAAGGACTGGAATGGCTGGGCGT CATCTGGGGAAGTGAGACCACCTACTATAATTCAGCCCTCAAGTCCCGGCTCACCATC ATTAAGGACAACTCCAAATCCCAGGTGTTCCTGAAGATGAATTCTCTCCAGACTGATG ACACAGCCATCTACTACTGTGCCAAGCATTATTATTACGGCGGGTCCTATGCCATGGA CTACTGGGGCCAGGGGACCAGTGTCACTGTTTCTTCTGAAAGCAAGTATGGCCCCCCA TGCCCTCCCTGCCCCGGACAGCCGCGGGAGCCCCAGGTCTACACCCTCCCACCCTCCA GAGATGAGCTGACCAAGAACCAGGTTTCACTGACATGCCTGGTGAAGGGCTTCTACCC CTCTGACATTGCTGTGGAGTGGGAGAGCAATGGGCAGCCAGAGAACAACTACAAGACC ACACCTCCTGTCCTGGACAGCGACGGCTCCTTCTTCCTCTACAGTAAGCTCACTGTGG ACAAGAGCCGCTGGCAGCAGGGCAATGTCTTCTCCTGCAGCGTGATGCACGAGGCCCT GCACAATGCCTATACCCAGAAGTCACTCTCTCTGAGCCCTGGAAAGAAGGATCCCAAG TTTTGGGTCCTCGTGGTGGTCGGGGGTGTGCTGGCCTGCTACAGCTTGCTGGTCACAG TGGCCTTCATCATCTTCTGGGTGCGCTCCAAGAGGAGCCGGCTGCTTCACAGTGATTA CATGAACATGACCCCCAGGAGGCCAGGACCCACCAGGAAGCACTACCAGCCCTACGCT CCCCCGCGGGACTTTGCTGCTTACCGCAGCagagtgaaggtgggtaccactgggcttt gggaggagggcacggggtcccccacttgatggatgttcagaggggccttggtcttgga | HDRT22 (Z3/Z6) |
| | aggtctcaagctcgggtggtgcctggggcttggtatccaggagcaaagcaaggaccag ccaagtgtgtgccttgagtgggctgaggaggaggtggcagtgtctggctgagatggac agggtaggagggagagcctggtgctaggcacctccatgacaagccgtacaaatgtgtg cacatcagagtgtcccagggaaggcgatgctactggtgacaaaggggcttacactcag gcagaggtccttctttccaagtgtgaatgaaggccatgttagcctttctcttgaaaag gccctttcctcatctgtaactggggaG | |
| 56 | atctcatttgaccatcatttgacctattgtctccctgtgggtaggcctcagagccaca cacctcaggccaggagtaccattcatccagacgtgaacatcttcccgaggcttccaga gttcttggttcacaccggggctaacatggctgggcttctgctgcagtggcaggagctc tgtgcacagagaacagcctcatctgctcgccttgtttccacctcccctcccattgccc caggttctttggccccacagcggccacatctgccgttggtgccaataggttttccagg agctggttgaggtgggagggagggagagggttgtgatcaggctgaggcatggggattg gatatagtctccgtgtcatgatttatttggtcagtcagtcctagtgcGaccctggggt aatggggatgtgttctcgtcacGttgggcctggctgaccagctttatctcttggcaca gaggcaggcagcggcGCTACCAATTTTTCTTTGTTGAAGCAGGCCGGGGATGTGGAGG AAAATCCGGGGCCAATGGCTCTTCCTGTGACTGCCCTTCTGCTGCCCCTGGCTCTCCT GCTCCATGCTGCCCGGCCAGACATCCAGATGACACAGACAACCAGCAGCCTCAGCGCC AGCCTGGGGGACAGAGTGACCATTAGCTGCCGGGCCTCTCAGGACATCAGCAAATACC TGAACTGGTACCAGCAGAAACCAGATGGCACTGTCAAGCTGCTGATTTACCACACCTC CAGGCTCCACAGCGGCGTGCCCAGTCGCTTCAGCGGCAGTGGGAGCGGGACAGATTAT TCCCTCACAATCTCCAACCTGGAGCAGGAAGATATTGCCACATACTTCTGCCAGCAAG GCAACACCCTGCCATACACATTTGGAGGCGGCACCAAATTGGAGATCACCGGCGGTGG TGGATCTGGAGGAGGGGGCAGCGGAGGTGGCGGCTCTGAGGTGAAACTGCAGGAGAGT GGCCCTGGCCTGGTGGCTCCCAGCCAGAGCCTTTCTGTCACCTGCACCGTGTCTGGGG TGTCCCTGCCTGACTATGGAGTCTCCTGGATCCGGCAGCCTCCAAGAAAAGGACTGGA ATGGCTGGGCGTCATCTGGGGAAGTGAGACCACCTACTATAATTCAGCCCTCAAGTCC CGGCTCACCATCATTAAGGACAACTCCAAATCCCAGGTGTTCCTGAAGATGAATTCTC TCCAGACTGATGACACAGCCATCTACTACTGTGCCAAGCATTATTATTACGGCGGGTC CTATGCCATGGACTACTGGGGCCAGGGGACCAGTGTCACTGTTTCTTCTGAAAGCAAG TATGGCCCCCCATGCCCTCCCTGCCCCGGACAGCCGCGGGAGCCCCAGGTCTACACCC TCCCACCCTCCAGAGATGAGCTGACCAAGAACCAGGTTTCACTGACATGCCTGGTGAA GGGCTTCTACCCCTCTGACATTGCTGTGGAGTGGGAGAGCAATGGGCAGCCAGAGAAC AACTACAAGACCACACCTCCTGTCCTGGACAGCGACGGCTCCTTCTTCCTCTACAGTA AGCTCACTGTGGACAAGAGCCGCTGGCAGCAGGGCAATGTCTTCTCCTGCAGCGTGAT GCACGAGGCCCTGCACAATGCCTATACCCAGAAGTCACTCTCTCTGAGCCCTGGAAAG AAGGATCCCAAGTTTTGGGTCCTCGTGGTGGTCGGGGGTGTGCTGGCCTGCTACAGCT TGCTGGTCACAGTGGCCTTCATCATCTTCTGGGTGCGCTCCAAGAGGAGCCGGCTGCT TCACAGTGATTACATGAACATGACCCCCAGGAGGCCAGGACCCACCAGGAAGCACTAC CAGCCCTACGCTCCCCCGCGGGACTTTGCTGCTTACCGCAGCagagtgaaggtgggta ccactgCgctttgCgaggagggcacggggtcccccacttgatggatgttcagaggggc cttggtcttggaaggtctcaagctcgggtggtgcctggggcttggtatccaggagcaa agcaaggaccagccaagtgtgtgccttgagtgggctgaggaggaggtggcagtgtctg gctgagatggacagggtaggagggagagcctggtgctaggcacctccatgacaagccg tacaaatgtgtgcacatcagagtgtcccagggaaggcgatgctactggtgacaaaggg gcttacactcaggcagaggtccttctttccaagtgtgaatgaaggccatgttagcctt tctcttgaaaaggccctttcctcatctgtaactggggaG | HDRT23 (Z3/Z7) |
| 57 | atctcatttgaccatcatttgacctattgtctccctgtgggtaggcctcagagccaca cacctcaggccaggagtaccattcatccagacgtgaacatcttcccgaggcttccaga gttcttggttcacaccggggctaacatggctgggcttctgctgcagtggcaggagctc tgtgcacagagaacagcctcatctgctcgccttgtttccacctcccctcccattgccc caggttctttggccccacagcggccacatctgccgttggtgccaataggttttccagg agctggttgaggtgggagggagggagagggttgtgatcaggctgaggcatggggattg gatatagtctccgtgtcatgatttatttggtcagtcagtcctagtgcGaccctggggt aatggggatgtgttctcgtcacGttgggcctggctgaccagctttatctcttggcaca gaggcaggcagcggcGCTACCAATTTTTCTTTGTTGAAGCAGGCCGGGGATGTGGAGG AAAATCCGGGGCCAATGGCTCTTCCTGTGACTGCCCTTCTGCTGCCCCTGGCTCTCCT GCTCCATGCTGCCCGGCCAGACATCCAGATGACACAGACAACCAGCAGCCTCAGCGCC AGCCTGGGGGACAGAGTGACCATTAGCTGCCGGGCCTCTCAGGACATCAGCAAATACC TGAACTGGTACCAGCAGAAACCAGATGGCACTGTCAAGCTGCTGATTTACCACACCTC CAGGCTCCACAGCGGCGTGCCCAGTCGCTTCAGCGGCAGTGGGAGCGGGACAGATTAT TCCCTCACAATCTCCAACCTGGAGCAGGAAGATATTGCCACATACTTCTGCCAGCAAG GCAACACCCTGCCATACACATTTGGAGGCGGCACCAAATTGGAGATCACCGGCGGTGG | HDRT23 (Z3/Z8) |
| | TGGATCTGGAGGAGGGGGCAGCGGAGGTGGCGGCTCTGAGGTGAAACTGCAGGAGAGT GGCCCTGGCCTGGTGGCTCCCAGCCAGAGCCTTTCTGTCACCTGCACCGTGTCTGGGG TGTCCCTGCCTGACTATGGAGTCTCCTGGATCCGGCAGCCTCCAAGAAAAGGACTGGA ATGGCTGGGCGTCATCTGGGGAAGTGAGACCACCTACTATAATTCAGCCCTCAAGTCC CGGCTCACCATCATTAAGGACAACTCCAAATCCCAGGTGTTCCTGAAGATGAATTCTC TCCAGACTGATGACACAGCCATCTACTACTGTGCCAAGCATTATTATTACGGCGGGTC CTATGCCATGGACTACTGGGGCCAGGGGACCAGTGTCACTGTTTCTTCTGAAAGCAAG TATGGCCCCCCATGCCCTCCCTGCCCCGGACAGCCGCGGGAGCCCCAGGTCTACACCC TCCCACCCTCCAGAGATGAGCTGACCAAGAACCAGGTTTCACTGACATGCCTGGTGAA GGGCTTCTACCCCTCTGACATTGCTGTGGAGTGGGAGAGCAATGGGCAGCCAGAGAAC AACTACAAGACCACACCTCCTGTCCTGGACAGCGACGGCTCCTTCTTCCTCTACAGTA AGCTCACTGTGGACAAGAGCCGCTGGCAGCAGGGCAATGTCTTCTCCTGCAGCGTGAT GCACGAGGCCCTGCACAATGCCTATACCCAGAAGTCACTCTCTCTGAGCCCTGGAAAG AAGGATCCCAAGTTTTGGGTCCTCGTGGTGGTCGGGGGTGTGCTGGCCTGCTACAGCT TGCTGGTCACAGTGGCCTTCATCATCTTCTGGGTGCGCTCCAAGAGGAGCCGGCTGCT TCACAGTGATTACATGAACATGACCCCCAGGAGGCCAGGACCCACCAGGAAGCACTAC CAGCCCTACGCTCCCCCGCGGGACTTTGCTGCTTACCGCAGCagagtgaaggtgggta ccactgCgctttgCgaggagggcacggggtcccccacttgatggatgttcagaggggc cttggtcttggaaggtctcaagctcgggtggtgcctggggcttggtatccaggagcaa agcaaggaccagccaagtgtgtgccttgagtgggctgaggaggaggtggcagtgtctg gctgagatggacagggtaggagggagagcctggtgctaggcacctccatgacaagccg tacaaatgtgtgcacatcagagtgtcccagggaaggcgatgctactggtgacaaaggg gcttacactcaggcagaggtccttctttccaagtgtgaatgaaggccatgttagcctt tctcttgaaaaggccctttcctcatctgtaactggggaG | |
| 58 | acaccggggctaacatggctgggcttctgctgcagtggcaggagctctgtgcacagag aacagcctcatctgctcgccttgtttccacctcccctcccattgccccaggttctttg gccccacagcggccacatctgccgttggtgccaataggttttccaggagctggttgag gtgggagggagggagagggttgtgatcaggctgaggcatggggattggatatagtctc cgtgtcatgatttatttggtcagtcagtcctagtgccaccctggggtaatggggatgt gttctcgtcaccttgggcctggctgaccagctttatctcttggcacagaggcacagag ctttggcctgctggatcccaaactctgctacctgctggatggaatcctcttcggcagc ggcGCTACCAATTTTTCTTTGTTGAAGCAGGCCGGGGATGTGGAGGAAAATCCGGGGC CAATGGCTCTTCCTGTGACTGCCCTTCTGCTGCCCCTGGCTCTCCTGCTCCATGCTGC CCGGCCAGACATCCAGATGACACAGACAACCAGCAGCCTCAGCGCCAGCCTGGGGGAC AGAGTGACCATTAGCTGCCGGGCCTCTCAGGACATCAGCAAATACCTGAACTGGTACC AGCAGAAACCAGATGGCACTGTCAAGCTGCTGATTTACCACACCTCCAGGCTCCACAG CGGCGTGCCCAGTCGCTTCAGCGGCAGTGGGAGCGGGACAGATTATTCCCTCACAATC TCCAACCTGGAGCAGGAAGATATTGCCACATACTTCTGCCAGCAAGGCAACACCCTGC CATACACATTTGGAGGCGGCACCAAATTGGAGATCACCGGCGGTGGTGGATCTGGAGG AGGGGGCAGCGGAGGTGGCGGCTCTGAGGTGAAACTGCAGGAGAGTGGCCCTGGCCTG GTGGCTCCCAGCCAGAGCCTTTCTGTCACCTGCACCGTGTCTGGGGTGTCCCTGCCTG ACTATGGAGTCTCCTGGATCCGGCAGCCTCCAAGAAAAGGACTGGAATGGCTGGGCGT CATCTGGGGAAGTGAGACCACCTACTATAATTCAGCCCTCAAGTCCCGGCTCACCATC ATTAAGGACAACTCCAAATCCCAGGTGTTCCTGAAGATGAATTCTCTCCAGACTGATG ACACAGCCATCTACTACTGTGCCAAGCATTATTATTACGGCGGGTCCTATGCCATGGA CTACTGGGGCCAGGGGACCAGTGTCACTGTTTCTTCTGAAAGCAAGTATGGCCCCCCA TGCCCTCCCTGCCCCGGACAGCCGCGGGAGCCCCAGGTCTACACCCTCCCACCCTCCA GAGATGAGCTGACCAAGAACCAGGTTTCACTGACATGCCTGGTGAAGGGCTTCTACCC CTCTGACATTGCTGTGGAGTGGGAGAGCAATGGGCAGCCAGAGAACAACTACAAGACC ACACCTCCTGTCCTGGACAGCGACGGCTCCTTCTTCCTCTACAGTAAGCTCACTGTGG ACAAGAGCCGCTGGCAGCAGGGCAATGTCTTCTCCTGCAGCGTGATGCACGAGGCCCT GCACAATGCCTATACCCAGAAGTCACTCTCTCTGAGCCCTGGAAAGAAGGATCCCAAG TTTTGGGTCCTCGTGGTGGTCGGGGGTGTGCTGGCCTGCTACAGCTTGCTGGTCACAG TGGCCTTCATCATCTTCTGGGTGCGCTCCAAGAGGAGCCGGCTGCTTCACAGTGATTA CATGAACATGACCCCCAGGAGGCCAGGACCCACCAGGAAGCACTACCAGCCCTACGCT CCCCCGCGGGACTTTGCTGCTTACCGCAGCagagtgaaggtgggtaccactgggcttt gggaggagggcacggggtcccccacttgatggatgttcagaggggccttggtcttgga aggtctcaagctcgggtggtgcctggggcttggtatccaggagcaaagcaaggaccag ccaagtgtgtgccttgagtgggctgaggaggaggtggcagtgtctggctgagatggac agggtaggagggagagcctggtgctaggcacctccatgacaagccgtacaaatgtgtg cacatcagagtgtcccagggaaggcgatgctactggtgacaaaggggcttacactcag | HDRT24 (Z4/Z5) |
| | gcagaggtccttctttccaagtgtgaatgaaggccatgttagcctttctcttgaaaag gccctttcctcatctgtaactggggaG | |
| 59 | atctcatttgaccatcatttgacctattgtctccctgtgggtaggcctcagagccaca cacctcaggccaggagtaccattcatccagacgtgaacatcttcccgaggcttccaga gttcttggttcacaccggggctaacatggctgggcttctgctgcagtggcaggagctc tgtgcacagagaacagcctcatctgctcgccttgtttccacctcccctcccattgccc caggttctttggccccacagcggccacatctgccgttggtgccaataggttttccagg agctggttgaggtgggagggagggagagggttgtgatcaggctgaggcatggggattg gatatagtctccgtgtcatgatttatttggtcagtcagtcctagtgcGaccctggggt aatggggatgtgttctcgtcacGttgggcctggctgaccagctttatctcttggcaca gaggcaggcagcggcGCTACCAATTTTTCTTTGTTGAAGCAGGCCGGGGATGTGGAGG AAAATCCGGGGCCAATGGCTCTTCCTGTGACTGCCCTTCTGCTGCCCCTGGCTCTCCT GCTCCATGCTGCCCGGCCAGACATCCAGATGACACAGACAACCAGCAGCCTCAGCGCC AGCCTGGGGGACAGAGTGACCATTAGCTGCCGGGCCTCTCAGGACATCAGCAAATACC TGAACTGGTACCAGCAGAAACCAGATGGCACTGTCAAGCTGCTGATTTACCACACCTC CAGGCTCCACAGCGGCGTGCCCAGTCGCTTCAGCGGCAGTGGGAGCGGGACAGATTAT TCCCTCACAATCTCCAACCTGGAGCAGGAAGATATTGCCACATACTTCTGCCAGCAAG GCAACACCCTGCCATACACATTTGGAGGCGGCACCAAATTGGAGATCACCGGCGGTGG TGGATCTGGAGGAGGGGGCAGCGGAGGTGGCGGCTCTGAGGTGAAACTGCAGGAGAGT GGCCCTGGCCTGGTGGCTCCCAGCCAGAGCCTTTCTGTCACCTGCACCGTGTCTGGGG TGTCCCTGCCTGACTATGGAGTCTCCTGGATCCGGCAGCCTCCAAGAAAAGGACTGGA ATGGCTGGGCGTCATCTGGGGAAGTGAGACCACCTACTATAATTCAGCCCTCAAGTCC CGGCTCACCATCATTAAGGACAACTCCAAATCCCAGGTGTTCCTGAAGATGAATTCTC TCCAGACTGATGACACAGCCATCTACTACTGTGCCAAGCATTATTATTACGGCGGGTC CTATGCCATGGACTACTGGGGCCAGGGGACCAGTGTCACTGTTTCTTCTGAAAGCAAG TATGGCCCCCCATGCCCTCCCTGCCCCGGACAGCCGCGGGAGCCCCAGGTCTACACCC TCCCACCCTCCAGAGATGAGCTGACCAAGAACCAGGTTTCACTGACATGCCTGGTGAA GGGCTTCTACCCCTCTGACATTGCTGTGGAGTGGGAGAGCAATGGGCAGCCAGAGAAC AACTACAAGACCACACCTCCTGTCCTGGACAGCGACGGCTCCTTCTTCCTCTACAGTA AGCTCACTGTGGACAAGAGCCGCTGGCAGCAGGGCAATGTCTTCTCCTGCAGCGTGAT GCACGAGGCCCTGCACAATGCCTATACCCAGAAGTCACTCTCTCTGAGCCCTGGAAAG AAGGATCCCAAGTTTTGGGTCCTCGTGGTGGTCGGGGGTGTGCTGGCCTGCTACAGCT TGCTGGTCACAGTGGCCTTCATCATCTTCTGGGTGCGCTCCAAGAGGAGCCGGCTGCT TCACAGTGATTACATGAACATGACCCCCAGGAGGCCAGGACCCACCAGGAAGCACTAC CAGCCCTACGCTCCCCCGCGGGACTTTGCTGCTTACCGCAGCagagtgaaggtgggta ccactgCgctttgCgaggagggcacggggtcccccacttgatggatgttcagaggggc cttggtcttggaaggtctcaagctcgggtggtgcctggggcttggtatccaggagcaa agcaaggaccagccaagtgtgtgccttgagtgggctgaggaggaggtggcagtgtctg gctgagatggacagggtaggagggagagcctggtgctaggcacctccatgacaagccg tacaaatgtgtgcacatcagagtgtcccagggaaggcgatgctactggtgacaaaggg gcttacactcaggcagaggtccttctttccaagtgtgaatgaaggccatgttagcctt tctcttgaaaaggccctttcctcatctgtaactggggaG | HDRT23 (Z4/Z7) |
| 60 | atctcatttgaccatcatttgacctattgtctccctgtgggtaggcctcagagccaca cacctcaggccaggagtaccattcatccagacgtgaacatcttcccgaggcttccaga gttcttggttcacaccggggctaacatggctgggcttctgctgcagtggcaggagctc tgtgcacagagaacagcctcatctgctcgccttgtttccacctcccctcccattgccc caggttctttggccccacagcggccacatctgccgttggtgccaataggttttccagg agctggttgaggtgggagggagggagagggttgtgatcaggctgaggcatggggattg gatatagtctccgtgtcatgatttatttggtcagtcagtcctagtgcGaccctggggt aatggggatgtgttctcgtcacGttgggcctggctgaccagctttatctcttggcaca gaggcaggcagcggcGCTACCAATTTTTCTTTGTTGAAGCAGGCCGGGGATGTGGAGG AAAATCCGGGGCCAATGGCTCTTCCTGTGACTGCCCTTCTGCTGCCCCTGGCTCTCCT GCTCCATGCTGCCCGGCCAGACATCCAGATGACACAGACAACCAGCAGCCTCAGCGCC AGCCTGGGGGACAGAGTGACCATTAGCTGCCGGGCCTCTCAGGACATCAGCAAATACC TGAACTGGTACCAGCAGAAACCAGATGGCACTGTCAAGCTGCTGATTTACCACACCTC CAGGCTCCACAGCGGCGTGCCCAGTCGCTTCAGCGGCAGTGGGAGCGGGACAGATTAT TCCCTCACAATCTCCAACCTGGAGCAGGAAGATATTGCCACATACTTCTGCCAGCAAG GCAACACCCTGCCATACACATTTGGAGGCGGCACCAAATTGGAGATCACCGGCGGTGG TGGATCTGGAGGAGGGGGCAGCGGAGGTGGCGGCTCTGAGGTGAAACTGCAGGAGAGT GGCCCTGGCCTGGTGGCTCCCAGCCAGAGCCTTTCTGTCACCTGCACCGTGTCTGGGG TGTCCCTGCCTGACTATGGAGTCTCCTGGATCCGGCAGCCTCCAAGAAAAGGACTGGA ATGGCTGGGCGTCATCTGGGGAAGTGAGACCACCTACTATAATTCAGCCCTCAAGTCC | HDRT23 (Z4/Z8) |
| | CGGCTCACCATCATTAAGGACAACTCCAAATCCCAGGTGTTCCTGAAGATGAATTCTC TCCAGACTGATGACACAGCCATCTACTACTGTGCCAAGCATTATTATTACGGCGGGTC CTATGCCATGGACTACTGGGGCCAGGGGACCAGTGTCACTGTTTCTTCTGAAAGCAAG TATGGCCCCCCATGCCCTCCCTGCCCCGGACAGCCGCGGGAGCCCCAGGTCTACACCC TCCCACCCTCCAGAGATGAGCTGACCAAGAACCAGGTTTCACTGACATGCCTGGTGAA GGGCTTCTACCCCTCTGACATTGCTGTGGAGTGGGAGAGCAATGGGCAGCCAGAGAAC AACTACAAGACCACACCTCCTGTCCTGGACAGCGACGGCTCCTTCTTCCTCTACAGTA AGCTCACTGTGGACAAGAGCCGCTGGCAGCAGGGCAATGTCTTCTCCTGCAGCGTGAT GCACGAGGCCCTGCACAATGCCTATACCCAGAAGTCACTCTCTCTGAGCCCTGGAAAG AAGGATCCCAAGTTTTGGGTCCTCGTGGTGGTCGGGGGTGTGCTGGCCTGCTACAGCT TGCTGGTCACAGTGGCCTTCATCATCTTCTGGGTGCGCTCCAAGAGGAGCCGGCTGCT TCACAGTGATTACATGAACATGACCCCCAGGAGGCCAGGACCCACCAGGAAGCACTAC CAGCCCTACGCTCCCCCGCGGGACTTTGCTGCTTACCGCAGCagagtgaaggtgggta ccactgCgctttgCgaggagggcacggggtcccccacttgatggatgttcagaggggc cttggtcttggaaggtctcaagctcgggtggtgcctggggcttggtatccaggagcaa agcaaggaccagccaagtgtgtgccttgagtgggctgaggaggaggtggcagtgtctg gctgagatggacagggtaggagggagagcctggtgctaggcacctccatgacaagccg tacaaatgtgtgcacatcagagtgtcccagggaaggcgatgctactggtgacaaaggg gcttacactcaggcagaggtccttctttccaagtgtgaatgaaggccatgttagcctt tctcttgaaaaggccctttcctcatctgtaactggggaG | |
| 61 | aagctcatttggccagagtggaaatggaattgggagaaatcgatgaccaaatgtaaac acttggtgcctgatatagcttgacaccaagttagccccaagtgaaataccctggcaat attaatgtgtcttttcccgatattcctcaggtactccaaagattcaggtttactcacg tcatccagcagagaatggaaagtcaaatttcctgaattgctatgtgtctgggtttcat ccatccgacattgaagttgacttactgaagaatggagagagaattgaaaaagtggagc attcagacttgtctttcagcaaggactggtctttctatctcttgtactacactgaatt cacccccactgaaaaagatgagtatgctTGCAGGGTTAACCATGTCACATTGTCACAA CCTAAAATTGTGAAATGGGACAGGGACATGGGTGGGGGCGGATCAGGCGGCGGCGGTT CTGGGGGAGGCGGCTCAGGTGGCGGTGGGAGTGGATCCCATTCCCTGAAGTATTTCCA CACCAGCGTTAGTCGGCCGGGAAGGGGAGAACCAAGATTCATTTCCGTCGGCTATGTC GACGATACCCAATTTGTGCGATTTGATAATGACGCAGCTTCACCCCGCATGGTGCCTC GGGCTCCTTGGATGGAGCAAGAAGGCTCAGAGTACTGGGACCGGGAGACCCGATCTGC GCGCGATACAGCACAAATCTTTAGGGTCAACCTTCGAACATTGAGGGGCTACTACAAC CAGAGTGAGGCAGGTTCCCATACGTTGCAATGGATGCATGGTTGCGAACTTGGTCCAG ATGGGAGGTTCCTCAGAGGTTATGAACAATTTGCTTACGATGGAAAGGACTACCTTAC ACTCAATGAGGACCTCCGCAGCTGGACCGCCGTTGACACCGCTGCTCAAATCTCCGAG CAAAAGAGTAATGACGCGTCAGAAGCGGAGCATCAGCGCGCCTACCTCGAGGACACGT GCGTTGAGTGGCTCCATAAATACCTGGAAAAAGGGAAAGAAACCCTCCTCCACCTGGA GCCACCGAAGACCCACGTCACGCATCACCCAATATCCGATCACGAGGCTACACTGAGG TGCTGGGCTCTCGGTTTCTATCCGGCAGAAATAACCCTGACCTGGCAGCAGGATGGTG AGGGCCATACGCAGGACACGGAGCTTGTCGAGACTCGACCCGCCGGTGATGGAACATT CCAAAAATGGGCTGCTGTCGTTGTACCCTCTGGAGAAGAACAACGATACACCTGCCAC GTGCAGCATGAGGGACTGCCGGAACCGGTAACCTTGCGCTGGAAGCCGGCGTCACAAC CAACGATACCCATCGTCGGCATTATAGCCGGACTTGTTCTCCTGGGAAGCGTGGTCAG CGGAGCTGTCGTAGCGGCCGTAATTTGGAGGAAAAAGTCTTCTGGGGGAAAGGGTGGG TCATACTCTAAAGCGGAGTGGTCTGACTCAGCACAGGGTTCCGAATCCCACTCCCTGT GAGCGGCCGCGTCGAGTCTAGAGGGCCCGTTTAAACCCGCTGATCAGCCTCGACTGTG CCTTCTAGTTGCCAGCCATCTGTTGTTTGCCCCTCCCCCGTGCCTTCCTTGACCCTGG AAGGTGCCACTCCCACTGTCCTTTCCTAATAAAATGAGGAAATTGCATCGCATTGTCT GAGTAGGTGTCATTCTATTCTGGGGGGTGGGGTGGGGCAGGACAGCAAGGGGGAGGAT TGGGAAGACAATAGCAGGCATGCTGGGGATGCGGTGGGCTCTATGGccaagatagtta agtggggtaagtcttacattcttttgtaagctgctgaaagttgtgtatgagtagtcat atcataaagctgctttgatataaaaaaggtctatggccatactaccctgaatgagtcc catcccatctgatataaacaatctgcatattgggattgtcagggaatgttcttaaaga tcagattagtggcacctgctgagatactgatgcacagcatggtttctgaaccagtagt ttccctgcagttgagcagggagcagcagcagcacttgcacaaatacatatacactctt aacacttcttacctactggcttcctctagcttttgtggcagcttcaggta | HDRT26 (B1/B8) |
| 62 | aagctcatttggccagagtggaaatggaattgggagaaatcgatgaccaaatgtaaac acttggtgcctgatatagcttgacaccaagttagccccaagtgaaataccctggcaat attaatgtgtcttttcccgatattcctcaggtactccaaagattcaggtttactcacg tcatccagcagagaatggaaagtcaaatttcctgaattgctatgtgtctgggtttcat ccatccgacattgaagttgacttactgaagaatggagagagaattgaaaaagtggagc | HDRT27 (B2/B6) |
| | attcagacttgtctttcagcaaggactggtctttctatctcttgtactacactgaatt cacccccactgaaaaagatgagtatgcCTGCCGTGTGAACCATGTGACTTTGTCACAG CCCAAGATTGTGAAATGGGACAGGGACATGGGTGGGGGCGGATCAGGCGGCGGCGGTT CTGGGGGAGGCGGCTCAGGTGGCGGTGGGAGTGGATCCCATTCCCTGAAGTATTTCCA CACCAGCGTTAGTCGGCCGGGAAGGGGAGAACCAAGATTCATTTCCGTCGGCTATGTC GACGATACCCAATTTGTGCGATTTGATAATGACGCAGCTTCACCCCGCATGGTGCCTC GGGCTCCTTGGATGGAGCAAGAAGGCTCAGAGTACTGGGACCGGGAGACCCGATCTGC GCGCGATACAGCACAAATCTTTAGGGTCAACCTTCGAACATTGAGGGGCTACTACAAC CAGAGTGAGGCAGGTTCCCATACGTTGCAATGGATGCATGGTTGCGAACTTGGTCCAG ATGGGAGGTTCCTCAGAGGTTATGAACAATTTGCTTACGATGGAAAGGACTACCTTAC ACTCAATGAGGACCTCCGCAGCTGGACCGCCGTTGACACCGCTGCTCAAATCTCCGAG CAAAAGAGTAATGACGCGTCAGAAGCGGAGCATCAGCGCGCCTACCTCGAGGACACGT GCGTTGAGTGGCTCCATAAATACCTGGAAAAAGGGAAAGAAACCCTCCTCCACCTGGA GCCACCGAAGACCCACGTCACGCATCACCCAATATCCGATCACGAGGCTACACTGAGG TGCTGGGCTCTCGGTTTCTATCCGGCAGAAATAACCCTGACCTGGCAGCAGGATGGTG AGGGCCATACGCAGGACACGGAGCTTGTCGAGACTCGACCCGCCGGTGATGGAACATT CCAAAAATGGGCTGCTGTCGTTGTACCCTCTGGAGAAGAACAACGATACACCTGCCAC GTGCAGCATGAGGGACTGCCGGAACCGGTAACCTTGCGCTGGAAGCCGGCGTCACAAC CAACGATACCCATCGTCGGCATTATAGCCGGACTTGTTCTCCTGGGAAGCGTGGTCAG CGGAGCTGTCGTAGCGGCCGTAATTTGGAGGAAAAAGTCTTCTGGGGGAAAGGGTGGG TCATACTCTAAAGCGGAGTGGTCTGACTCAGCACAGGGTTCCGAATCCCACTCCCTGT GAGCGGCCGCGTCGAGTCTAGAGGGCCCGTTTAAACCCGCTGATCAGCCTCGACTGTG CCTTCTAGTTGCCAGCCATCTGTTGTTTGCCCCTCCCCCGTGCCTTCCTTGACCCTGG AAGGTGCCACTCCCACTGTCCTTTCCTAATAAAATGAGGAAATTGCATCGCATTGTCT GAGTAGGTGTCATTCTATTCTGGGGGGTGGGGTGGGGCAGGACAGCAAGGGGGAGGAT TGGGAAGACAATAGCAGGCATGCTGGGGATGCGGTGGGCTCTATGGaaaaggtctatg gccatactaccctgaatgagtcccatcccatctgatataaacaatctgcatattggga ttgtcagggaatgttcttaaagatcagattagtggcacctgctgagatactgatgcac agcatggtttctgaaccagtagtttccctgcagttgagcagggagcagcagcagcact tgcacaaatacatatacactcttaacacttcttacctactggcttcctcTAGCTTTTG TGGCAGCTTCAGGTATATTTAGCACTGAACGAACATCTCAAGAAGGTATAGGCCTTTG TTTGTAAGTCCTGCTGTCCTAGCATCCTATAATCCTGGACTTCTCCAGTACTTTCTGG CTGGATTGGTATCTGAGGCTAGTAGGA | |
| 63 | aagctcatttggccagagtggaaatggaattgggagaaatcgatgaccaaatgtaaac acttggtgcctgatatagcttgacaccaagttagccccaagtgaaataccctggcaat attaatgtgtcttttcccgatattcctcaggtactccaaagattcaggtttactcacg tcatccagcagagaatggaaagtcaaatttcctgaattgctatgtgtctgggtttcat ccatccgacattgaagttgacttactgaagaatggagagagaattgaaaaagtggagc attcagacttgtctttcagcaaggactggtctttctatctcttgtactacactgaatt cacccccactgaaaaagatgagtatgcATGCCGTGTGAACCATGTGACTTTGTCACAG CCTAAAATTGTGAAATGGGACAGGGACATGGGTGGGGGCGGATCAGGCGGCGGCGGTT CTGGGGGAGGCGGCTCAGGTGGCGGTGGGAGTGGATCCCATTCCCTGAAGTATTTCCA CACCAGCGTTAGTCGGCCGGGAAGGGGAGAACCAAGATTCATTTCCGTCGGCTATGTC GACGATACCCAATTTGTGCGATTTGATAATGACGCAGCTTCACCCCGCATGGTGCCTC GGGCTCCTTGGATGGAGCAAGAAGGCTCAGAGTACTGGGACCGGGAGACCCGATCTGC GCGCGATACAGCACAAATCTTTAGGGTCAACCTTCGAACATTGAGGGGCTACTACAAC CAGAGTGAGGCAGGTTCCCATACGTTGCAATGGATGCATGGTTGCGAACTTGGTCCAG ATGGGAGGTTCCTCAGAGGTTATGAACAATTTGCTTACGATGGAAAGGACTACCTTAC ACTCAATGAGGACCTCCGCAGCTGGACCGCCGTTGACACCGCTGCTCAAATCTCCGAG CAAAAGAGTAATGACGCGTCAGAAGCGGAGCATCAGCGCGCCTACCTCGAGGACACGT GCGTTGAGTGGCTCCATAAATACCTGGAAAAAGGGAAAGAAACCCTCCTCCACCTGGA GCCACCGAAGACCCACGTCACGCATCACCCAATATCCGATCACGAGGCTACACTGAGG TGCTGGGCTCTCGGTTTCTATCCGGCAGAAATAACCCTGACCTGGCAGCAGGATGGTG AGGGCCATACGCAGGACACGGAGCTTGTCGAGACTCGACCCGCCGGTGATGGAACATT CCAAAAATGGGCTGCTGTCGTTGTACCCTCTGGAGAAGAACAACGATACACCTGCCAC GTGCAGCATGAGGGACTGCCGGAACCGGTAACCTTGCGCTGGAAGCCGGCGTCACAAC CAACGATACCCATCGTCGGCATTATAGCCGGACTTGTTCTCCTGGGAAGCGTGGTCAG CGGAGCTGTCGTAGCGGCCGTAATTTGGAGGAAAAAGTCTTCTGGGGGAAAGGGTGGG TCATACTCTAAAGCGGAGTGGTCTGACTCAGCACAGGGTTCCGAATCCCACTCCCTGT GAGCGGCCGCGTCGAGTCTAGAGGGCCCGTTTAAACCCGCTGATCAGCCTCGACTGTG CCTTCTAGTTGCCAGCCATCTGTTGTTTGCCCCTCCCCCGTGCCTTCCTTGACCCTGG AAGGTGCCACTCCCACTGTCCTTTCCTAATAAAATGAGGAAATTGCATCGCATTGTCT | HDRT29 (B2/B9) |
| | GAGTAGGTGTCATTCTATTCTGGGGGGTGGGGTGGGGCAGGACAGCAAGGGGGAGGAT TGGGAAGACAATAGCAGGCATGCTGGGGATGCGGTGGGCTCTATGGaaaaggtctatg gccatactaccctgaatgagtcccatcccatctgatataaacaatctgcatattggga ttgtcagggaatgttcttaaagatcagattagtggcacctgctgagatactgatgcac agcatggtttctgaaccagtagtttccctgcagttgagcagggagcagcagcagcact tgcacaaatacatatacactcttaacacttcttacctactggcttcctcTAGCTTTTG TGGCAGCTTCAGGTATATTTAGCACTGAACGAACATCTCAAGAAGGTATAGGCCTTTG TTTGTAAGTCCTGCTGTCCTAGCATCCTATAATCCTGGACTTCTCCAGTACTTTCTGG CTGGATTGGTATCTGAGGCTAGTAGGA | |
| 64 | aagctcatttggccagagtggaaatggaattgggagaaatcgatgaccaaatgtaaac acttggtgcctgatatagcttgacaccaagttagccccaagtgaaataccctggcaat attaatgtgtcttttcccgatattcctcaggtactccaaagattcaggtttactcacg tcatccagcagagaatggaaagtcaaatttcctgaattgctatgtgtctgggtttcat ccatccgacattgaagttgacttactgaagaatggagagagaattgaaaaagtggagc attcagacttgtctttcagcaaggactggtctttctatctcttgtactacactgaatt cacccccactgaaaaagatgagtatgctTGCAGGGTTAACCATGTCACATTGTCACAA CCTAAAATTGTGAAATGGGACAGGGACATGGGTGGGGGCGGATCAGGCGGCGGCGGTT CTGGGGGAGGCGGCTCAGGTGGCGGTGGGAGTGGATCCCATTCCCTGAAGTATTTCCA CACCAGCGTTAGTCGGCCGGGAAGGGGAGAACCAAGATTCATTTCCGTCGGCTATGTC GACGATACCCAATTTGTGCGATTTGATAATGACGCAGCTTCACCCCGCATGGTGCCTC GGGCTCCTTGGATGGAGCAAGAAGGCTCAGAGTACTGGGACCGGGAGACCCGATCTGC GCGCGATACAGCACAAATCTTTAGGGTCAACCTTCGAACATTGAGGGGCTACTACAAC CAGAGTGAGGCAGGTTCCCATACGTTGCAATGGATGCATGGTTGCGAACTTGGTCCAG ATGGGAGGTTCCTCAGAGGTTATGAACAATTTGCTTACGATGGAAAGGACTACCTTAC ACTCAATGAGGACCTCCGCAGCTGGACCGCCGTTGACACCGCTGCTCAAATCTCCGAG CAAAAGAGTAATGACGCGTCAGAAGCGGAGCATCAGCGCGCCTACCTCGAGGACACGT GCGTTGAGTGGCTCCATAAATACCTGGAAAAAGGGAAAGAAACCCTCCTCCACCTGGA GCCACCGAAGACCCACGTCACGCATCACCCAATATCCGATCACGAGGCTACACTGAGG TGCTGGGCTCTCGGTTTCTATCCGGCAGAAATAACCCTGACCTGGCAGCAGGATGGTG AGGGCCATACGCAGGACACGGAGCTTGTCGAGACTCGACCCGCCGGTGATGGAACATT CCAAAAATGGGCTGCTGTCGTTGTACCCTCTGGAGAAGAACAACGATACACCTGCCAC GTGCAGCATGAGGGACTGCCGGAACCGGTAACCTTGCGCTGGAAGCCGGCGTCACAAC CAACGATACCCATCGTCGGCATTATAGCCGGACTTGTTCTCCTGGGAAGCGTGGTCAG CGGAGCTGTCGTAGCGGCCGTAATTTGGAGGAAAAAGTCTTCTGGGGGAAAGGGTGGG TCATACTCTAAAGCGGAGTGGTCTGACTCAGCACAGGGTTCCGAATCCCACTCCCTGT GAGCGGCCGCGTCGAGTCTAGAGGGCCCGTTTAAACCCGCTGATCAGCCTCGACTGTG CCTTCTAGTTGCCAGCCATCTGTTGTTTGCCCCTCCCCCGTGCCTTCCTTGACCCTGG AAGGTGCCACTCCCACTGTCCTTTCCTAATAAAATGAGGAAATTGCATCGCATTGTCT GAGTAGGTGTCATTCTATTCTGGGGGGTGGGGTGGGGCAGGACAGCAAGGGGGAGGAT TGGGAAGACAATAGCAGGCATGCTGGGGATGCGGTGGGCTCTATGGccaagatagtta agtggggtaagtcttacattcttttgtaagctgctgaaagttgtgtatgagtagtcat atcataaagctgctttgatataaaaaaggtctatggccatactaccctgaatgagtcc catcccatctgatataaacaatctgcatattgggattgtcagggaatgttcttaaaga tcagattagtggcacctgctgagatactgatgcacagcatggtttctgaaccagtagt ttccctgcagttgagcagggagcagcagcagcacttgcacaaatacatatacactctt aacacttcttacctactggcttcctctagcttttgtggcagcttcaggta | HDRT26 (B4/B8) |
| 65 | aagctcatttggccagagtggaaatggaattgggagaaatcgatgaccaaatgtaaac acttggtgcctgatatagcttgacaccaagttagccccaagtgaaataccctggcaat attaatgtgtcttttcccgatattcctcaggtactccaaagattcaggtttactcacg tcatccagcagagaatggaaagtcaaatttcctgaattgctatgtgtctgggtttcat ccatccgacattgaagttgacttactgaagaatggagagagaattgaaaaagtggagc attcagacttgtctttcagcaaggactggtctttctatctcttgtactacactgaatt cacccccactgaaaaagatgagtatgctTGCAGGGTTAACCATGTCACATTGTCACAA CCTAAAATTGTGAAATGGGACAGGGACATGGGTGGGGGCGGATCAGGCGGCGGCGGTT CTGGGGGAGGCGGCTCAGGTGGCGGTGGGAGTGGATCCCATTCCCTGAAGTATTTCCA CACCAGCGTTAGTCGGCCGGGAAGGGGAGAACCAAGATTCATTTCCGTCGGCTATGTC GACGATACCCAATTTGTGCGATTTGATAATGACGCAGCTTCACCCCGCATGGTGCCTC GGGCTCCTTGGATGGAGCAAGAAGGCTCAGAGTACTGGGACCGGGAGACCCGATCTGC GCGCGATACAGCACAAATCTTTAGGGTCAACCTTCGAACATTGAGGGGCTACTACAAC CAGAGTGAGGCAGGTTCCCATACGTTGCAATGGATGCATGGTTGCGAACTTGGTCCAG ATGGGAGGTTCCTCAGAGGTTATGAACAATTTGCTTACGATGGAAAGGACTACCTTAC ACTCAATGAGGACCTCCGCAGCTGGACCGCCGTTGACACCGCTGCTCAAATCTCCGAG | HDRT26 (B5/B8) |
| | CAAAAGAGTAATGACGCGTCAGAAGCGGAGCATCAGCGCGCCTACCTCGAGGACACGT GCGTTGAGTGGCTCCATAAATACCTGGAAAAAGGGAAAGAAACCCTCCTCCACCTGGA GCCACCGAAGACCCACGTCACGCATCACCCAATATCCGATCACGAGGCTACACTGAGG TGCTGGGCTCTCGGTTTCTATCCGGCAGAAATAACCCTGACCTGGCAGCAGGATGGTG AGGGCCATACGCAGGACACGGAGCTTGTCGAGACTCGACCCGCCGGTGATGGAACATT CCAAAAATGGGCTGCTGTCGTTGTACCCTCTGGAGAAGAACAACGATACACCTGCCAC GTGCAGCATGAGGGACTGCCGGAACCGGTAACCTTGCGCTGGAAGCCGGCGTCACAAC CAACGATACCCATCGTCGGCATTATAGCCGGACTTGTTCTCCTGGGAAGCGTGGTCAG CGGAGCTGTCGTAGCGGCCGTAATTTGGAGGAAAAAGTCTTCTGGGGGAAAGGGTGGG TCATACTCTAAAGCGGAGTGGTCTGACTCAGCACAGGGTTCCGAATCCCACTCCCTGT GAGCGGCCGCGTCGAGTCTAGAGGGCCCGTTTAAACCCGCTGATCAGCCTCGACTGTG CCTTCTAGTTGCCAGCCATCTGTTGTTTGCCCCTCCCCCGTGCCTTCCTTGACCCTGG AAGGTGCCACTCCCACTGTCCTTTCCTAATAAAATGAGGAAATTGCATCGCATTGTCT GAGTAGGTGTCATTCTATTCTGGGGGGTGGGGTGGGGCAGGACAGCAAGGGGGAGGAT TGGGAAGACAATAGCAGGCATGCTGGGGATGCGGTGGGCTCTATGGccaagatagtta agtggggtaagtcttacattcttttgtaagctgctgaaagttgtgtatgagtagtcat atcataaagctgctttgatataaaaaaggtctatggccatactaccctgaatgagtcc catcccatctgatataaacaatctgcatattgggattgtcagggaatgttcttaaaga tcagattagtggcacctgctgagatactgatgcacagcatggtttctgaaccagtagt ttccctgcagttgagcagggagcagcagcagcacttgcacaaatacatatacactctt aacacttcttacctactggcttcctctagcttttgtggcagcttcaggta | |

In one preferred embodiment, guide RNA T1 (SEQ ID NO. 1) is used with guide RNA T7 (SEQ ID NO. 7) and HDRT1 (SEQ ID NO. 38). In preferred embodiments, guide RNA T1 (SEQ ID NO. 1) is used with guide RNA T7 (SEQ ID NO. 7) and the homology arms of HDRT1 (SEQ ID NO. 38), without the knocked-in sequence of SEQ ID NO. 38.

In one preferred embodiment, guide RNA T1 (SEQ ID NO. 1) is used with guide RNA T8 (SEQ ID NO. 8) and HDRT2 (SEQ ID NO. 39). In preferred embodiments, guide RNA T1 (SEQ ID NO. 1) is used with guide RNA T8 (SEQ ID NO. 8) and the homology arms of HDRT2 (SEQ ID NO. 39), without the knocked-in sequence of SEQ ID NO. 39.

In one preferred embodiment, guide RNA T1 (SEQ ID NO. 1) is used with guide RNA T9 (SEQ ID NO. 9) and HDRT3 (SEQ ID NO. 40). In preferred embodiments, guide RNA T1 (SEQ ID NO. 1) is used with guide RNA T8 (SEQ ID NO. 9) and the homology arms of HDRT3 (SEQ ID NO. 40) without the knocked-in sequence of SEQ ID NO. 40.

In one preferred embodiment, guide RNA T1 (SEQ ID NO. 1) is used with guide RNA T10 (SEQ ID NO. 10) and HDRT4 (SEQ ID NO. 41). In preferred embodiments, guide RNAT1 (SEQ ID NO. 1) is used with guide RNA T10 (SEQ ID NO. 10) and the homology arms of HDRT4 (SEQ ID NO. 41) without the knocked-in sequence of SEQ ID NO. 41.

In one preferred embodiment, guide RNA T1 (SEQ ID NO. 1) is used with guide RNA T11 (SEQ ID NO. 11) and HDRT1 (SEQ ID NO. 42). In preferred embodiments, guide RNA T1 (SEQ ID NO. 1) is used with guide RNA T11 (SEQ ID NO. 11) and the homology arms of HDRT1 (SEQ ID NO. 42) without the knocked-in sequence of SEQ ID NO. 42.

In one preferred embodiment, guide RNA T1 (SEQ ID NO. 1) is used with guide RNA T12 (SEQ ID NO. 12) and HDRT1 (SEQ ID NO. 43). In preferred embodiments, guide RNA T1 (SEQ ID NO. 1) is used with guide RNA T12 (SEQ ID NO. 12) and the homology arms of HDRT1 (SEQ ID NO. 43) without the knocked-in sequence of SEQ ID NO. 43.

In one preferred embodiment, guide RNA T2 (SEQ ID NO. 2) is used with guide RNA T8 (SEQ ID NO. 8) and HDRT6 (SEQ ID NO. 44). In preferred embodiments, guide RNA T2 (SEQ ID NO. 2) is used with guide RNA T8 (SEQ ID NO. 8) and the homology arms of HDRT6 (SEQ ID NO. 44) without the knocked-in sequence of SEQ ID NO. 44.

In one preferred embodiment, guide RNA T2 (SEQ ID NO. 2) is used with guide RNA T9 (SEQ ID NO. 9) and HDRT7 (SEQ ID NO. 45). In preferred embodiments, guide RNA T2 (SEQ ID NO. 2) is used with guide RNA T9 (SEQ ID NO. 9) and the homology arms of HDRT7 (SEQ ID NO. 45) without the knocked-in sequence of SEQ ID NO. 45.

In one preferred embodiment, guide RNA T2 (SEQ ID NO. 2) is used with guide RNA T10 (SEQ ID NO. 10) and HDRT8 (SEQ ID NO. 46). In preferred embodiments, guide RNA T2 (SEQ ID NO. 2) is used with guide RNA T10 (SEQ ID NO. 10) and the homology arms of HDRT8 (SEQ ID NO. 46) without the knocked-in sequence of SEQ ID NO. 46.

In one preferred embodiment, guide RNA T3 (SEQ ID NO. 3) is used with guide RNA T12 (SEQ ID NO. 12) and HDRT9 (SEQ ID NO. 47). In preferred embodiments, guide RNA T3 (SEQ ID NO. 3) is used with guide RNA T12 (SEQ ID NO. 12) and the homology arms of HDRT9 (SEQ ID NO. 47) without the knocked-in sequence of SEQ ID NO. 47.

In one preferred embodiment, guide RNA Z2 (SEQ ID NO. 14) is used with guide RNA Z5 (SEQ ID NO. 17) and HDRT17 (SEQ ID NO. 48). In preferred embodiments, guide RNA Z2 (SEQ ID NO. 14) is used with guide RNA Z5 (SEQ ID NO. 17) and the homology arms of HDRT17 (SEQ ID NO. 47) without the knocked-in sequence of SEQ ID NO. 48.

In one preferred embodiment, guide RNA Z2 (SEQ ID NO. 14) is used with guide RNA Z6 (SEQ ID NO. 18) and HDRT17 (SEQ ID NO. 49). In preferred embodiments, guide RNA Z2 (SEQ ID NO. 14) is used with guide RNA Z6 (SEQ ID NO. 18) and the homology arms of HDRT17 (SEQ ID NO. 49) without the knocked-in sequence of SEQ ID NO. 49.

In one preferred embodiment, guide RNA Z3 (SEQ ID NO. 15) is used with guide RNA Z5 (SEQ ID NO. 17) and HDRT20 (SEQ ID NO. 50). In preferred embodiments, guide RNA Z3 (SEQ ID NO. 15) is used with guide RNA Z5 (SEQ ID NO. 17) and the homology arms of HDRT20 (SEQ ID NO. 50) without the knocked-in sequence of SEQ ID NO. 50.

In one preferred embodiment, guide RNA Z3 (SEQ ID NO. 15) is used with guide RNA Z6 (SEQ ID NO. 18) and HDRT20 (SEQ ID NO. 51). In preferred embodiments, guide RNA Z3 (SEQ ID NO. 15) is used with guide RNA Z6 (SEQ ID NO. 18) and the homology arms of HDRT20 (SEQ ID NO. 51) without the knocked-in sequence of SEQ ID NO. 51.

In one preferred embodiment, guide RNA Z3 (SEQ ID NO. 15) is used with guide RNA Z7 (SEQ ID NO. 19) and HDRT21 (SEQ ID NO. 52). In preferred embodiments, guide RNA Z3 (SEQ ID NO. 15) is used with guide RNA Z7 (SEQ ID NO. 19) and the homology arms of HDRT21 (SEQ ID NO. 52) without the knocked-in sequence of SEQ ID NO. 52.

In one preferred embodiment, guide RNA Z3 (SEQ ID NO. 15) is used with guide RNA Z8 (SEQ ID NO. 20) and HDRT21 (SEQ ID NO. 53). In preferred embodiments, guide RNA Z3 (SEQ ID NO. 15) is used with guide RNA Z8 (SEQ ID NO. 20) and the homology arms of HDRT21 (SEQ ID NO. 53) without the knocked-in sequence of SEQ ID NO. 53.

In one preferred embodiment, guide RNA Z3 (SEQ ID NO. 15) is used with guide RNA Z5 (SEQ ID NO. 17) and HDRT22 (SEQ ID NO. 54). In preferred embodiments, guide RNA Z3 (SEQ ID NO. 15) is used with guide RNA Z5 (SEQ ID NO. 17) and the homology arms of HDRT22 (SEQ ID NO. 54) without the knocked-in sequence of SEQ ID NO. 54.

In one preferred embodiment, guide RNA Z3 (SEQ ID NO. 15) is used with guide RNA Z6 (SEQ ID NO. 18) and HDRT22 (SEQ ID NO. 55). In preferred embodiments, guide RNA Z3 (SEQ ID NO. 15) is used with guide RNA Z6 (SEQ ID NO. 18) and the homology arms of HDRT22 (SEQ ID NO. 55) without the knocked-in sequence of SEQ ID NO. 55.

In one preferred embodiment, guide RNA Z3 (SEQ ID NO. 15) is used with guide RNA Z7 (SEQ ID NO. 19) and HDRT23 (SEQ ID NO. 56). In preferred embodiments, guide RNA Z3 (SEQ ID NO. 15) is used with guide RNA Z7 (SEQ ID NO. 19) and the homology arms of HDRT23 (SEQ ID NO. 56) without the knocked-in sequence of SEQ ID NO. 56.

In one preferred embodiment, guide RNA Z3 (SEQ ID NO. 15) is used with guide RNA Z8 (SEQ ID NO. 20) and HDRT23 (SEQ ID NO. 57). In preferred embodiments, guide RNA Z3 (SEQ ID NO. 15) is used with guide RNA Z8 (SEQ ID NO. 20) and the homology arms of HDRT23 (SEQ ID NO. 57) without the knocked-in sequence of SEQ ID NO. 57.

In one preferred embodiment, guide RNA Z4 (SEQ ID NO. 16) is used with guide RNA Z5 (SEQ ID NO. 17) and HDRT24 (SEQ ID NO. 58). In preferred embodiments, guide RNA Z4 (SEQ ID NO. 16) is used with guide RNA Z5 (SEQ ID NO. 17) and the homology arms of HDRT24 (SEQ ID NO. 58) without the knocked-in sequence of SEQ ID NO. 58.

In one preferred embodiment, guide RNA Z4 (SEQ ID NO. 16) is used with guide RNA Z7 (SEQ ID NO. 19) and HDRT23 (SEQ ID NO. 59). In preferred embodiments, guide RNA Z4 (SEQ ID NO. 16) is used with guide RNA Z7 (SEQ ID NO. 19) and the homology arms of HDRT23 (SEQ ID NO. 59) without the knocked-in sequence of SEQ ID NO. 59.

In one preferred embodiment, guide RNA Z4 (SEQ ID NO. 16) is used with guide RNA Z8 (SEQ ID NO. 20) and HDRT23 (SEQ ID NO. 60). In preferred embodiments, guide RNA Z4 (SEQ ID NO. 16) is used with guide RNA Z8 (SEQ ID NO. 20) and the homology arms of HDRT23 (SEQ ID NO. 60) without the knocked-in sequence of SEQ ID NO. 60.

In one preferred embodiment, guide RNA B1 (SEQ ID NO. 21) is used with guide RNA B8 (SEQ ID NO. 28) and HDRT26 (SEQ ID NO. 61). In preferred embodiments, guide RNA B1 (SEQ ID NO. 21) is used with guide RNA B8 (SEQ ID NO. 28) and the homology arms of HDRT26 (SEQ ID NO. 61) without the knocked-in sequence of SEQ ID NO. 61.

In one preferred embodiment, guide RNA B2 (SEQ ID NO. 22) is used with guide RNA B6 (SEQ ID NO. 26) and HDRT27 (SEQ ID NO. 62). In preferred embodiments, guide RNA B2 (SEQ ID NO. 22) is used with guide RNA B6 (SEQ ID NO. 26) and the homology arms of HDRT27 (SEQ ID NO. 62) without the knocked-in sequence of SEQ ID NO. 62.

In one preferred embodiment, guide RNA B2 (SEQ ID NO. 22) is used with guide RNA B9 (SEQ ID NO. 29) and HDRT29 (SEQ ID NO. 63). In preferred embodiments, guide RNA B2 (SEQ ID NO. 22) is used with guide RNA B9 (SEQ ID NO. 29) and the homology arms of HDRT29 (SEQ ID NO. 63) without the knocked-in sequence of SEQ ID NO. 63.

In one preferred embodiment, guide RNA B4 (SEQ ID NO. 24) is used with guide RNA B8 (SEQ ID NO. 28) and HDRT26 (SEQ ID NO. 64). In preferred embodiments, guide RNA B4 (SEQ ID NO. 24) is used with guide RNA B8 (SEQ ID NO. 28) and the homology arms of HDRT26 (SEQ ID NO. 64) without the knocked-in sequence of SEQ ID NO. 64.

In one preferred embodiment, guide RNA B5 (SEQ ID NO. 25) is used with guide RNA B8 (SEQ ID NO. 28) and HDRT26 (SEQ ID NO. 65). In preferred embodiments, guide RNA B5 (SEQ ID NO. 25) is used with guide RNA B8 (SEQ ID NO. 28) and the homology arms of HDRT26 (SEQ ID NO. 65) without the knocked-in sequence of SEQ ID NO. 65.

A person skilled in the art is able to identify the homology arms of a HDR template, the gRNAs, and the knock-in sequence, based on the sequences provided in Table 1. In some embodiments, the combination of rRNA and HDRT lead to unexpectedly good results and high frequencies of the desired genetic modification. In some embodiments, the gRNAs work well due to their relative position in combination with the particular homology arms of the HDRT sequences, thus the homology arms of the HDRT sequences could be used without the specific knock-in sequence provided above, as described in the sequence table, but using some other construct to be knocked in, at essence in the same position of the target genome.

In a further aspect of the invention, the invention relates to an isolated nucleic acid molecule, selected from the group consisting of:
a) a nucleic acid molecule comprising a nucleotide sequence according to any one or more of SEQ ID NO 1-65,
b) a nucleic acid molecule comprising the homology arms of an HDRT sequence presented in one or more of SEQ ID NO 38-65, although with any given knock-in sequence (different from sequences to be knocked-in of SEQ ID NO 38-65)
c) a nucleic acid molecule which is complementary to a nucleotide sequence in accordance with a) or b);
d) a nucleic acid molecule comprising a nucleotide sequence having sufficient sequence identity to be functionally analogous/equivalent to a nucleotide sequence according to a) through c), comprising preferably a sequence identity to a nucleotide sequence according to a) through c) of at least 70%, 80%, 90% or 95%;
e) a nucleic acid molecule which, as a consequence of the genetic code, is degenerate to a nucleotide sequence according to a) through d); and/or
f) a nucleic acid molecule according to a nucleotide sequence of a) through e) which is modified by deletions, additions, substitutions, translocations, inversions and/or insertions and is functionally analogous/equivalent to a nucleotide sequence according to a) through e).

The term degenerate to (or degenerated into) refers to differences in a nucleotide sequence of a nucleic acid molecule, but according to the genetic code, do not lead to differences in amino acid sequence of the protein product of the nucleotide sequence after translation.

The various aspects of the invention are unified by, benefit from, are based on, and/or are linked by the structural and/or functional features, including functional properties and beneficial technical effects, of the in vitro method for modifying dsDNA in an eukaryotic cell described herein. The features disclosed in the context of the in vitro method also apply to and are considered disclosed in the context of the genetically modified eukaryotic cell and vice versa. Any features disclosed in further aspects of the invention, such as the kit, are also considered disclosed in the context of the in vitro method and vice versa.

### DETAILED DESCRIPTION OF THE INVENTION

The present invention relates to an in vitro method for modifying double stranded DNA (dsDNA) in a eukaryotic cell.

Base editors (BEs) are a class of genome editing tools that enable the precise conversion of a single nucleotide in DNA or RNA without generating double-strand breaks (DSBs) or requiring donor DNA templates, making them a safer alternative to traditional gene-editing methods like CRISPR/Cas9, which rely on DSBs and can cause undesirable genomic damage. BEs typically consist of a catalytically impaired or dead Cas protein (e.g., Cas9 or Cas12), which directs the system to a specific genomic locus via a guide RNA (gRNA) fused to a deaminase enzyme that chemically modifies target bases. This allows for the direct conversion of cytosine (C) to thymine (T) or adenine (A) to guanine (G), effectively enabling four types of base transitions (C to T, G to A, A to G, and T to C). Non-limiting examples for deaminase enzymes are adenosine deaminases (ADA) or cytidine deaminase (CDA). Usually, ADA is used for C-to-T base editing. CDA is used for A-to-G base editing. The skilled person in the art is able to select a corresponding deaminase enzyme without undue effort.

There are two main types of DNA base editors: cytosine base editors (CBEs) and adenine base editors (ABEs). CBEs convert a C•G base pair into a T•A base pair through the deamination of cytosine to uracil, which is recognized as thymine during DNA replication or repair. ABEs, on the other hand, convert an A•T base pair into a G•C base pair by deaminating adenine to inosine, which is read as guanine by the cell's replication machinery. Both CBEs and ABEs use a single-stranded DNA bubble formed by the CRISPR-Cas complex as a substrate for deamination, ensuring high specificity to the target site.

Glycosylase base editors (GBEs) are a specialized class of base editors that use a combination of Cas9 nickase, cytidine deaminase, and uracil-DNA glycosylase (Ung) to achieve base transversions, such as converting cytosine (C) to guanine (G). Unlike traditional base editors, which only induce base transitions, GBEs induce transversions by creating an apurinic/apyrimidinic (AP) site following the removal of uracil, the deaminated form of cytosine. This triggers the DNA repair machinery to complete the conversion. GBEs can perform C-to-G edits with high specificity and efficiency in both prokaryotic and mammalian cells, expanding the scope of possible genetic modifications beyond the traditional adenine-to-guanine and cytosine-to-thymine transitions. Modifications such as the integration of engineered variants of enzymes like APOBEC1 or the use of Saccharomyces cerevisiae Ung1 have enhanced GBE editing efficiency. These systems can achieve C-to-G conversions with varying efficiencies, offering the potential for addressing disease-causing mutations. Further improvements, such as the development of GBE2.0, have enhanced both the efficiency and purity of these edits. GBEs complement existing base editing tools by allowing for the correction of G/C-related mutations.

Within first-generation cytosine base editors (BE1), a catalytically impaired dCas9 protein is usually linked to a deaminase. The first generation of base editors encountered low base editing efficiency. CBEs mediate the deamination of cytosines, generating a uracil (U) base recognized as thymine by the cell's DNA polymerases. However, uracil can be recognized and eliminated by the enzyme uracil DNA N-glycosylase (UNG) during the initiation of BER, resulting in fewer C to T edits and increased C to A or C to G edits. Second-generation cytosine base editors (BE2) additionally comprise a uracil glycosylase inhibitor (UGI) fused to the base editor. UGI inhibits the action of UNG, thus conserving the uracil base and achieving higher editing efficiency and purity. In third-generation base editors (BE3), instead of a catalytically dead Cas 9 (dCas9) protein, the nickase Cas9n is used, introducing a nick on the non-modified DNA strand. This nick serves to bias the cellular repair mechanisms to preferentially replace this strand and use the mutagenic intermediate as a template for repair, thus increasing editing efficiency. Fourth-generation cytosine base editors (BE4) comprise an additional copy of UGI, further reducing C-to-G or C-to-A conversions due to UNG.

Any base editor known in the art may be employed in accordance with the present invention.

As used herein, a "base editor system" refers to a system comprising a base editor and at least two guide RNA molecules. In embodiments, the base editor comprises an RNA-guided DNA nickase linked to a single-stranded DNA nucleobase-modifying enzyme or a nucleic acid encoding said base editor. In further embodiments, the guide RNA molecules comprise a first sgRNA that hybridizes to a second target sequence in an opposing strand of the dsDNA.

As used herein, an "editing window" refers to the precise range of DNA nucleotides within which the base editor is designed to target and modify. The editing window is determined by the gRNA sequence and the deaminase enzyme used in the base editor. The gRNA binds to a specific DNA sequence, and the deaminase enzyme acts on the nucleotides within a certain distance of the gRNA binding site. This distance defines the editing window. Depending on the base editor the base editing window is 3 to 15 nucleotides in length, such as 3, 4, 5, 6, 7, 8, 9, 10, 11 ,12, 13, 14 or 15 nucleotides in length.

The term "double-stranded DNA (dsDNA)" molecule relates to two deoxyribonucleic acid polynucleotide strands that are bound together or hybridized by pairing the bases or nucleotides of the two strands through hydrogen bonds, resulting in double-stranded DNA. The method of the present invention can be performed using any dsDNA molecule, including, without limitation, genomic dsDNA of any origin or organism, chromosomal DNA, synthetic dsDNA, and amplified or isolated dsDNA. In the context of the present invention, the term "modifying" a double-stranded DNA refers to any kind of alteration, modification, or change of a dsDNA molecule. In particular, modifying relates to deleting, inserting, replacing, substituting, or translocating one or more nucleotides or pairs of nucleotides or nucleotide sequences from a dsDNA molecule. In the context of the invention, a dsDNA molecule to be modified is the dsDNA molecule, on which one or more of these modifications are introduced by the method of the invention.

As used herein, the term "hybridizing" refers to a reaction in which one or more polynucleotides react to form a complex that is stabilized via hydrogen bonding between the bases of the nucleotide residues. The hydrogen bonding may occur by Watson-Crick base pairing, Hoogstein binding, or any other sequence-specific manner. The complex may comprise two strands forming a duplex structure, three or more strands forming a multi-stranded complex, a single self-hybridizing strand, or any combination. A hybridization reaction may constitute a step in a more extensive process, such as the initiation of PCR or the cleavage of a polynucleotide by an enzyme. A sequence capable of hybridizing with a given sequence may be referred to as the "complement" of the given sequence, even if sequence complementarity is only partial.

Stringent conditions for hybridization refer to conditions under which a nucleic acid having complementarity to a target sequence hybridizes predominantly with the target sequence and substantially does not hybridize with non-target sequences. Stringent conditions are generally sequence-dependent and vary depending on several factors. In general, the longer the sequence, the higher the temperature at which it specifically hybridizes with its target sequence. Non-limiting examples of stringent conditions are described in detail in Tijssen (1993), Laboratory Techniques In Biochemistry And Molecular Biology-Hybridization With Nucleic Acid Probes Part I, Second Chapter "Overview of principles of hybridization and the strategy of nucleic acid probe assay", Elsevier, N.Y. When reference is made to a polynucleotide sequence, complementary or partially complementary sequences are also envisaged. These are preferably capable of hybridizing to the reference sequence under highly stringent conditions. Generally, to maximize the hybridization rate, relatively low-stringency hybridization conditions are selected: about 20 to 25° C. lower than the thermal melting point (Tm). The Tm is the temperature at which 50% of the specific target sequence hybridizes to a perfectly complementary probe in solution at a defined ionic strength and pH. Generally, to require at least about 85% nucleotide complementarity of hybridized sequences, highly stringent washing conditions are selected to be about 5 to 15° C. lower than the Tm. In order to require at least about 70% nucleotide complementarity of hybridized sequences, moderately stringent washing conditions are selected to be about 15 to 30° C. lower than the Tm. Highly permissive (very low stringency) washing conditions may be as low as 50° C. below the Tm, allowing a high level of mismatching between hybridized sequences. Those skilled in the art will recognize that other physical and chemical parameters in the hybridization and wash stages can also be altered to affect the outcome of a detectable hybridization signal from a specific level of homology between target and probe sequences. Preferred highly stringent conditions comprise incubation in 50% formamide, 5×SSC, and 1% SDS at 42° C., or incubation in 5×SSC and 1% SDS at 65° C., with a wash in 0.2×SSC and 0.1% SDS at 65° C.

The term "insertion," per the present invention, is defined by the pertinent art and refers to incorporating one or more nucleotides into a nucleic acid molecule. Insertion of parts of genes, such as parts of exons or introns, as well as insertion of entire genes, is also encompassed by the term "insertion." When the number of inserted nucleotides is not dividable by three, the insertion can result in a frameshift mutation within a gene's coding sequence. Such frameshift mutations will alter the amino acids encoded by a gene following the mutation. In some cases, such a mutation will cause the active translation of the gene to encounter a premature stop codon, resulting in an end to translation and the production of a truncated protein. When the number of inserted nucleotides is instead dividable by three, the resulting insertion is an "in-frame insertion." In this case, the reading frame remains intact after the insertion, and translation will most likely run to completion if the inserted nucleotides do not code for a stop codon. However, because of the inserted nucleotides, the finished protein will contain, depending on the size of the insertion, one or multiple new amino acids that may affect the function of the protein.

The term "deletion," as used per the present invention, is defined by the pertinent art and refers to the loss of nucleotides or larger parts of genes, such as exons or introns, as well as entire genes. As defined with regard to the term "insertion," the deletion of a number of nucleotides that is not evenly dividable by three, in particular in a coding sequence, will lead to a frameshift mutation, causing all of the codons occurring after the deletion to be read incorrectly during translation, potentially producing a severely altered and most likely nonfunctional protein. If a deletion does not result in a frameshift mutation, i.e., because the number of nucleotides deleted is dividable by three, the resulting protein is nonetheless altered as the finished protein will lack. Depending on the size of the deletion, one or several amino acids may affect the function of the protein.

A "cell" in the sense of the present invention refers to, without limitation, any biological cell, which might be derived from any kind of organism, comprising unicellular organisms as well as multicellular organisms, such as any kind of plant or animal, including mammals, fish, amphibians, reptiles, birds, mollusks, arthropods, annelids, nematodes, flatworms, cnidarians, ctenophores and sponges. Cells of the present invention further comprise blood cells, stem cells, hematopoietic stem cells, hematopoietic stem and progenitor cells (HSPC), immune cells (such as B-cells, dendritic cells, granulocytes, innate lymphoid cells (ILCs), megakaryocytes, monocytes, macrophages, myeloid-derived Suppressor Cells (MDSC), natural killer (NK) cells, platelets, red blood cells (RBCs), T-cells or thymocytes), cancer cells, tumor cells and circulating tumor cells. In some embodiments of the invention, the cell in which the dsDNA is to be modified is a vertebrate cell, more preferably a mammalian cell, such as a human cell.

The term "introducing into the cell," as used herein, relates to any known method of introducing a protein or a nucleic acid molecule into a cell. Non-limiting examples include microinjection, infection with viral vectors, electroporation, and transfection, such as transfection using formulations with cationic lipids. The skilled person knows suitable methods for introducing the components of the present invention into a cell.

Clustered Regularly Interspaced Short Palindromic Repeats (CRISPR) are a family of DNA sequences found in bacteria containing fragments of viral DNA from past infections. These fragments enable the bacteria to recognize and neutralize future attacks by similar viruses. Integral to the bacterial immune defense, CRISPR sequences have given rise to the CRISPR/Cas technology, which allows for precise and efficient gene editing in living organisms.

Sequences within CRISPR loci are transcribed and processed into CRISPR RNAs (crRNAs), which, in conjunction with trans-activating crRNAs (tracrRNAs), form complexes with CRISPR-associated (Cas) proteins. These complexes guide Cas nucleases to specific DNA targets through Watson-Crick base pairing between nucleic acids (Wiedenheft, B et al. (2012). Nature 482: 331-338; Horvath, P et al (2010). Science 327: 167-170; Fineran, PC et a. (2012). Virology 434: 202-209). The type II CRISPR nuclease system requires three key components: the Cas9 protein, mature crRNA, and tracrRNA. However, fusing the crRNA and tracrRNA into a single guide RNA (sgRNA) can simplify this system into two components. Re-targeting the Cas9/sgRNA complex to new DNA sites is achievable by modifying a short sequence within the gRNA (Garneau, JE et al. (2010). Nature 468: 67-71; Deltcheva, E et al. (2011). Nature 471: 602-607, Jinek, M et al. (2012) Science 337: 816-821).

CRISPR-Cas systems are RNA-guided adaptive immune systems of bacteria and archaea that provide sequence-specific resistance against viruses or other invading genetic material. This immune-like response is divided into two classes based on the architecture of the effector module responsible for target recognition and the cleavage of the invading nucleic acid (Makarova KS et al. Nat Rev Microbiol. 2015 Nov; 13(11):722-36.). Class 1 comprises multi-subunit Cas protein effectors, and Class 2 consists of a single large effector protein. Class 1 and 2 use CRISPR RNAs (crRNAs) to guide a Cas nuclease component to its target site, where it cleaves the invading nucleic acids. Due to their simplicity, Class 2 CRISPR-Cas systems are the most studied and widely applied for genome editing. The most widely used CRISPR-Cas system is CRISPR-Cas9.

It was demonstrated that the CRISPR/Cas9 system could be engineered for efficient genetic modification in mammalian cells. The only sequence limitation of the CRISPR/Cas system appears to derive from the necessity of a protospacer-adjacent motif (PAM) located immediately 3' to the target site. The PAM sequence is specific to the species of Cas9. For example, the PAM sequence 5'-NGG-3' is necessary to bind and cleavage DNA by the commonly used Cas9 from Streptococcus pyogenes. However, Cas9 variants with novel PAMs have been and may be engineered by directed evolution, thus expanding the number of potential target sequences. Cas9 complexed with the crRNA and tracrRNA undergoes a conformational change and associates with PAM motifs throughout the genome, interrogating the sequence directly upstream to determine sequence complementarity with the gRNA. The formation of a DNA-RNA heteroduplex at a matched target site allows for cleavage of the target DNA by the Cas9-RNA complex. These methods and mechanisms are well-known in the art. Upon binding of the Cas9-RNA complex (preferably comprising a sgRNA) to the target site/target sequence of a dsDNA to be modified, an RNA/DNA Cas9 complex is formed.

As known in the art, CRISPR/Cas9 has been exploited to develop potent tools for genome manipulation in animals, plants, and microorganisms. The RNA-guided Cas9 endonuclease first recognizes a 2- to 4-base-pair conserved sequence named the protospacer-adjacent motif (PAM), which flanks a target DNA site (target sequence). Upon binding to the PAM, Cas9 interrogates the flanking DNA sequences for base-pairing complementarity to a guide RNA. If the first 12 base pairs are complementary (the `seed' sequence) of the guide RNA and the target DNA strand, RNA strand invasion accompanies local DNA unwinding to form an R-loop. Precise cleavage of each DNA strand by the RuvC and HNH domains of Cas9 generates a blunt double-strand DNA (dsDNA) break (DSB) at a position three base pairs upstream of the 3' edge of the protospacer sequence, measuring from the PAM.

CRISPR/Cas9 genome-editing experiments have been exploiting the host cell machinery to repair the genome precisely at the site of the Cas9-generated DSB. Mutations can arise either by non-homologous end joining (NHEJ) or homology-directed repair (HDR) of DSBs. NHEJ can produce small insertions or deletions (INDELs) at the cleavage site, whereas HDR uses a native (or engineered) DNA template to replace the targeted allele with an alternative sequence by recombination. Additional DNA repair pathways such as single-strand annealing, alternative end joining, microhomology-mediated joining, mismatch, and base- and nucleotide-excision repair can also produce genome edits.

As used herein, the term "INDEL" relates to the insertion or deletion of bases in an organism's genome generated upon repair after a dsDNA break.

Cas9, also named Csn1, is a large protein that participates in crRNA biogenesis and the destruction of invading DNA. Cas9 has been described in different bacterial species such as S. thermophilus (Sapranauskas, Gasiunas, et al. 2011), listeria innocua (Gasiunas, Barrangou, et al. 2012; Jinek, Chylinski, et al. 2012) and S. pyogenes (Deltcheva, Chylinski, et al. 2011). The large Cas9 protein (>1200 amino acids) contains two predicted nuclease domains, namely the HNH (McrA-like) nuclease domain that is located in the middle of the protein and a split RuvC-like nuclease domain (RNase H fold) (Haft, Selengut, et al. 2005; Makarova, Grishin, et al. 2006). In wild-type Cas9, these two domains result in blunt cleavage of the invasive DNA within the same target sequence (protospacer) near the PAM (Jinek, Chylinski, et al. 2012).

Cas9 variants derived from Streptococcus pyogenes Cas9 (SpCas9) have been generated for use as nickases, dual nickases, or Fokl fusion variants. More recently, Cas9 orthologs and other nucleases derived from class 2 CRISPR-Cas systems, including Cpf1 and C2c1, have been added to the CRISPR toolbox. These ongoing efforts to mine the abundant bacterial and archaeal CRISPR-Cas systems should increase the range of molecular tools available to researchers.

A nickase (or nicking enzyme or nicking endonuclease) is an enzyme that cuts one strand of a double-stranded DNA at a specific recognition nucleotide sequence known as a restriction site. Such enzymes hydrolyze (cut) only one strand of the DNA duplex to produce DNA molecules that are "nicked" rather than cleaved. Over 200 nicking enzymes have been studied, and 13 of these are available commercially and are routinely used for research and in commercial products.

Nickases can be and have been generated by mutating the nuclease domains of Cas9 independently of each other to create DNA nickase capable of introducing a single-strand cut with the same specificity as a regular CRISPR/Cas9 nuclease (Gasiunas G, Barrangou R, Horvath P, Siksnys V. Cas9-crRNA ribonucleoprotein complex mediates specific DNA cleavage for adaptive immunity in bacteria. Proc. Natl Acad. Sci. USA 109, E2579-E2586 (2012). The use of Cas9 nickases is essentially the same as the use of the fully functional enzyme.

Cas9 nickase is created by mutating one of two Cas9 nuclease domains. Cas9 nickase creates a single-strand rather than a double-strand break. For example, the D10A mutation inactivates the RuvC domain, so this nickase cleaves only the target strand. Conversely, the H840 mutation in the HNH domain creates a non-target strand-cleaving nickase; instead of cutting both strands bluntly with WT Cas9 and one gRNA, a staggered cut using a Cas9 nickase and two gRNAs.

In other words, in RNA-guided DNA nickases, the naturally occurring endonuclease function of cleaving both strands of a double-stranded target DNA is altered into an endonuclease that cleaves (i.e., nicks) only one of the strands. Means and methods of modifying RNA-guided DNA endonuclease such as Cas9 are well known in the art and include, for example, the introduction of amino acid replacements into Cas9 that render one of the nuclease domains inactive. More specifically, aspartate can be replaced against alanine at position 10 of the Streptococcus pyogenes Cas9 (SpCas9 D10A; Cong et al. (2013) Science 339:819-823). Further examples are known in the art, for example the H840A replacement in SpCas9 (Mali P et al. Nat Biotechnol. 2013 Sep; 31(9):833-8; Ran FA et al. Cell. 2013 Sep 12; 154(6):1380-9).

The catalytic residues of the compact SpCas9 protein are those corresponding to amino acids D10, D31, H840, H868, N882, and N891 or aligned positions using the CLUSTALW method on homologs of Cas Family members. Any of these residues can be replaced by any other amino acids, preferably by alanine residue. Mutation in the catalytic residues means either substituting with another amino acid or deleting or adding amino acids that induce the inactivation of at least one of the catalytic domains of Cas9. (Sapranauskas, Gasiunas et al. 2011; Jinek, Chylinski et al. 2012). In a particular embodiment, Cas9 may comprise one or several of the above mutations. In another particular embodiment, Cas9 may comprise only one of the two RuvC and HNH catalytic domains. In the present invention, Cas9 nickases of different species, Cas9 homologs, and Cas9 engineered, as well as functional variants thereof, can be used.

In the context of the present invention, the term "RNA-guided DNA endonuclease" refers to DNA endonucleases that interact with at least one RNA molecule. In the context of the present invention, the terms RNA-guided DNA endonuclease and RNA-guided endonuclease are used interchangeably. DNA endonucleases cleave the phosphodiester bond within a DNA polynucleotide chain. In the case of RNA-guided DNA endonuclease, the interacting RNA molecule may guide the RNA-guided DNA endonuclease to the site or location in a DNA where the endonuclease becomes active. In particular, the term RNA-guided DNA endonuclease refers to naturally occurring or genetically modified Cas nuclease components or CRISPR-Cas systems, which include, without limitation, multi-subunit Cas protein effectors of class 1 CRISPR-Cas systems as well as single large effector Cas proteins of class 2 systems. In the context of the present invention, DNA endonucleases functioning as nickases are used. Accordingly, in the context of the present invention, the RNA-guided DNA endonuclease is an RNA-guided DNA nickase. In the context of the invention, the RNA guiding the DNA nickase to the target site/sequence is a guide RNA.

Details of the technical application of CRISPR/Cas systems and suitable RNA-guided endonuclease are known to the skilled person and have been described in detail in the literature, for example, by Barrangou R et al. (Nat Biotechnol. 2016 Sep 8;34(9):933-941), Maeder ML et al. (Mol Ther. 2016 Mar;24(3):430-46) and Cebrian-Serrano A et al. (Mamm Genome. 2017; 28(7): 247-261). The present invention uses guide RNA-guided DNA nickases but is not limited to the use of a specific RNA-guided nickase and, therefore, comprises any given RNA-guided nickase in the sense of the present invention suitable for use in the method described herein.

Any RNA-guided DNA nickase known in the art may be employed in accordance with the present invention. A suitable nickase may be constructed based on an RNA-guided DNA endonuclease selected from the group comprising, without limitation, Cas proteins of class 1 CRISPR-Cas systems, such as Cas3, Cas8a, Cas5, Cas8b, Cas8c, Cas10d, Cse1, Cse2, Csy1, Csy2, Csy3, GSU0054, Cas10, Csm2, Cmr5, Csx11, Csx10, and Csf1; Cas proteins of class 2 CRISPR-Cas systems, such as Cas9, Csn2, Cas4, Cpf1, C2c1, C2c3, and C2c2; corresponding orthologous enzymes/CRISPR effectors from various bacterial and archeal species; engineered CRISPR effectors with for example novel PAM specificities, increased fidelity, such as SpCas9-HF1/eSpCas9, or altered functions. Particularly preferred are nickases based on RNA-guided DNA endonuclease selected from the group comprising Streptococcus pyogenes Cas9 (SpCas9), Staphylococcus aureus Cas9, Streptococcus thermophilus Cas9, Neisseria meningitidis Cas9 (NmCas9), Francisella novicida Cas9 (FnCas9), Campylobacter jejuni Cas9 (CjCas9), Cas12a (Cpf1) and Cas13a (C2C2) (Makarova KS et al. (November 2015). Nature Reviews Microbiology. 13 (11): 722-36).

In accordance with the method of the invention, the RNA-guided DNA nickase may be introduced as a protein, but alternatively, it may also be introduced as a nucleic acid molecule encoding said protein. It will be appreciated that the nucleic acid molecule encodes said RNA-guided DNA nickase in an inexpressible form such that expression in the cell results in a functional RNA-guided DNA nickase protein. Means and methods to ensure the expression of a functional polypeptide are well-known in the art.

For example, the coding sequences for the nickase may be comprised in a vector, such as a plasmid, cosmid, virus, bacteriophage, or another vector used conventionally, e.g., in genetic engineering. The coding sequences inserted in the vector can, e.g., be synthesized by standard methods or isolated from natural sources. The coding sequences may further be ligated to transcriptional regulatory elements and/or to other amino acid encoding sequences. Such regulatory sequences are well known to those skilled in the art and include, without being limiting, regulatory sequences ensuring the initiation of transcription, internal ribosomal entry sites (IRES), and optionally regulatory elements ensuring termination of transcription and stabilization of the transcript. Non-limiting examples for regulatory elements ensuring the initiation of transcription comprise a translation initiation codon, transcriptional enhancers such as e.g. the SV40-enhancer, insulators and/or promoters, such as the cytomegalovirus (CMV) promoter, SV40-promoter, RSV-promoter (Rous sarcome virus), the lacZ promoter, chicken beta-actin promoter, CAG-promoter (a combination of chicken beta-actin promoter and cytomegalovirus immediate-early enhancer), the gai10 promoter, human elongation factor 1a-promoter, A0X1 promoter, GAL1 promoter CaM-kinase promoter, the lac, trp or tac promoter, the lacUV5 promoter, the autographa californica multiple nuclear polyhedrosis virus (AcMNPV) polyhedral promoter or a globin intron in mammalian and other animal cells. Non-limiting examples for regulatory elements ensuring transcription termination include the V40-poly-A site, the tk-poly-A site, or the SV40, lacZ, or AcMNPV polyhedral polyadenylation signals, which are to be included downstream of the nucleic acid sequence of the invention. Additional regulatory elements may include translational enhancers, Kozak sequences, and intervening sequences flanked by donor and acceptor sites for RNA splicing. Moreover, elements such as the origin of replication, drug resistance genes, or regulators (as part of an inducible promoter) may also be included.

As used herein, "nucleic acid" shall mean any nucleic acid molecule, including, without limitation, DNA, RNA, and hybrids or modified variants thereof. An "exogenous nucleic acid" or "exogenous genetic element" relates to any nucleic acid introduced into the cell that is not a component of the cell's "original" or "natural" genome. Exogenous nucleic acids may be integrated or nonintegrated in the genetic material of the target cell or relate to stably transduced nucleic acids.

Nucleic acid molecules encoding said RNA-guided DNA nickase include DNA, such as cDNA or genomic DNA, as well as RNA, particularly mRNA. The skilled person will readily appreciate that more than one nucleic acid molecule may encode an RNA-guided DNA nickase under the present invention due to the degeneracy of the genetic code. Degeneracy results because a triplet code designates 20 amino acids and a stop codon. Because four bases are utilized to encode genetic information, triplet codons are required to produce at least 21 different codes. The possible e possibilities for bases in triplets give 64 possible codons, meaning that some degeneracy must exist. As a result, some amino acids are encoded by more than one triplet, i.e., by up to six. The degeneracy mostly arises from alterations in the third position in a triplet. This means that nucleic acid molecules having different sequences but still encoding the same RNA-guided DNA endonuclease can be employed by the present invention.

The nucleic acid molecules in the present invention may be of natural and/or (semi) synthetic origin. Thus, they may, for example, be nucleic acid molecules that have been synthesized according to conventional protocols of organic chemistry. The person skilled in the art is familiar with the preparation and use of said probes (see, e.g., Sambrook and Russel, "Molecular Cloning, A Laboratory Manual," Cold Spring Harbor Laboratory, N.Y. (2001)).

In embodiments, the nucleic acids encoding for the RNA-guided endonuclease, base editor, or prime editor, in the context of the present invention, includes nucleic acids, preferably mRNA, containing modified backbones, non-natural internucleoside linkages, modified nucleotides, poly-(A)-tail and/or untranslated regions (UTRs). The invention encompasses nucleic acids with at least one structural modification that provides improved stability, reduced immunogenicity, and/or half-life of said nucleic acid molecule post-administration in a cell and/or organism compared to a structurally unmodified nucleic acid of the same sequence.

Non-limiting examples of modified oligonucleotide backbones include, for example, phosphorothioates, chiral phosphorothioates, phosphorodithioates, phosphotriesters, aminoalkylphosphotriesters, methyl, and other alkyl phosphonates. Various salts, mixed salts, and free acid forms are also included.

The nucleic acid molecules used in accordance with the invention may be nucleic acid mimicking molecules known in the art, such as synthetic or semi-synthetic derivatives of nucleic acid molecules and mixed polymers. They may contain additional non-natural or derivatized nucleotide bases, as will be readily appreciated by those skilled in the art. Nucleic acid mimicking molecules or nucleic acid derivatives, according to the invention, include without being limiting, phosphorothioate nucleic acid, phosphoramidite nucleic acid, morpholino nucleic acid, hexitol nucleic acid (HNA), peptide nucleic acid (PNA) and locked nucleic acid (LNA).

Modified nucleobases include other synthetic and natural nucleobases such as N'-methylpseudouridine, 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C≡C-CH3) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-amino-adenine, 8-azaguanine and 8-azaadenine, 7-deazaguanine and 7-deazaadenine and 3-deazaguanine and 3-deazaadenine. Further modified nucleobases include tricyclic pyrimidines such as phenoxazine cytidine(1H-pyrimido[5,4-b][1,4]benzoxazin-2(3H)-one), phenothiazine cytidine (1H-pyrimido[5,4-b][1,4]benzothiazin-2(3H)-one), G-clamps such as a substituted phenoxazine cytidine (e.g. 9-(2-aminoethoxy)-H-pyrimido[5,4-b][1,4]benzoxazin-2(3H)-one), carbazole cytidine (2H-pyrimido[4,5-b]indol-2-one), pyridoindole cytidine (H-pyrido[3',2':4,5]pyrrolo[2,3-d]pyrimidin-2-one). Modified nucleobases may also include those in which the purine or pyrimidine base is replaced with other heterocycles, for example 7-deaza-adenine, 7-deazaguanosine, 2-aminopyridine and 2-pyridone.

It is not necessary for all positions in a given nucleic acid to be uniformly modified, and in fact, more than one of the aforementioned modifications may be incorporated in a single compound or even at a single nucleoside within a nucleic acid.

A skilled person is aware of appropriate methods of nucleic acid synthesis. Modern techniques enable rapid and inexpensive custom-made nucleic acids of a desired sequence. For example, a common process relates to solid-phase synthesis using the phosphoramidite method and phosphoramidite building blocks derived from protected 2'-deoxynucleosides (dA, dC, dG, and T), ribonucleosides (A, C, G, and U), or chemically modified nucleosides, e.g., LNA, BNA. To obtain the desired oligonucleotide, the building blocks (modified or naturally occurring) are sequentially coupled to the growing oligonucleotide chain in the order required by the sequence of the product. Upon the completion of the chain assembly, the product is released from the solid phase to the solution, deprotected, and collected. Products may be isolated by high-performance liquid chromatography (HPLC) to obtain the desired nucleic acids in high purity if required.

Furthermore, the present invention's method comprises introducing at least two guide RNAs into the cell. In the context of the present invention, a "guide RNA" refers to RNA molecules interacting with RNA-guided DNA endonuclease, such as a nickase, leading to the recognition of the target sequence to be cleaved by the RNA-guided DNA endonuclease. According to the present invention, the term "guide RNA" therefore comprises, without limitation, target sequence-specific CRISPR RNAs (crRNA), trans-activating crRNAs (tracrRNA), and chimeric single guide RNAs (sgRNA).

Guide RNA (gRNA) and single guide RNA (sgRNA) are components of the CRISPR-Cas system, primarily used for gene editing. gRNA is a short RNA sequence that guides Cas9 endonuclease or other Cas proteins to specific DNA sites for targeted cleavage. In bacteria and archaea, gRNAs are integral to the adaptive immune response, directing Cas enzymes to degrade foreign DNA. sgRNA, a more streamlined version, combines the crRNA (which binds to the target DNA) and tracrRNA (which activates Cas9) into a single molecule, enhancing efficiency and ease of use in CRISPR applications. Both gRNA and sgRNA enable precise targeting of genes, facilitating various applications in genetic research and biotechnology. As used herein, the terms "single guide RNA" and "guide RNA" can be used interchangeably.

crRNAs differ depending on the RNA-guided endonuclease and the CRISPR/Cas system but typically contain a target-specific sequence of between 20 to 72 nucleotides in length, flanked by two direct repeats (DR) of a length of between 21 to 46 nucleotides. In the case of S. pyogenes, the DRs are 36 nucleotides long, and the target sequence is 30 nucleotides long. The 3' located DR of the crRNA is complementary to and hybridizes with the corresponding tracr RNA, which in turn binds to the Cas9 protein.

As used herein, the term "trans-activating crRNA (tracrRNA)" refers to a small RNA that is complementary to and base pairs with a pre-crRNA (3' located DR of the crRNA), thereby forming an RNA duplex. This pre-crRNA is then cleaved by an RNA-specific ribonuclease to form a crRNA/tracrRNA hybrid, which subsequently acts as a guide for the endonuclease Cas9, which cleaves the invading nucleic acid.

As described herein, the genes encoding the elements of a CRISPR/Cas system, such as Cas9, tracrRNA, and crRNA, are typically organized in an operon(s). DR sequences functioning together with RNA-guided endonucleases, such as Cas9 proteins of other bacterial species, may be identified by bioinformatic analysis of sequence repeats occurring in the respective CRISPR/Cas operons and by experimental binding studies of Cas9 protein and tracrRNA together with putative DR sequence flanked target sequences.

Alternatively, a chimeric single guide RNA sequence comprising such a target sequence-specific crRNA and tracrRNA may be employed. Such a chimeric (ch) RNA may be designed by the fusion of a target-specific sequence of 20 or more nucleotides (nt) with a part or the entire DR sequence (defined as part of a crRNA) with the entire or part of a tracrRNA, as shown by (Jinek et al. Science 337:816-821). Within the chimeric RNA, a segment of the DR and the tracrRNA sequence are complementary and able to hybridize and form a hairpin structure. In the context of the invention, sgRNAs as guide RNAs are preferred.

Moreover, the two guide RNAs of the present invention differing in their target-specific sequence may also be encoded by a nucleic acid molecule, which is introduced into the cell. The definitions and preferred embodiments concerning the nucleic acid molecule encoding the nickase apply equally to the nucleic acid molecule encoding these RNAs. Regulatory elements for expressing RNAs are known to one skilled in the art, such as a U6 promoter.

The present invention relates to the generation of single-strand breaks of the dsDNA molecule to be modified, wherein the dsDNA molecule comprises at least two target sequences, which are targeted by at least two guide RNAs. The first guide RNA targets/hybridizes to a first target sequence in the dsDNA to be modified, and the second guide RNA hybridizes to a second target sequence in the dsDNA to be modified, preferably with the target-specific sequence of the guide RNA.

In accordance with the present invention, a "target sequence" is a nucleotide sequence in the dsDNA molecule that is recognized by the guide RNA and is associated with the RNA-guided nickase due to the target-specific sequence comprised by the guide RNA. The target sequence is at least partially complementary to the target-specific sequence of the guide RNA and is associated with a so-called protospacer adjacent motif (PAM). PAM is a 2-6 base pair DNA sequence located adjacent to the target sequence and can be located either at the 5'-end (for example, for the Crispr/Cpf1 system) or at the 3'-end of the target sequence (for example, for the Crispr/Cas9 system), depending on the Crispr/Cas system employed. An RNA-guided nickase, such as a Cas9 or Cpf1-based nickase, will not successfully bind to and cleave the targeted dsDNA molecule if the recognized target sequence is not associated with a PAM sequence. Forming a DNA-RNA heteroduplex between the target sequence and the target-specific sequence of the guide RNA with the bound nickase allows for cleavage of the target DNA by this guide RNA/DNA nickase complex. Cleavage of the targeted dsDNA molecule occurs within the target sequence or at a site in close proximity or adjacent to the target sequence, depending on the function of the used RNA-guided nickase and CRISPR/Cas system.

For example, in the case of SpCas9, the DNA target sequence of at least 20 nucleotides is located directly upstream/at the 5'-end of an invariant 5'-NGG-3' PAM. Correct pairing of the guide RNA to the DNA target sequence leads to the generation of a cut in the dsDNA molecule ("cleavage" of the dsDNA molecule by SpCas9) 3 base-pairs (bp) upstream of the PAM within the target sequence. However, SpCas9 mutants have been developed with altered PAM requirements (e.g. minimal PAMs, e.g. SpCas9-NG, SpyR).

In the case of the CRISPR/Cpf1 system, the Cpf1-crRNA complex cleaves target DNA by identification of a target sequence that may be located downstream/at the 3' end of a protospacer adjacent motif (for example, 5'-YTN-3' (where "Y" is a pyrimidine and "N" is any nucleobase) or 5'-TTN-3'). Cpf1 can introduce a sticky end/staggered end DNA double-strand breaks. In the case of AsCpf1 and LbCpf1 , a double-strand break with a 4 nucleotides overhang can be generated, which can occur 19 bp downstream of the PAM on the targeted (+)-strand and 23 bp downstream of the PAM on the (-)-strand. Corresponding nickases function analogously.

The exact site of the cut depends on the CRISPR/Cas system or the RNA-guided endonuclease/nickase employed in the method of the invention and can, therefore, be determined by the person skilled in the art upon selection of the RNA-guided DNA nickase.

In the context of the present invention, a cleavage site in close proximity to the target sequence is located within 100 nucleotides or base pairs upstream or downstream from the 5'- or 3'-end of the target sequence. Preferably, the double-strand break is generated within 90, 80, 70, 60, 50, 40, 30, 20, 10, 5, 4, 3, 2, or 1 nucleotides/base pairs upstream or downstream from the 5'- or 3'-end of the target sequence or within the target sequence.

In the context of the present invention, the target sequence may also be called a "protospacer." The term "target site" may refer to a location or sequence in the dsDNA molecule comprising the target sequence and an associated PAM.

In the context of the present invention, a "single strand break" (SSB) refers to a disruption involving only one of the two strands within a double-stranded DNA (dsDNA) molecule. Such a break does not result in the physical dissociation of the upstream and downstream segments of the dsDNA at the site of the lesion. In contrast, a "double-strand break" or "DSB" refers to the interruption of both strands of a dsDNA molecule, leading to the separation of the parts of the dsDNA molecule that lie upstream and downstream of the side of the double-strand break.

In the context of the present invention, at least two SSBs can occur due to the cleavage of both strands of a dsDNA, which is to be modified by an RNA-guided nickase. In embodiments, a DSB may occur due to cleavage (introduction of SSBs) by the nickase at the two target sites on the opposing strands of the dsDNA to be modified, i.e. the (+)- and the (-)-strand, despite the spacer/distance between the SSBs.

In the context of the invention, two SSBs are produced intentionally by RNA-guided nucleases to achieve modification of the dsDNA through homology-directed repair.

Cellular DNA repair mechanisms implicated in the repair of double-strand breaks (DSBs) include homology-directed repair (HDR), alternative non-homologous end joining (a-NHEJ), and classical non-homologous end joining (c-NHEJ). The c-NHEJ pathway remains active throughout all phases of the cell cycle. In contrast, HDR is confined to the S and G2 phases, requiring homologous DNA sequences to mediate repair. During mitosis, DSB repair is entirely suppressed to prevent telomere fusion, a critical safeguard for chromosomal integrity. In the G1 (and G0) phases, as well as in quiescent cells, c-NHEJ predominates due to the silencing of HDR. However, in the S and G2 phases, all repair pathways are active and complete. However, c-NHEJ often predominates when DSBs are induced in a population of cycling cells, resulting in a spectrum of edited alleles.

"Homology-directed repair (HDR)" is a precise cellular mechanism for repairing double-stranded DNA breaks. The most well-known form of HDR is homologous recombination, utilized primarily during the G2 and S phases when a homologous DNA template is present in the nucleus. HDR also encompasses other pathways, such as single-strand annealing and break-induced replication. In the absence of a homologous DNA sequence, the cell resorts to non-homologous end joining (NHEJ), which does not require sequence homology for repair.

In proliferating cells, homology-directed repair (HDR) is facilitated primarily through the homologous recombination (HR) pathway. This native mechanism utilizes intact homologous sequences from sister chromatids as a template to repair double-strand breaks (DSBs), leading to the restoration of the wild-type allele. The art has demonstrated that precise sequence modifications can be introduced at targeted DSBs by leveraging the HR pathway. Targeted modifications can be achieved by providing an exogenous DNA donor template-also referred to as a repair template-containing sequences homologous to the DSB ends. The sequence located between the homologous ends, whether it involves an insertion or a replacement, is seamlessly integrated into the target locus during HR, allowing for the generation of precisely modified 'knock-in' alleles. These modifications are particularly useful for codon replacements or the insertion of reporter genes. Notably, large sequence insertions often necessitate the use of double-stranded DNA donor templates, such as plasmid-based gene-targeting vectors with homology arms of at least 100 base pairs (preferably 500 base pairs or more), to ensure efficient integration.

The HDR-mediated repair of DSBs requires resection at the DSB ends, generating 3' single-stranded DNA (ssDNA) overhangs. These overhangs subsequently anneal with a homologous DNA sequence, which serves as a template for DNA repair synthesis across the DSB. HDR predominantly occurs via the high-fidelity homologous recombination repair (HRR) pathway but can also proceed through error-prone mechanisms such as single-strand annealing (SSA) or microhomology-mediated end joining (MMEJ). HRR and SSA share initial steps involving ATM signaling, formation of ionizing radiation-induced foci (IRIF), extensive resection of DSB ends, and activation of ATR signaling. In HRR, the 3'-ssDNA overhangs anneal with complementary sequences on sister chromatids, ensuring accurate repair. In contrast, SSA involves annealing the 3'-ssDNA overhangs via homologous direct repeats, leading to the deletion of one repeat and the intervening sequence during the repair process. While HRR and SSA rely on the annealing of long, highly homologous DNA sequences, MMEJ utilizes much shorter homology regions (typically up to 20 nucleotides), making it a more promiscuous repair mechanism prone to joining unrelated DNA fragments. The error-prone nature of MMEJ is further exacerbated by the involvement of the low-fidelity DNA polymerase theta (POLQ), which mediates DNA synthesis during this repair process. For comprehensive reviews on these mechanisms, refer to Khanna KK, Nat Genet 2001; Thompson LH and Schild D, Mutat Res 2001; Thompson LH and Schild D, Mutat Res 2002; Ciccia A and Elledge SJ, Mol. Cell 2010.

A DNA repair modulator is an agent, either naturally occurring or synthetic, that influences the efficiency or dynamics of DNA repair mechanisms. DNA repair modulators can target specific enzymes or proteins involved in DNA repair pathways, such as DNA glycosylases, AP endonucleases, DNA polymerases, and DNA ligases, which work sequentially to recognize, excise, and repair damaged DNA segments. Modulators may either enhance or inhibit the kinetics of DNA repair. Enhancers increase the activity of repair enzymes or facilitate the recruitment of repair complexes to damaged sites, while inhibitors may reduce or inhibit repair processes by interfering with enzymatic activity or repair intermediates.

In embodiments, the method of the present invention involves contacting a cell with one or more DNA-PK inhibitors or DNA PolQ inhibitors.

DNA-PK and DNA PolQ inhibitors are established inhibitors targeting DNA repair pathways. DNA-PK inhibitors are compounds that target DNA-PK, a protein kinase that plays a crucial role in NHEJ. By inhibiting DNA-PK, these compounds can interfere with the repair of DSBs, leading to cell death. Non-limiting examples of DNA-PK inhibitors are NU7026, NU7441, M3814, AZD7648, and KU-0060648.

DNA PolQ is a DNA polymerase that is also involved in NHEJ, but also considered to be important for microhomology-mediated end joining (MMEJ). It is particularly active in repairing DSBs that are difficult to repair by other mechanisms. Inhibitors of DNA PolQ can also interfere with NHEJ and lead to cell death.

Any of DNA-PK inhibitor or DNA PolQ inhibitor known in the art is suitable for the method of the present invention. A skilled person is capable of selecting a suitable inhibitor without undue effort.

In the context of the present invention, the terms "homology arms" or "homology arms that are targeted to the dsDNA molecule to be modified" refer to regions or sequences of the exogenous nucleic acid molecule that are homologous to the sequences in proximity to the two double-strand break ends of the dsDNA molecule to be modified.

Homology arms may have 90 %, preferably 95 %, 97 %, 98 %, 99 %, or 100 % sequence identity to the corresponding sequences of the dsDNA molecule to be modified. Homology arms have sufficient sequence identity to ensure specific binding to the target sequence. Methods to evaluate the identity level between two nucleic acid sequences are well known in the art. For example, the sequences can be aligned electronically using suitable computer programs known in the art. Such programs comprise BLAST (Altschul et al. (1990) J. Mol. Biol. 215, 403), variants thereof such as WU-BLAST (Altschul and Gish (1996) Methods Enzymol. 266, 460), FASTA (Pearson and Lipman (1988) Proc. Natl. Acad. Sci. USA 85, 2444) or implementations of the Smith-Waterman algorithm (SSEARCH, Smith and Waterman (1981) J. Mol. Biol., 147, 195). These programs, in addition to providing a pairwise sequence alignment, also report the sequence identity level (usually in percent identity) and the probability for the occurrence of the alignment by chance (P-value). In accordance with the present invention, BLAST is preferred to be used to determine the level of identification between two nucleic acid sequences.

As used herein, the term "exogenous DNA repair template" refers to an exogenous DNA molecule that is introduced into the cell and that comprises a sequence to be integrated into the dsDNA molecule to be modified (the DNA template sequence). In the context of the present invention, the template sequence replaces a DNA sequence of the dsDNA molecule to be modified that is in proximity to, adjacent to, and/or between the two SSBs introduced into the dsDNA molecule.

In preferred embodiments, the sequence to be replaced is a mutated sequence resulting in a functionally impaired or inactive gene product encoded by the dsDNA molecule to be modified, and the DNA template sequence comprises a corrected/functional version of the respective sequence so that replacement of the mutated sequence by the template sequence leads to expression of a functional gene product by the modified dsDNA molecule.

In further embodiments, the DNA template sequence comprises an endogenous and/or exogenous sequence to the cell. Examples of a sequence to be integrated include DNA sequences encoding a protein, a non-coding RNA (e.g., a microRNA) or a landing pad sequence for a recombinase (e.g. att sites for bacteriophage derived large serine integrases). In embodiments, the template sequence can comprise a coding sequence that is operably linked to a regulatory sequence for controlling gene expression, such as promoter or promoter/enhancer sequence sequences. In embodiments, a reporter gene or reporter cassette can be integrated into the dsDNA to be modified by replacing a DNA sequence in proximity to, adjacent to, and/or between the two SSBs.

The homologous sequences required for recombination, commonly referred to as homology arms, are designed to flank the sequence to be replaced and are homologous to regions located upstream and downstream of the target site. It is understood that these homologous regions are part of the endogenous DNA sequence targeted for modification. In some embodiments, the upstream homology arm consists of a nucleic acid sequence that shares significant sequence identity with the genome sequence upstream of the integration or replacement site within the dsDNA molecule. Likewise, the downstream homology arm corresponds to a nucleic acid sequence homologous to the chromosomal sequence downstream of the integration site. For example, when replacing an exon that harbors one or more mutations, the upstream and downstream homology arms may correspond to the flanking intronic sequences.

In embodiments, the homology arms in the exogenous polynucleotide template have 75%, 80%, 85%, 90%, 95%, or 100% sequence identity with the upstream and downstream sequences of the targeted genome sequence. Preferably, the sequence identity is about 95%, 96%, 97%, 98%, 99%, or 100%.

In certain embodiments, the homology arms of the DNA donor template range from approximately 20 bp to about 5000 bp in length, for example, about 50, 100, 200, 300, 400, 500, 600, 700, 800, 900, 1000, 1100, 1200, 1300, 1400, 1500, 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 4000, 4100, 4200, 4300, 4400, 4500, 4600, 4700, 4800, 4900, or 5000 bp.

In some methods, the exogenous DNA template sequence may further comprise a marker. Such a marker may make it easy to screen for targeted integrations. Examples of suitable markers include restriction sites, fluorescent proteins, or selectable markers, such as (antibiotic) resistance genes. The exogenous DNA donor template of the invention can be constructed using recombinant techniques (see, for example, Sambrook et al., 2001 and Ausubel et al., 1996).

The method of the invention comprises introducing into the cell an exogenous DNA donor template comprising a DNA substitute sequence. The exogenous DNA donor template is an exogenous DNA molecule, not a component of the cell's "original" or "natural" DNA composition, particularly its genome. The DNA donor template comprises or consists of the DNA template sequence.

In the context of the present invention, the "DNA template sequence," which may also be called "DNA substitute sequence" or "replacement sequence," is a DNA sequence that is introduced into the dsDNA molecule to be modified, replacing a DNA sequence of the dsDNA to be modified that is positioned in proximity to, such as adjacent to and/or between the two single-strand breaks (SSBs).

In preferred embodiments, the exogenous DNA donor template consists of the DNA substitute sequence. This is possible, for example, if the exogenous DNA donor template is a linear dsDNA molecule, such as a PCR amplification product or a DNA mini-circle. A mini-circle is a plasmid-like circular DNA with all other parts except the sequence of interest removed. Thus, a single cut can linearize a fragment ready to integrate.

Sequence variants of the claimed nucleic acids, proteins, antibodies, antibody fragments, and/or CARs, for example, those defined by % sequence identity, that maintain similar properties of the invention are also included in the scope of the invention. Such variants, which show alternative sequences, maintain essentially the same properties, such as target specificity, as the specific sequences provided, are known as functional analogs or functionally analogous. Sequence identity relates to the percentage of identical nucleotides or amino acids when carrying out a sequence alignment.

The recitation "sequence identity," as used herein, refers to the extent that sequences are identical on a nucleotide-by-nucleotide basis or an amino acid-by-amino acid basis over a window of comparison. Thus, a "percentage of sequence identity" may be calculated by comparing two optimally aligned sequences over the window of comparison, determining the number of positions at which the identical nucleic acid base (e.g., A, T, C, G, I) or the identical amino acid residue (e.g., Ala, Pro, Ser, Thr, Gly, Val, Leu, He, Phe, Tyr, Trp, Lys, Arg, His, Asp, Glu, Asn, Gin, Cys, and Met) occurs in both sequences to yield the number of matched positions, dividing the number of matched positions by the total number of positions in the window of comparison (i.e., the window size), and multiplying the result by 100 to yield the percentage of sequence identity. Included are nucleotides and polypeptides having at least about 50%, 55%, 60%, 65%, 70%, 75%, 80%, 85%, 90%, 95%, 96%, 97%, 98%, 99% or 100% sequence identity to any of the reference sequences described herein, typically where the polypeptide variant maintains at least one biological activity of the reference polypeptide.

It will be appreciated by those of ordinary skill in the art that, as a result of the degeneracy of the genetic code, there are many nucleotide sequences that encode a polypeptide as described herein. Some of these polynucleotides bear minimal homology or sequence identity to the nucleotide sequence of any native gene. Nonetheless, polynucleotides that vary due to differences in codon usage are specifically contemplated by the present invention. Deletions, substitutions, and other changes in sequence that fall under the described sequence identity are also encompassed in the invention.

### Application

A "chimeric antigen receptor (CAR)" polypeptide comprises an extracellular antigen-binding domain, comprising an antibody or antibody fragment that binds a target antigen, a transmembrane domain, and an intracellular domain. CARs are typically described as comprising an extracellular ectodomain (antigen-binding domain) derived from an antibody and an endodomain comprising signaling modules derived from T-cell signaling proteins.

In a preferred embodiment, the ectodomain preferably comprises variable regions from the heavy and light chains of an immunoglobulin configured as a single-chain variable fragment (scFv). The scFv is preferably attached to a hinge region that provides flexibility and transduces signals through an anchoring transmembrane moiety to an intracellular signaling domain. The transmembrane domains originate preferably from either CD8α or CD28. In the first generation of CARs, the signaling domain consists of the zeta chain of the TCR complex. The term "generation" refers to the structure of the intracellular signaling domains. Second-generation CARs are equipped with a single costimulatory domain originating from CD28 or 4-1 BB. Third-generation CARs already include two costimulatory domains, e.g., CD28, 4-1BB, ICOS or OX40, CD3 zeta. The present invention preferably relates to a second or third-generation CAR, although the antigen-binding fragments described herein may be employed in any given CAR format.

As used herein, the term "chimeric" describes being composed of parts of different proteins or DNAs from different origins.

The "extracellular antigen-binding domain" or "extracellular binding domain" are used interchangeably and provide a CAR with the ability to bind specifically to the target antigen of interest. The binding domain may be derived either from a natural, synthetic, semi-synthetic, or recombinant source. Preferred are scFv domains.

"Specific binding" is to be understood by a person skilled in the art, whereby the skilled person is aware of various experimental procedures that can be used to test binding and binding specificity. Methods for determining equilibrium association or equilibrium dissociation constants are known in the art. Cross-reaction or background binding may be inevitable in many protein-protein interactions; this is not to detract from the "specificity" of the binding between CAR and epitope. "Specific binding" describes the binding of an anti-herpes virus antigen-antibody or antigen-binding fragment thereof (or a CAR comprising the same) to said herpes virus antigen at greater binding affinity than background binding. The term "directed against" is also applicable when considering the term "specificity" in understanding the interaction between antibodies and epitopes.

An "antigen (Ag)" refers to a compound, composition, or substance that can stimulate the production of antibodies or a T cell response in an animal. An "epitope" refers to the region of an antigen to which a binding agent binds. Epitopes can be formed both from contiguous amino acids or noncontiguous amino acids juxtaposed by tertiary folding of a protein. Any suitable antigen can be selected depending on the disease to be treated.

As used herein, the term "genetically engineered" or "genetically modified" refers to the addition of extra genetic material in the form of DNA or RNA into the total genetic material in a cell. The terms, "genetically modified cells," "modified cells," and, "redirected cells," are used interchangeably.

An "immune cell" or "immune effector cell" is any cell of the immune system that has one or more effector functions (e.g., cytotoxic cell killing activity, secretion of cytokines, induction of ADCC and/or CDC).

In embodiments, the immune effector cell is a cytotoxic immune effector cell, preferably a lymphocyte, more preferably a T cell or natural killer (NK) cell.

Immune effector cells of the invention can be autologous/autogeneic ("self') or non-autologous ("non-self," e.g., allogeneic, syngeneic, or xenogeneic). "Autologous," as used herein, refers to cells from the same subject. "Allogeneic," as used herein, refers to cells of the same species that differ genetically from the cell in comparison. "Syngeneic," as used herein, refers to cells of a different subject that are genetically identical to the cell in comparison. "Xenogeneic," as used herein, refers to cells of a different species to the cell in comparison. In preferred embodiments, the cells of the invention are autologous or allogeneic.

Illustrative immune effector cells used with the CARs contemplated herein include T lymphocytes. The terms "T cell" or "T lymphocyte" are art-recognized and are intended to include thymocytes, immature T lymphocytes, mature T lymphocytes, resting T lymphocytes, cytokine-induced killer cells (CIK cells), or activated T lymphocytes. Cytokine-induced killer (CIK) cells are typically CD3- and CD56-positive, non-major histocompatibility complex (MHC)-restricted, natural killer (NK)-like T lymphocytes. A T cell can be a T helper (Th; CD4+ T cell) cell, for example, a T helper 1 (Th1) or a T helper 2 (Th2) cell. The T cell can be a cytotoxic T cell (CTL; CD8+ T cell), CD4+CD8+ T cell, CD4 CD8 T cell, or any other subset of T cells. The T cell can be a CD4+ regulatory T cell (Treg; CD4+CD25+Foxp3+ T cell) or a CD8+ regulatory T cells (CD8+CD25+Foxp3+), Other illustrative populations of T cells suitable for use in particular embodiments include naive T cells and memory T cells.

As would be understood by the skilled person, other cells may also be used as immune effector cells with the CARs described herein. In particular, immune effector cells include NK cells, NKT cells, neutrophils, and macrophages. Immune effector cells also include progenitors of effector cells, wherein such progenitor cells can be induced to differentiate into immune effector cells *in vivo* or*in vitro.*

"Natural Killer (NK) cells," also known as large granular lymphocytes (LGL), are a vital component of the innate immune system, comprising 5-20% of circulating lymphocytes in humans. Functionally analogous to cytotoxic T cells in adaptive immunity, NK cells respond rapidly to virus-infected cells and tumors, acting approximately three days after infection. Unlike most immune cells, NK cells do not require antibodies or major histocompatibility complex (MHC) for target recognition, allowing for a swift immune response. Identified by the presence of CD56 and absence of CD3, NK cells differentiate from CD127⁺ common innate lymphoid progenitors and mature in various tissues before entering circulation. CD56^{bright} NK cells, predominant in bone marrow and lymphoid tissues, exhibit immunoregulatory roles, while CD56dim NK cells in peripheral blood excel in cell killing. There are various sources from which NK cells can be derived, e.g., peripheral blood mononuclear cells, cord blood, immortalized cell lines, hematopoietic stem and progenitor cells (HSPCs), and induced pluripotent stem cells (iPSCs). All sources can provide clinically meaningful cell doses, are amenable to CAR receptor engineering, and have transitioned into in-human studies. NK cells express distinct receptors, including inhibitory receptors recognizing self-MHC I and activating receptors (Raftery MJ, Ann Rev of Cancer Biol, 2023). Activating molecules may include the natural cytotoxicity receptors, NKG2D, some CD94/NKG2 complexes, and CD16 (Fcγlll). Activating molecules recognize partner ligands on cells except for CD16, a low-affinity Fc receptor responsible for antibody-dependent cellular cytotoxicity (ADCC). Inhibitory molecules may include the killer cell immunoglobulin-like receptor (KIR) family of molecules, some CD94/NKG2 complexes, and leukocyte Ig-like receptors (LIRs). These receptors govern NK cell activity, adjusting to environmental cues. NK cells contribute to both innate and adaptive immunity, demonstrating antigen-specific immunological memory.

### General remarks

All words and terms used herein shall have the same meaning commonly given to them by the person skilled in the art unless the context indicates a different meaning. All terms used in the singular shall include the plural of that term and vice versa.

It will be understood that particular embodiments described herein are shown by way of illustration and not as limitations of the invention. The principal features of this invention can be employed in various embodiments without departing from the scope of the invention. Those skilled in the art will recognize or be able to ascertain, using most routine study, numerous equivalents to the specific procedures described herein. Such equivalents are considered to be within the scope of this invention and are covered by the claims. All publications and patent applications mentioned in the specification indicate the skill level of those skilled in the art to which this invention pertains. All publications and patent applications are herein incorporated by reference to the same extent as if each individual publication or patent application was specifically and individually indicated to be incorporated by reference.

The use of the word "a" or "an" when used in conjunction with the term "comprising" in the claims and/or the specification may mean "one," but it is also consistent with the meaning of "one or more," "at least one," and "one or more than one." The term "or" in the claims is used to mean "and/or" unless explicitly indicated to refer to alternatives only or the alternatives are mutually exclusive. However, the disclosure supports a definition of only alternatives and "and/or." Throughout this application, where relevant, the term "about" indicates that a value includes the inherent variation of error for the device, the method employed to determine the value or the variation among the study subjects.

### FIGURES

The invention is demonstrated by way of example in the following figures. The figures are to provide a further description of potentially preferred embodiments that enhance the support of one or more non-limiting embodiments of the invention.

### Brief description of the figures:

**Figure 1****:** Base editing mediated knock-in (BEKI) into the *TRAC* locus.
**Figure 2****:** Base editing mediated knock-in (BEKI) of a truncated CAR into *CD3ζ* and HLA-E into *B2M.*
**Figure 3****:** Enhancing homology directed repair (HDR) for BEKI by using DNA-PK and PolQ inhibitors and Cas target sequences (CTS).
**Figure 4****:** Enhancement of BEKI using cas target sequences (CTS).
**Figure 5****:** Base editing mediated knock-in (BEKI) can be combined with multiple base edits for functional enhancement of T cell therapies.
**Figure 6****:** BEKI mediated multiplexed KI and KO reduced the risk for chromosomal translocations compared to multiplexed editing using Cas9.
**Figure 7****:** Quintuple BEKI-edited CAR T cells resist allo-specific immune rejection.
**Figure 8****:** BEKI mediated simultaneous double knock-in of a truncated CAR into CD3ζ and HLA-E into B2M.

### Detailed description of the figures:

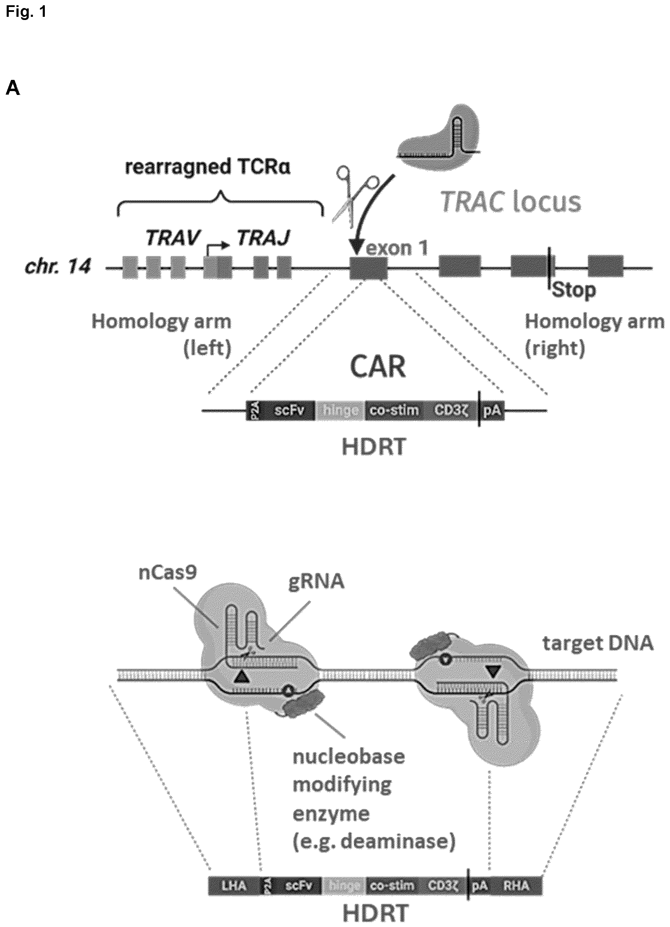

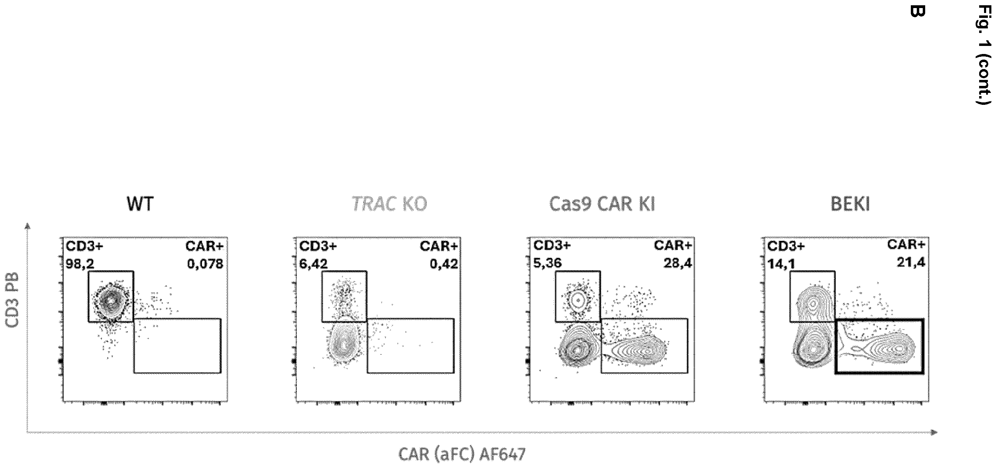

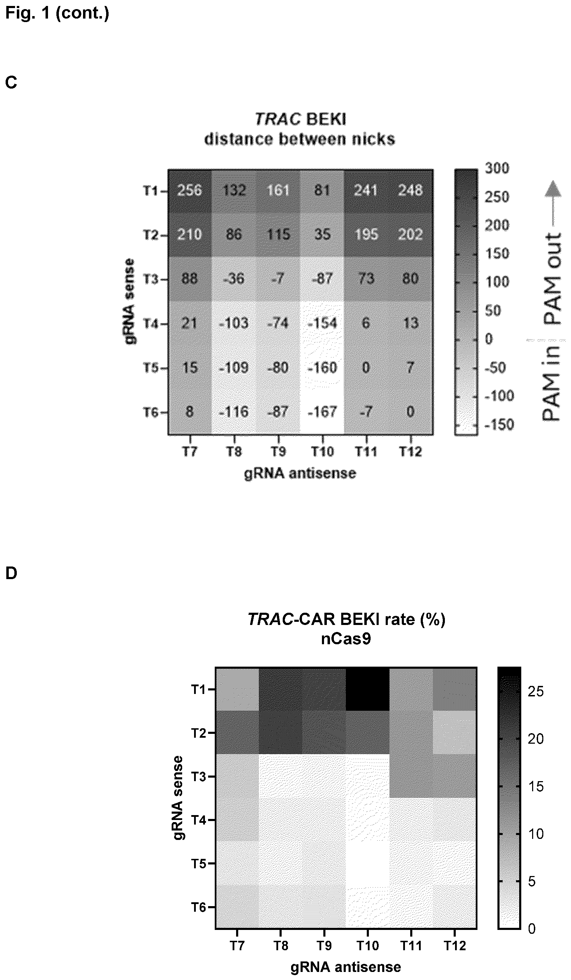

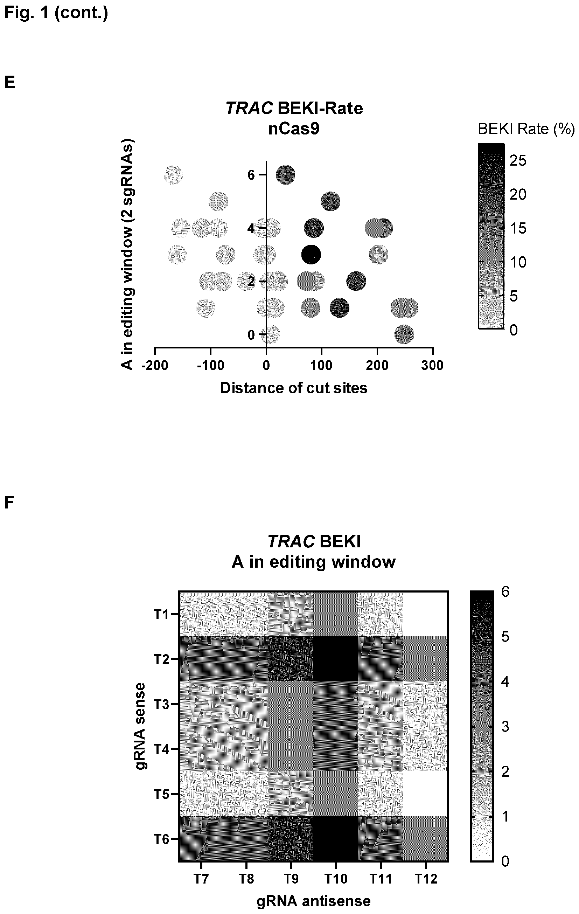

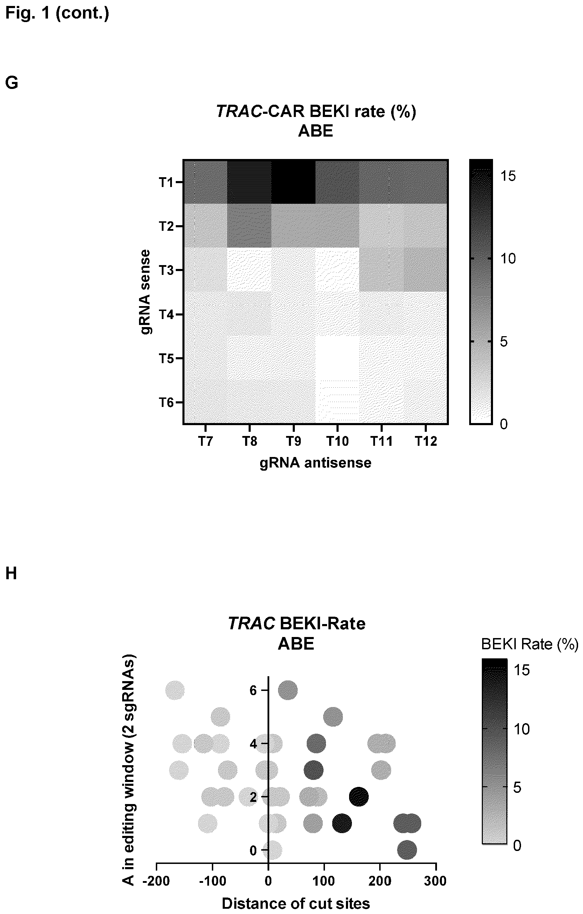

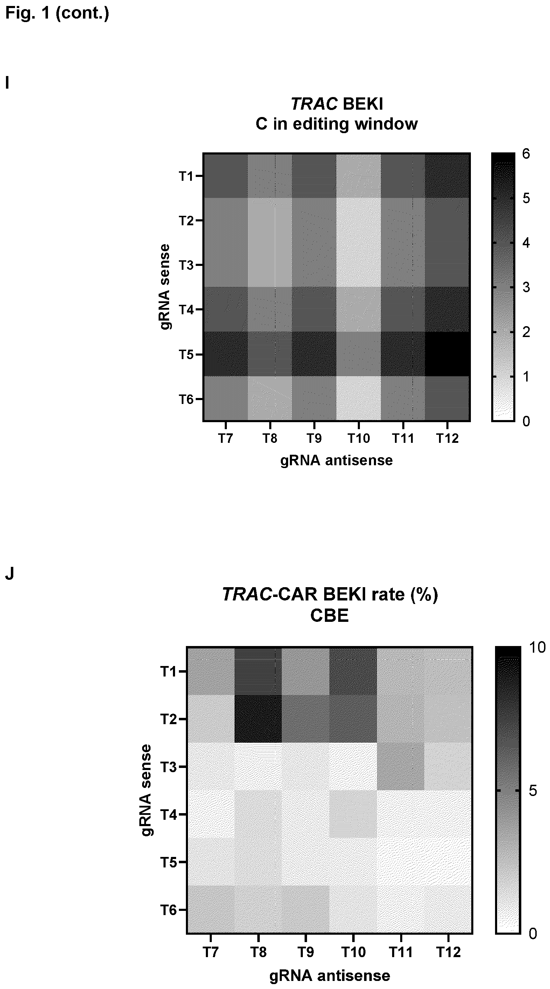

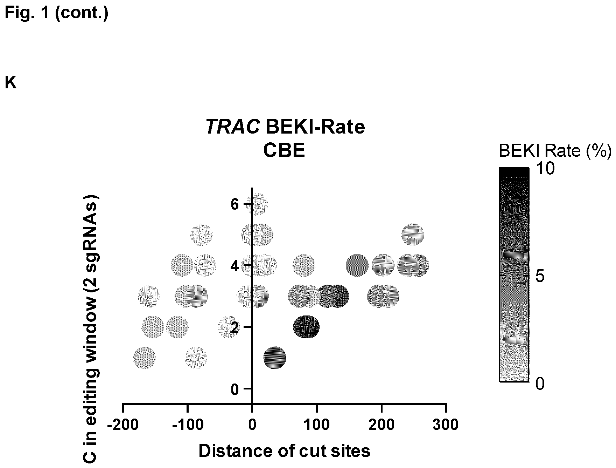

**Figure 1****: Base editing mediated knock-in (BEKI) into the *TRAC* locus.** (A) Non-viral knock-in strategy to integrate a full-length CAR into the *TRAC* locus by using Cas9 precomplexed with a sgRNA targeting exon 1 (left) and integration strategy harnessing two nickase Cas9 (nCas9) base editors for base editing mediated knock-in (BEKI) (right). (B) Flow cytometric analysis after knock-in. CAR transgene integration into *TRAC* disrupts expression of the TCR/CD3 complex. (C) Heat map of the distance between the DNA nicks created using the combination of one sgRNA complementary to the sense strand and one sgRNA complementary to the antisense strand. The 'PAM in' orientation resulting from a gRNA complementary to the sense strand binding downstream of a gRNA complementary to the antisense strand is depicted as negative distance between the nicks. (D) KI efficiency of a CAR into *TRAC* determined by flow cytometry for different sgRNA combinations after nCas9 mediated KI. (E) Bubble Plot displaying the Kl-rate of a CAR into *TRAC* in relation to the distance between the two DNA nicks and the adenines in the editing window. (F) Heat map of Adenine in editing window for different sgRNA combinations. (G) Heat map of flow cytometry KI data for different sgRNA combinations after adenine base editing mediated KI (BEKI). (H) Bubble Plot displaying the CAR BEKI-rate in relation to the distance between the two DNA nicks and the number of potential base edits in the editing window. (I) Heat map of cytosine in editing window for different sgRNA combinations. (J) Heat map of flow cytometry KI data for different sgRNA combinations after cytosine base editing mediated KI (BEKI). (K) Bubble Plot displaying the CAR BEKI-rate in relation to the distance between the two DNA nicks and the number of potential base edits in the editing window. (n=2 healthy donors, 1 µg DNA per electroporation, no HDR enhancing agents)

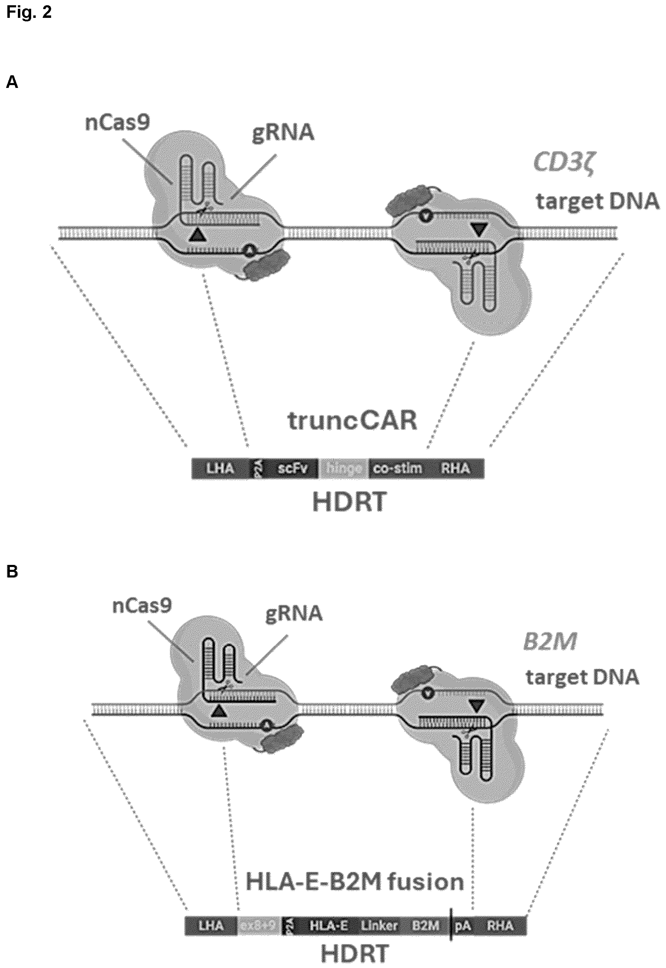

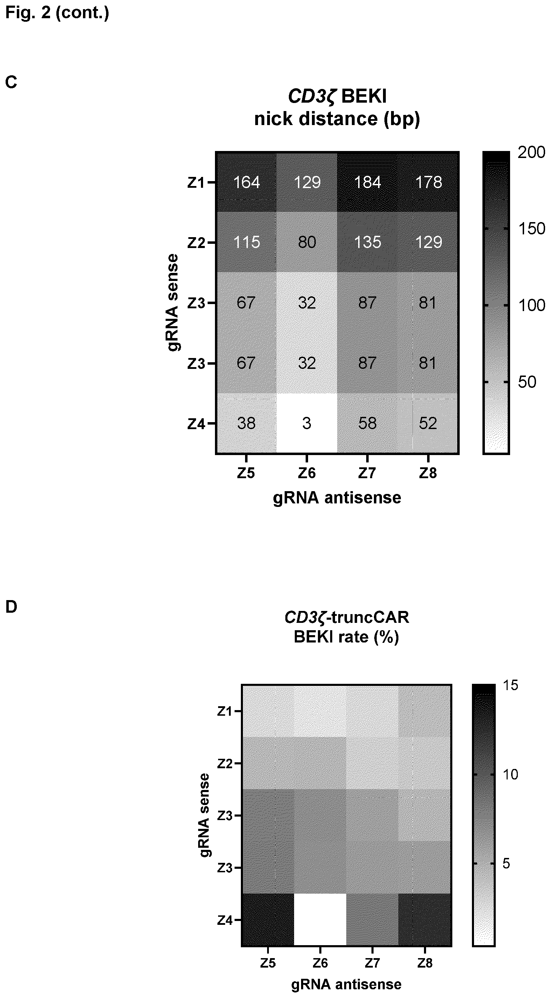

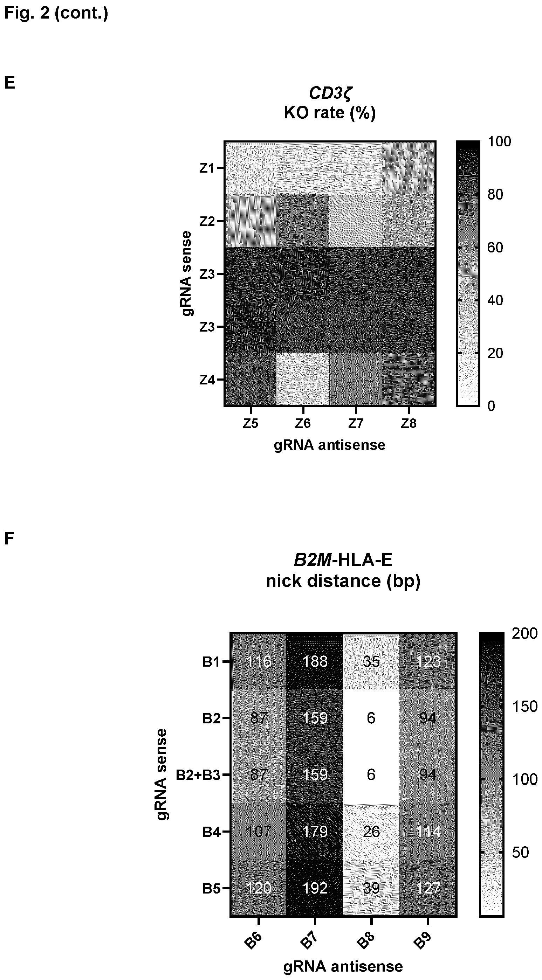

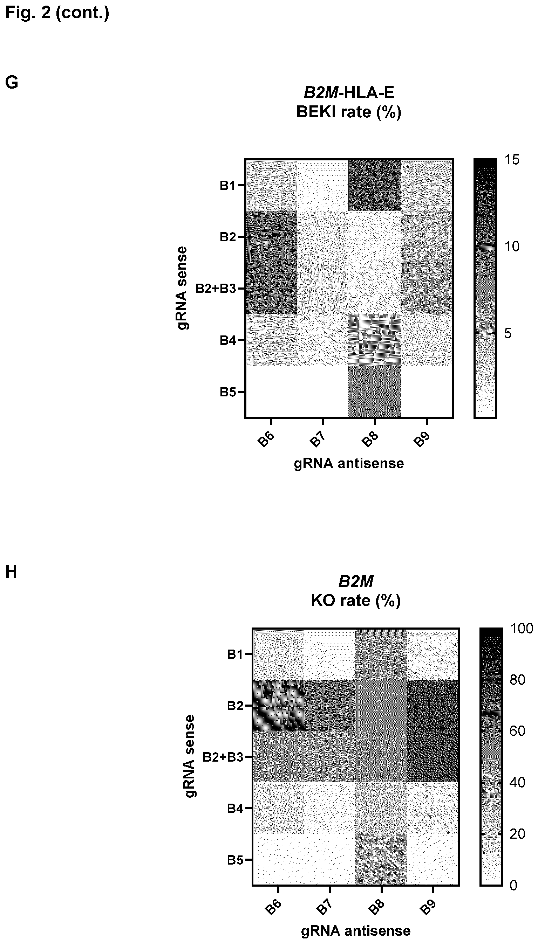

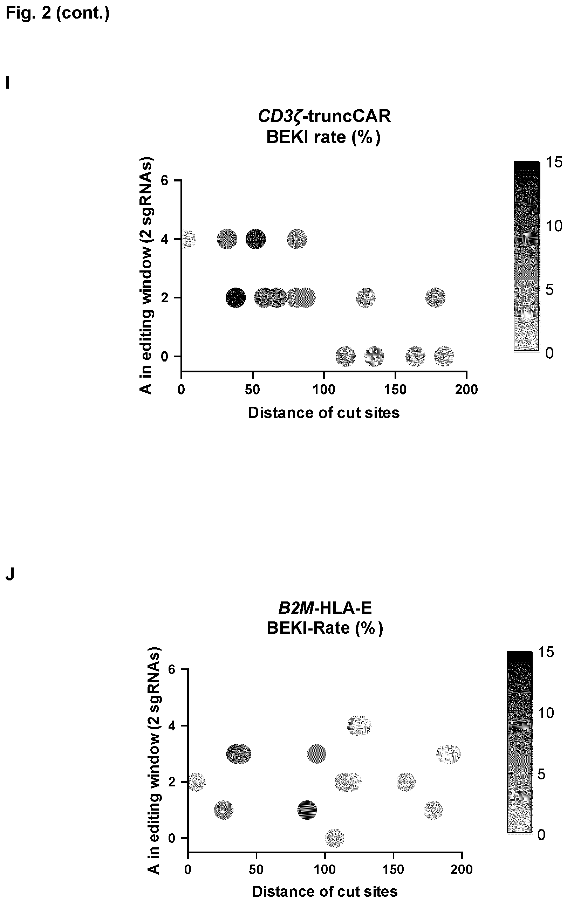

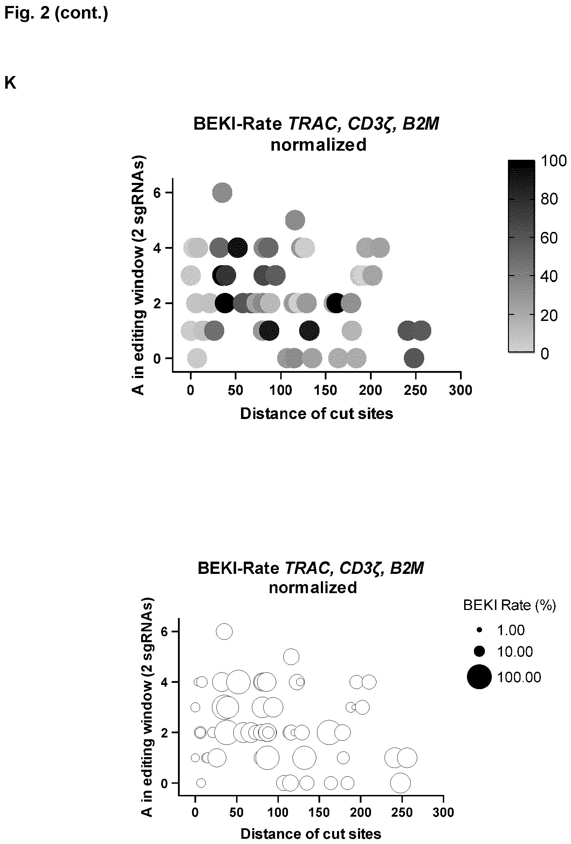

**Figure 2****: Base editing mediated knock-in (BEKI) of a truncated CAR into *CD3ζ* and HLA-E into *B2M.*** (A) BEKI strategy to integrate a truncated CAR *(truncCAR)* into *CD3ζ.* (B) BEKI strategy to insert HLA-E with a short linker sequence into B2M, generating an HLA-E-B2M fusion protein. (C) Heat map of the distance between the DNA nicks created using the combination of one sgRNA complementary to the sense strand and one sgRNA complementary to the antisense strand of the *CD3ζ* gene. The PAM in orientation resulting from a sense strand complementary sgRNA that binds downstream of an antisense complementary sgRNA is depicted as negative distance between the nicks. (D) KI efficiency determined by flow cytometry for different sgRNA combinations after BEKI of a *truncCAR* into *CD3ζ.* (E) Heat map of CD3 knock-out (KO) rate for different sgRNA combinations after *CD3ζ* BEKI. (F) Heat map of the distance between the DNA nicks created using the combination of one sgRNA complementary to the sense strand and one sgRNA complementary to the antisense strand of the *B2M* gene. The PAM in orientation resulting from a sense strand complementary sgRNA that binds downstream of an antisense complementary sgRNA is depicted as negative distance between the nicks. (G) KI efficiency determined by flow cytometry for different sgRNA combinations after BEKI of HLA-E into *B2M.* (H) Heat map of B2M knock-out (KO) rate for different sgRNA combinations after *B2M* BEKI. (I) Bubble Plot displaying the BEKI-rate of a *truncCAR* into *CD3ζ* in relation to the distance between the two DNA nicks and the number of potential base edits in the editing window. (J) Bubble Plot displaying the BEKI-rate of HLA-E into *B2M* in relation to the distance between the two DNA nicks and the number of potential base edits in the editing window. (K) Summary bubble Plots using color (left) or size (right) displaying the BEKI-rate of a CAR into *TRAC, truncCAR* into CD3ζ and HLA-E into *B2M* in relation to the distance between the two DNA nicks and the number of potential base edits in the editing window. (D, E, I: n=4 healthy donors, 2 µg DNA per electroporation, no HDR enhancing agents; G, H, J: n=2 healthy donors, 2 µg DNA per electroporation, no HDR enhancing agents)

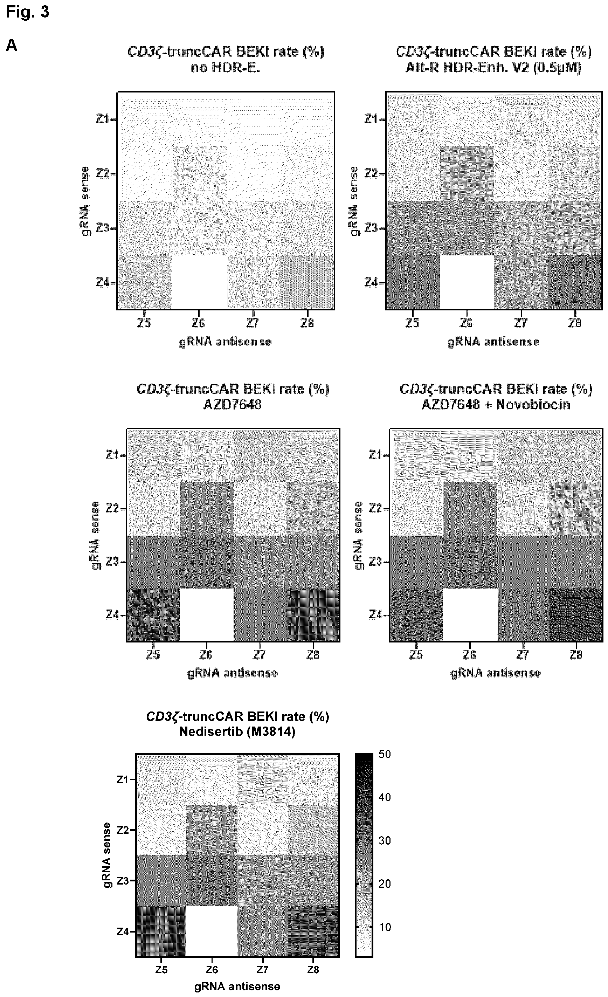

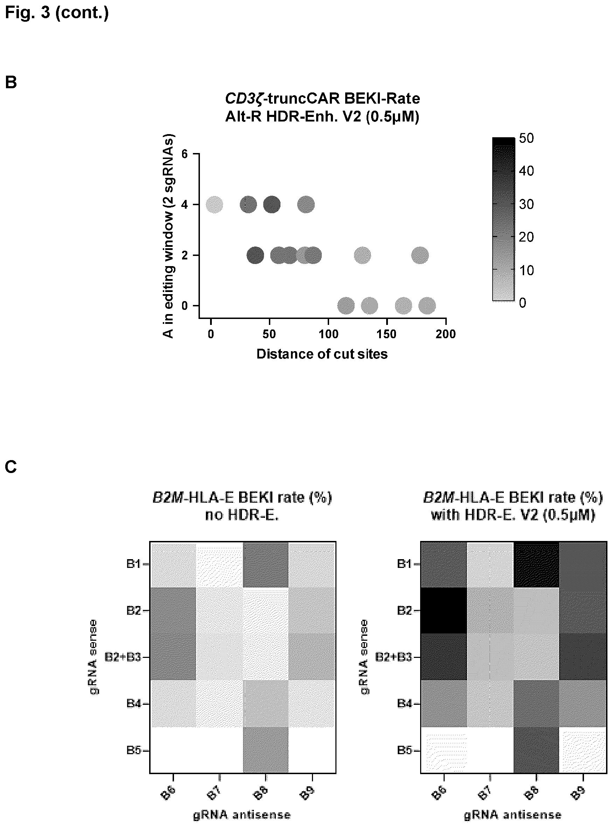

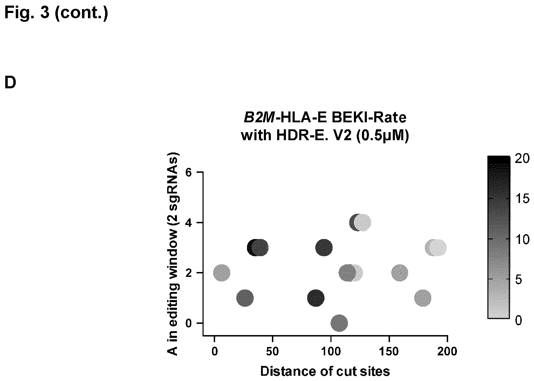

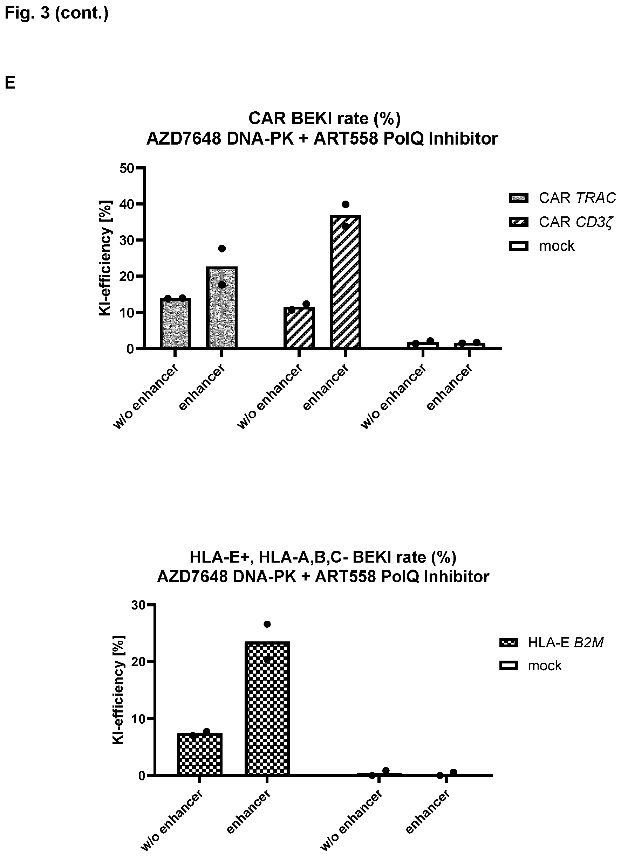

**Figure 3****: Enhancing homology directed repair (HDR) for BEKI by using DNA-PK and PolQ inhibitors and Cas target sequences (CTS).** (A) Heat map of BEKI rate for different sgRNA combinations after BEKI of a *truncCAR* into *CD3ζ* without pharmaceutical enhancement of HDR and with treatment of 0.5µM Alt-R HDR-Enh. V2 (Integrated DNA Technologies [IDT]), DNA-PK inhibitor AZD7648, DNA-PK inhibitor AZD7648 and Pol-Q inhibitor Novobiocin or DNA-PK inhibitor Nedisertib (M3814) (left to right). (B) Bubble Plot displaying the BEKI-rate of a *truncCAR* into *CD3ζ* in relation to the distance between the two DNA nicks and the number of potential base edits in the editing window with treatment of 0.5µM Alt-R HDR-Enh. V2 (IDT). (C) Heat map of BEKI rate for different sgRNA combinations after BEKI of HLA-E into *B2M* without pharmaceutical enhancement of HDR (left) and with treatment of 0.5µM Alt-R HDR-Enh. V2 (IDT) (right). (D) Bubble Plot displaying the BEKI-rate HLA-E into *B2M* in relation to the distance between the two DNA nicks and the number of potential base edits in the editing window with treatment of 0.5µM Alt-R HDR-Enh. V2 (IDT). (E) BEKI efficiency of a CAR into *TRAC* and a *truncCAR* into *CD3ζ* (left) or HLA-E into *B2M* (right) without or with addition of DNA-PK inhibitor AZD7648 and Pol-Q inhibitor ART558. (A, B, C, D: n=2 healthy donors, 2 µg DNA per electroporation; E: n=2 healthy donors, 1 µg DNA per electroporation)

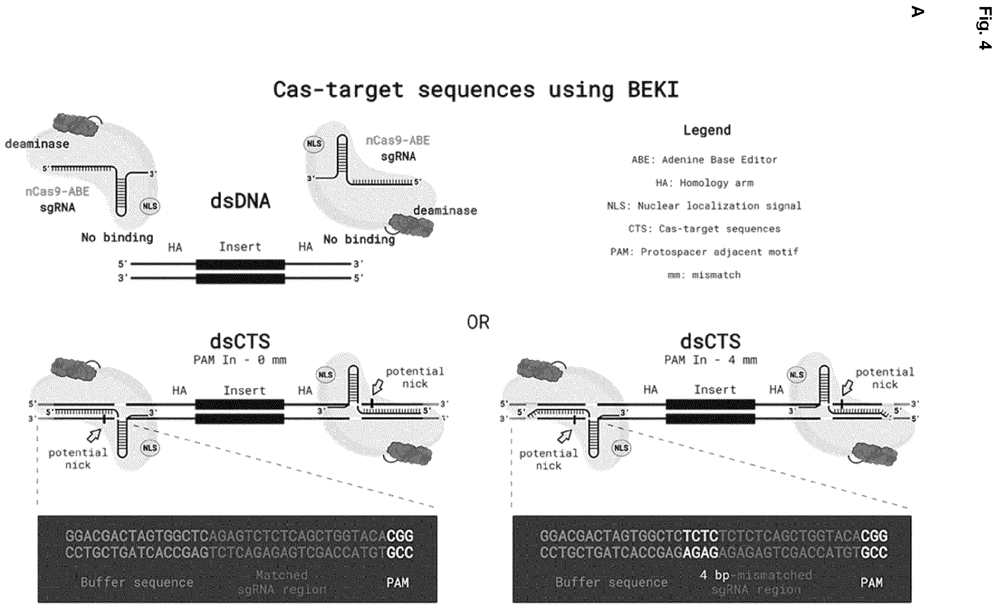

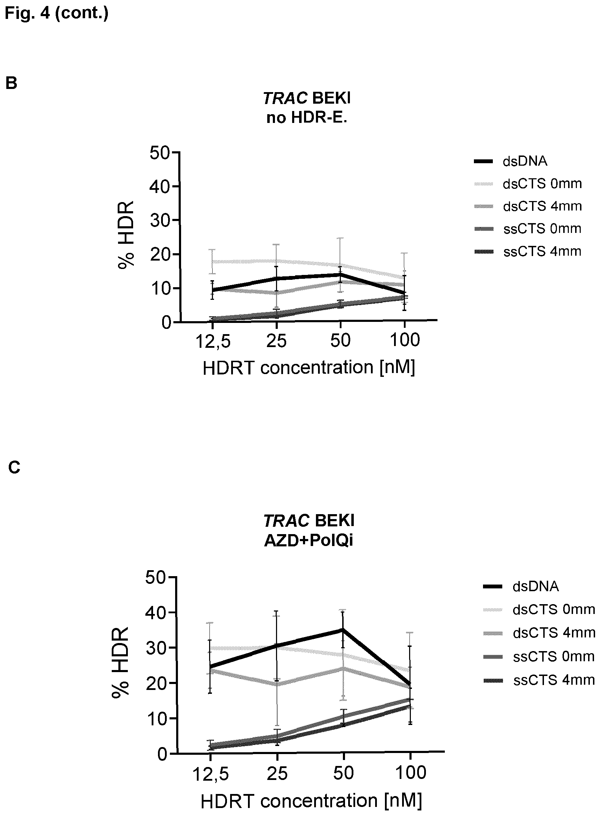

**Figure 4****: Enhancement of BEKI using cas target sequences (CTS).** (A) Cas target sequence (CTS) design for binding of the base editor sgRNA complex to the HDRT in order to enhance transport of the template to the nucleus via the nuclear localization sequence (NLS) (B) BEKI rate using dsDNA, dsCTS 0mm, dsCTS 4mm, ssCTS 0mm and ssCTS 4mm HDRTs without pharmaceutical enhancement of HDR (n = 3 healthy donors). (C) BEKI rate using dsDNA, dsCTS 0mm, dsCTS 4mm, ssCTS 0mm and ssCTS 4mm HDRTs with treatment of DNA-PK inhibitor AZD7648 and Pol-Q inhibitor ART558 (n = 3 healthy donors). Thick lines indicate mean values, error bars indicate Standard error of the mean (SEM).

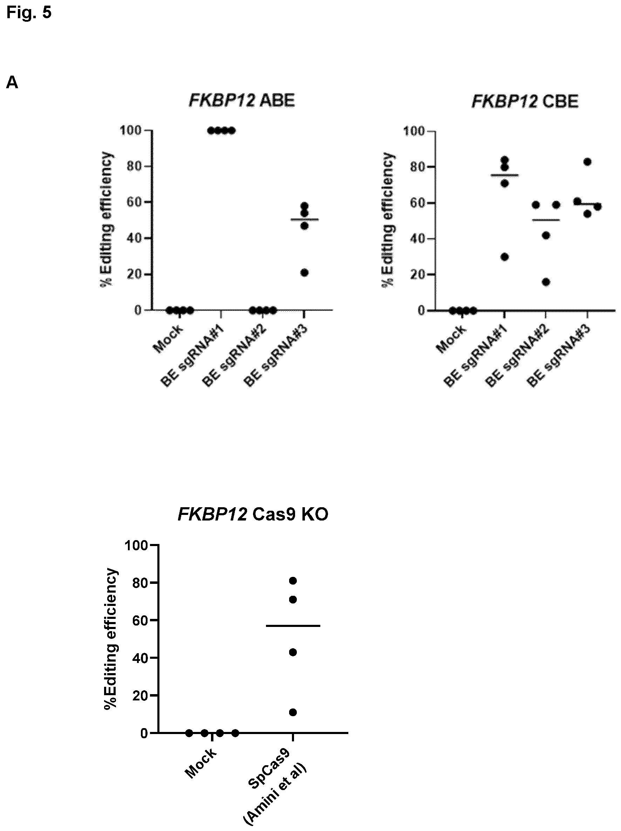

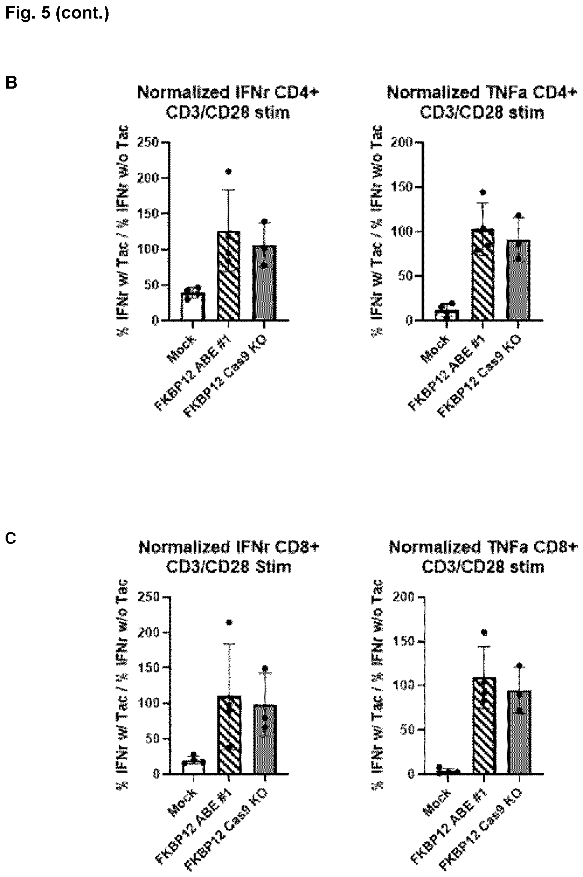

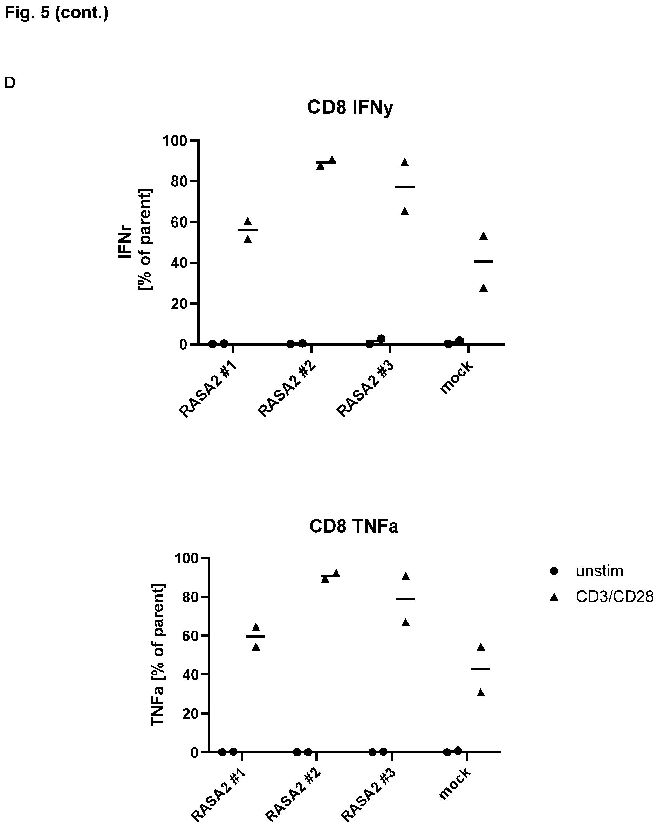

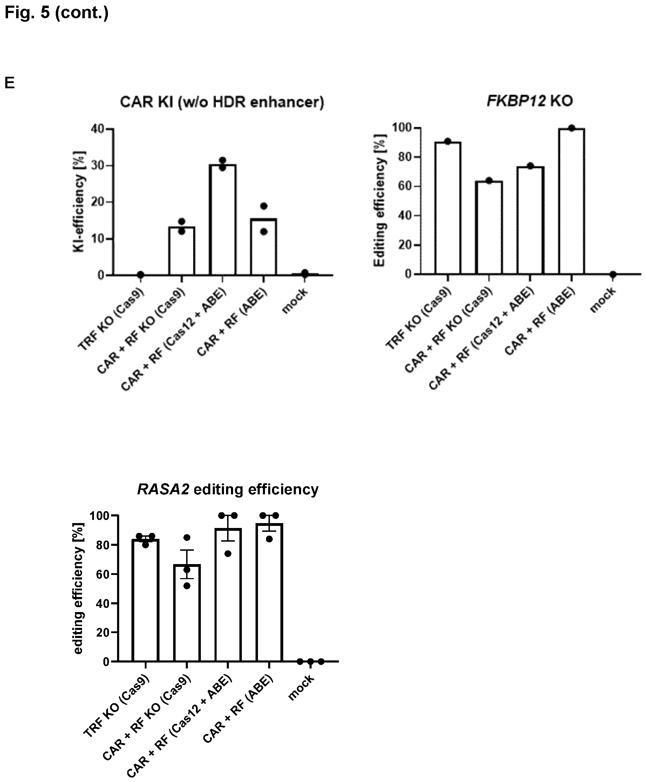

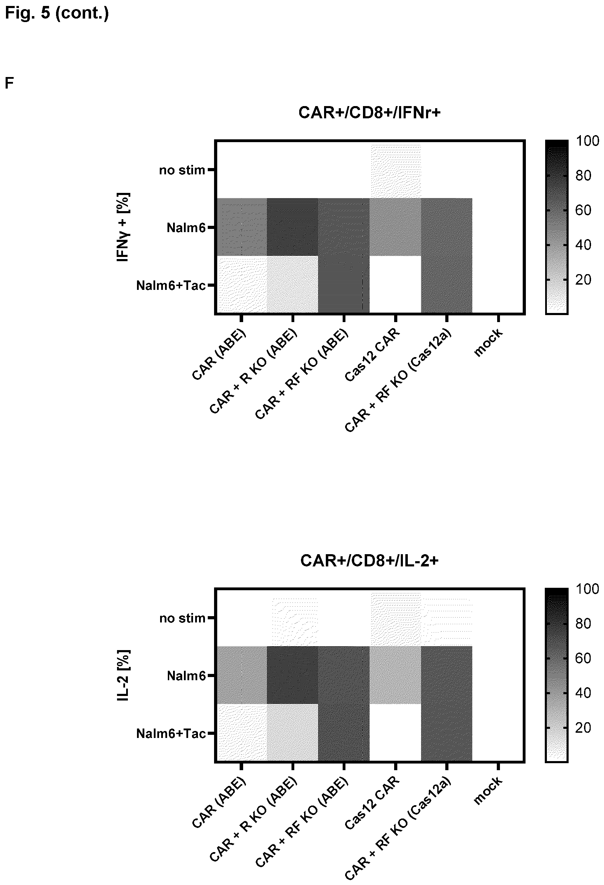

**Figure 5****: Base editing mediated knock-in (BEKI) can be combined with multiple base edits for functional enhancement of T cell therapies.** (A) *FKBP12* ABE efficiency using sgRNA #1 with an NGG PAM base editor (ABE8.20m) and sgRNA #2 and #3 with an NG PAM base editor (ABEmax, nSpCas9-VRQR) (left). *FKBP12* CBE efficiency using sgRNA #1 with an NGG PAM base editor (BE4) and sgRNA #2 and #3 with an NG PAM base editor (CBE4max-SpG) (middle). *FKBP12* KO efficiency after SpCas9 cleavage using the *FKBP12* sgRNA published by Amini et al. (right). (B) Frequency of mock, ABE *FKBP12* KO or Cas9 mediated *FKBP12* KO CD4+ T cells producing IFNy (left) or TNFα (right) after CD3/CD28 stimulation in presence of Tacrolimus normalized to cytokine production without the treatment of tacrolimus. (E) Frequency of mock, ABE *FKBP12* KO or Cas9 mediated *FKBP12* KO CD8+ T cells producing IFNy (left) or TNFa (right) after CD3/CD28 stimulation in presence of Tacrolimus normalized to cytokine production without the treatment of tacrolimus. (D) Frequency of CD8+ T cells without or with ABE mediated *RASA2* KO using sgRNA #1 ,#2 or #3 producing IFNy (left) or TNFα (right) with or without CD3/CD28 stimulation. (E) CAR KI efficiency (left), *FKBP12* KO efficiency or *RASA2* KO efficiency of T cells modified by Cas9 *TRAC, RASA2* and FKBP12 triple KO (TRF KO), Cas9 CAR into *TRAC* plus additional *RASA2* and *FKBP12* KO (Cas9 CAR+RF KO), Cas12a CAR into *TRAC* plus additional *RASA2* and *FKBP12* KO via base editing (Cas12a CAR+RF BE), BEKI CAR into *TRAC* plus additional *RASA2* and *FKBP12* KO via base editing (BEKI CAR+RF BE). (F) Frequency of CD8+ CAR T cells producing IFNy (left) or IL-2 (right) with or without stimulation with Nalm6 cells in presence or absence of tacrolimus. *TRAC* CAR T cells were compared to CAR T cells with additional *RASA2* KO or combined *RASA2-* and *FKBP12-KO.* (A: n=4, B, C: n=3-4, D: n=2, E: n=1-3, F: n=1 healthy donors)

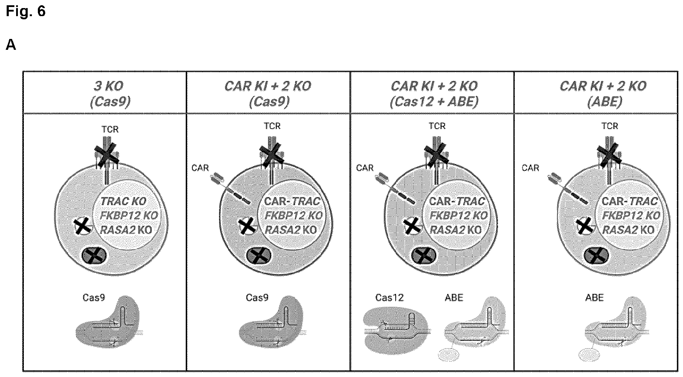

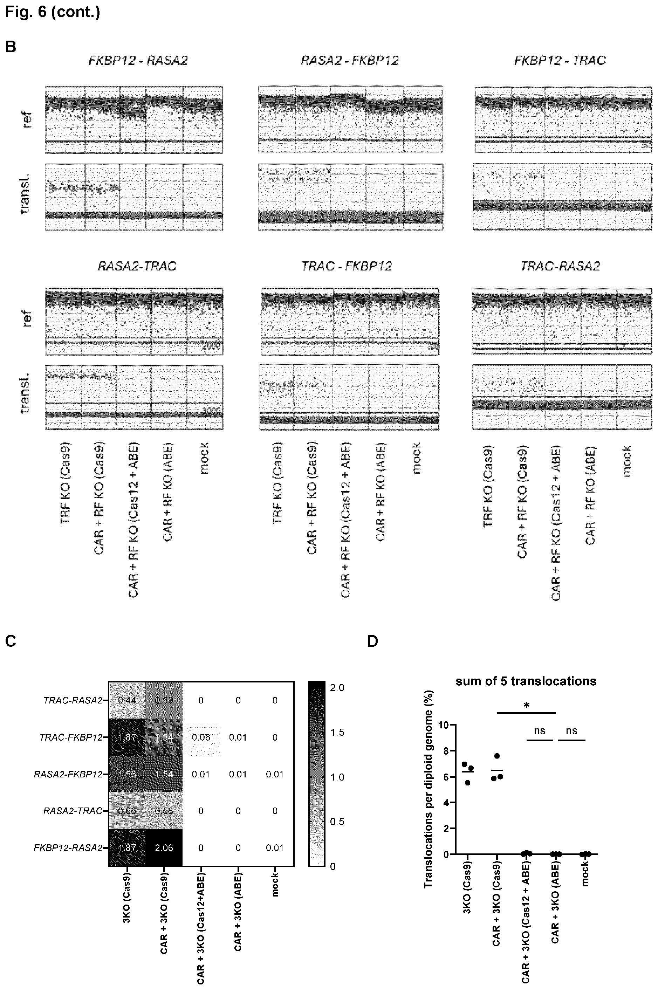

**Figure 6****: BEKI mediated multiplexed KI and KO reduced the risk for chromosomal translocations compared to multiplexed editing using Cas9.** (A) Scheme highlighting four different conditions compared by ddPCR analysis. TRF KO (Cas9): T cells with 3 KOs *(TRAC, FKBP12* and *RASA2)* using conventional Cas9 nuclease. CAR-KI + RF-KO (Cas9): *TRAC-*CAR knock-in (KI) combined with 2 KO *(FKBP12, RASA2)* with conventional Cas9 nuclease. CAR KI + RF KO (Cas12 + ABE): Cas12a-mediated KI of the CAR into TRAC combined with simultaneous KO of *RASA2* and *FKBP12* using ABE8.20m. CAR + RF KO (ABE) / BEKI : ABE-mediated KI into TRAC combined with ABE-mediated KO of *RASA2* and *FKBP12.* (B) ddPCR results of the 6 balanced translocations between the cut sites at the *TRAC, FKBP12 and RASA2* loci showing the reference gene *RPP30* (top) and the translocation (bottom) in T cells harvested on day 14 after Cas9 *TRAC, RASA2* and *FKBP12* triple KO (TRF KO), Cas9 CAR into *TRAC* plus additional RASA2 and *FKBP12* KO (Cas9 CAR+RF KO), Cas12 CAR into *TRAC* plus additional *RASA2* and *FKBP12* KO via base editing (Cas12 CAR+RF BE), BEKI CAR into *TRAC* plus additional *RASA2* and *FKBP12* KO via base editing (BEKI CAR+RF BE) (n=1 healthy donor). (C) Heat map showing the mean frequency of translocations detected by ddPCR and quantified using the reference gene *RPP30* (n=3 healthy donors). (D) Summary of 5 balanced translocations across different conditions (n=3 healthy donors, biological replicates).

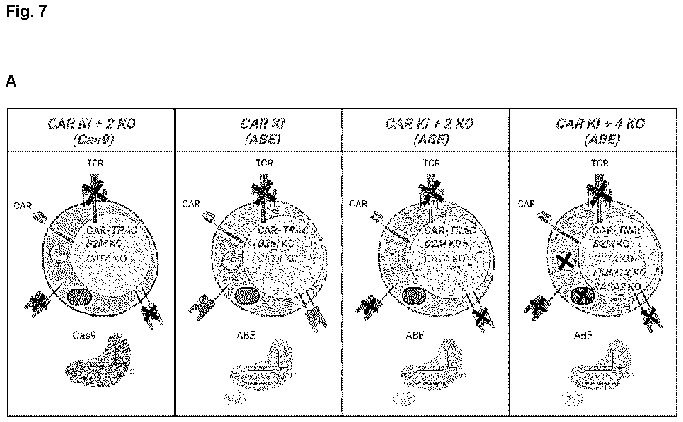

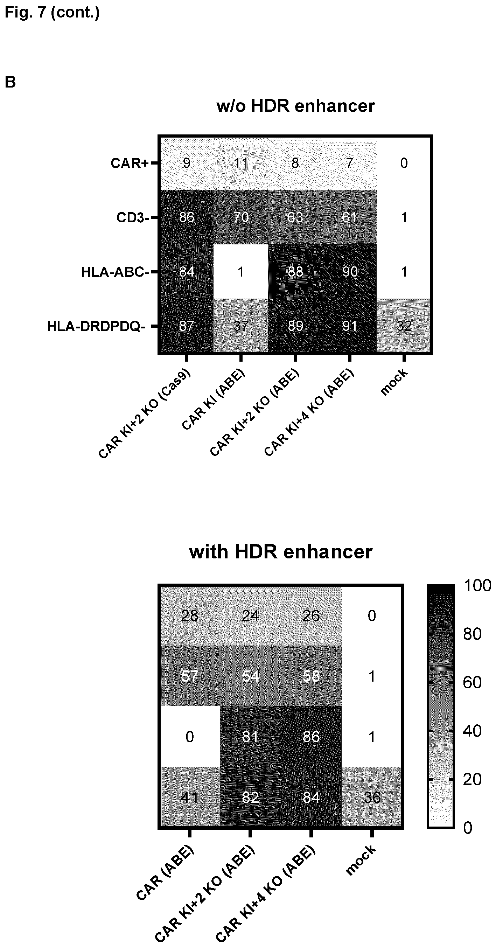

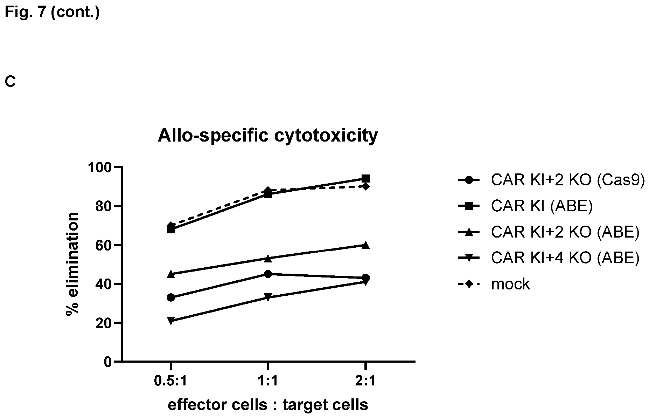

**Figure 7****: Quintuple BEKI edited CAR T cells resist allo-specific immune rejection.** (A) Scheme highlighting four different T cell preparations generated for testing resistance to allo-reactive killing. CAR-KI + 2 KO (Cas9): T cells with CD19-CAR KI into *TRAC* and *B2M* and *CIITO KO's* using conventional Cas9 nuclease. CAR-KI (ABE): TRAC-CAR KI using BEKI approach using ABE8.20m. CAR + 2 KO (ABE): BEKI-mediated of the CAR into *TRAC* combined with simultaneous KO of *B2M* and *CIITA* using ABE8.20m. CAR + 4 KO (ABE): BEKI-mediated of the CAR into *TRAC* combined with simultaneous KO of *B2M* and *CIITA* (for HLA-editing) and KO's for *RASA2* and *FKBP12* using ABE8.20m = 5 edited loci. (B) Heat map showing mean editing frequency for CAR-KI rate, CD3/TRAC-KO rate, HLA-A,B,C-expression and HLA-DR,DQ,DP-expression in different edited T cell conditions treated with or without HDR enhancer (AZD7648 and ART558) (n= 3 healthy donors). (C) Cytotoxicity assay evaluating %-eliminated T cells after overnight co-culture with allo-specific allogeneic HLA-mismatched T cells at different effector:target ratios. Allo-specific T cells were generated by 2 stimulations with irradiated T-cell-depleted PBMCs of the same donor used to create the CAR T cells.

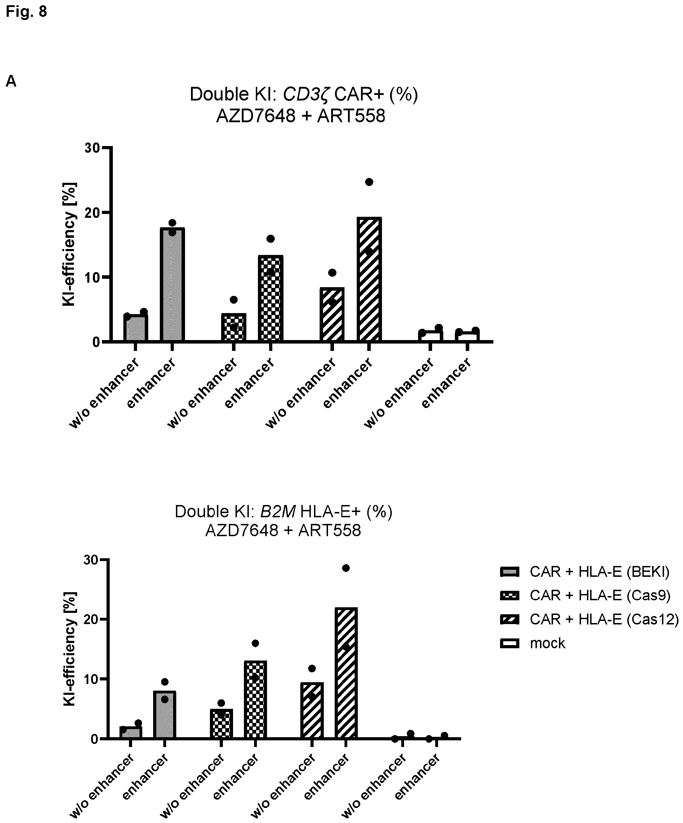

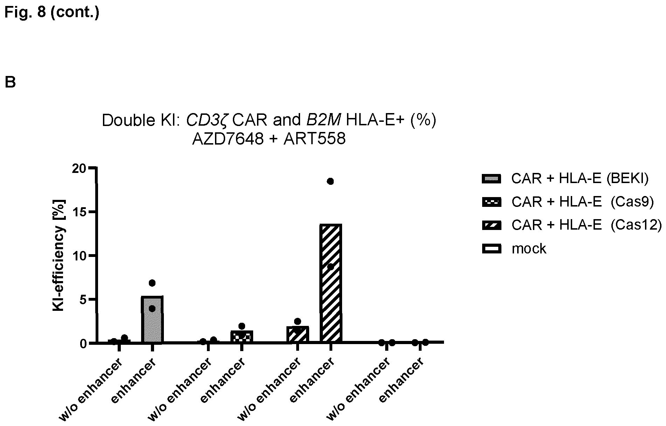

**Figure 8****: BEKI mediated simultaneous double knock-in of a truncated CAR into CD3ζ and HLA-E into *B2M.*** (A) BEKI efficiency of a truncCAR into *CD3ζ* (left) or HLA-E into *B2M* (right) after performing *CD3ζ* and *B2M* double KI using BEKI, Cas9 or Cas12a without or with addition of DNA-PK inhibitor AZD7648 and Pol-Q inhibitor ART558. (B) BEKI efficiency of cells carrying both a truncCAR into *CD3ζ* and HLA-E into *B2M* after performing *CD3ζ* and *B2M* double KI using BEKI, Cas9 orCas12a without or with addition of DNA-PK inhibitor AZD7648 and Pol-Q inhibitor ART558. (n=2 healthy donors, 1 µg DNA per electroporation).

### EXAMPLES

The invention is demonstrated by way of the examples disclosed below. The examples provide technical support for and a more detailed description of potentially preferred, non-limiting embodiments of the invention.

### Summary of the Examples

In order to demonstrate the functionality and beneficial properties of the in vitro method for modifying double-stranded DNA (dsDNA) in a eukaryotic cell described herein, the following examples are to be considered:
- T cell isolation and cell culture
- In vitro transctiption for mRNA production
- Design of DNA templates for homology-directed insertion of transgenes
- Generation of HDRT
- HDR-templates with Cas target sequences
- Gene editing
- Flow cytometry
- Intracellular cytokine detection
- Digital droplet polymerase chain reaction (ddPCR) for translocation quantification
- Sanger sequencing - indel and base editing quantification
- Data analysis, statistics, and presentation
- Combination of two sgRNAs and a nickase Cas9 base editor enables knock-in of transgenes
- Base editor knock-ins require distinct sgRNA designs for efficient editing with both adenine base editors and cytosine base editors
- BEKI can be designed for knock-in of different transgenes and target sites and can be optimized to provide simultaneous knock-out of the endogenous gene at the integration locus
- BEKI rates are increased by exposing cells to DNA repair modulating agents that inhibit non-homologous end joining and microhomology-mediated end joining
- BEKI rates can be enhanced by modification of the non-viral templates with Cas-target sequence motifs
- BEKI can be combined with additional base edits to force gene knock-outs or to induce gain-of-function or loss-of-function mutations alongside transgene integration
- Combining BEKI with additional simultaneous edits in a single transfection prevents translocations
- BEKI can be used to integrate two transgenes at different loci simultaneously while reducing translocations between the different integration sites

### Example 1: T cell isolation and cell culture

Peripheral blood mononuclear cells (PBMCs) were isolated from the peripheral blood of healthy donors via density gradient centrifugation using Pancoll separation solution (Pan Biotech) in 50mL Leucosep Tubes (Greiner). CD3+ T cells were positively enriched using magnetic column enrichment with human CD3 microbeads according to the manufacturer's recommendations (LS columns, Miltenyi Biotec). T cells were cultured in T cell medium containing a 1:1 mixture of advanced RPMI (Gibco, Thermo Fisher Scientific) and Click's (Fujifilm Irvine Scientific, Santa Ana, CA) media supplemented with 10% inactivated fetal calf serum (FCS) (Biochrom), 1% Glutamax (Thermo Fisher Scientific), interleukin-7 (IL-7) (10 ng/mL, Sartorius CellGeni) and IL-15 (5 ng/mL, Sartorius CellGenix). All cell culture experiments were performed at 37°C and 5% CO2. Polyclonal T-cell stimulation was performed for 48 h on anti-CD3/anti-CD28-coated tissue culture plates. Coating of vacuum gas plasma-treated polystyrene Tissue-Culture plates (Corning) was performed overnight with sterile ddH2O (Ampuwa) supplemented with 1 µg/mL anti-CD3 monoclonal antibody (mAb) (clone OKT3; Invitrogen) and 1 µg/mL anti-CD28 mAb (clone CD28.2; BioLegend). Plates were washed in PBS before seeding the T cells at a density of 0.75 × 106 cells/mL. The Nalm-6 cell line was cultured in RPMI medium (Gibco, Thermo Fisher Scientific) supplemented with 10% FCS.

### Example 2: In vitro transcription for mRNA production

The ABE8.20-m, BE4-RrA3F, CBE4max-SpG and ABEmax(7.10)-SpCas9-NG plasmids was kindly provided by Nicole Gaudelli and Benjamin Kleinstiver (Addgene plasmid # 136300, #138340, #139998 and #140005, respectively)(Gaudelli et al., 2020; Walton et al., 2020). The SpCas9 D10A nickase was subcloned from the ABE8.20-m plasmid. The SpCas9 was obtained by subcloning the SpCas9 D10A nickase from the ABE8.20-m plasmid by correcting the D10A mutation and replacing the Alanine (A10) with aspartic acid (D10). The editors were cloned into a plasmid containing a dT7 promoter followed by a 5' untranslated region (UTR), Kozak sequence, the ABE sequence, and a 3' UTR (proprietary backbone plasmid by Trilink). The plasmid was used as a template for PCR (KAPA HiFi HotStart 2x Readymix; Roche) with a *forward* primer correcting the mutation in the T7 promotor and a reverse primer adding the 120 bp long poly-A. The PCR product was purified using DNA Clean & Concentrator-5 kit (Zymo Research), followed by the in vitro transcription using the HiScribe^{™} T7 High Yield RNA Synthesis Kit (New England Biolabs (NEB) according to the manufacturer's instructions with N1-methyl-pseudouridine (Trilink) instead of uridine and co-transcriptional capping with CleanCap AG (TriLink Biotechnologies) with 1 µg linear PCR product as a template. To remove template DNA, 70 µL Ambion nuclease-free water (Life Technology Corp.), 10 µL of 10X DNase I Buffer (NEB) and 2 µL of RNase-free DNase I (NEB) were added to 20 µL of IVT reaction and incubated at 37°C for 15 minutes The mRNA was purified using the Monarch^{®} RNA clean up kit (NEB) and the purity was checked using a 1.5 % agarose gel using (2X) RNA Loading dye (NEB) and ssRNA Ladder (NEB) after denaturation at 70°C for 10 min and placing the RNA on ice for 2 min. The mRNA was quantified using Qubit 2.0 fluorometer (Invitrogen, Life Technologies) measurement with the Qubit^{™} RNA BR Assay kit (Invitrogen, Life Technologies) and stored at -80°C prior to use.

### Example 3: Design of DNA templates for homology-directed insertion of transgenes

DNA templates used for transgene integration via nuclease-induced homology-directed DNA repair (HDR) consist of a transgene to be integrated into the genome as well as flanking sequences homologous to the genomic DNA adjacent to the nuclease target site. The following HDR templates (HDRT) were previously described: HDRT for targeted insertion of:
(1) a 2nd generation CD19 CAR into the *TRAC* locus using Cas9 (Kath et al., 2022)
(2) a 2nd generation CD19 CAR into the *TRAC* locus using Cas12a (Glaser et al., 2023)
(3) a truncated 2nd generation CD19 CAR into the *CD3ζ* (*CD247*) gene using Cas9, enabling expression of a functional CAR protein by utilizing the endogenous *CD3ζ* sequence as the CAR's signaling domain. (Kath et al., 2024)
(4) HLA-E into the *B2M* gene to create a B2M-HLA-E fusion protein while simultaneously disrupting the expression of free B2M protein. (McCallion et al., 2023)

In some instances, the homology arms of HDRT for BEKI were modified to
(1.) prevent binding of the Cas9-based gene editor protein to the HDRT. This was achieved by incorporating point mutations (preferably in the PAM sequence adjacent to the gRNA target sequences).
(2.) ensure in-frame transgene insertion at the respective gRNA target sites (according to the specific positioning of the base-editor-induced DNA nicks). To do so, the left (upstream) homology arm comprised DNA sequences homologous to the genomic DNA upstream (left) of the upstream (left) gRNA target site, while the downstream (right) homology arm comprised DNA sequences homologous to the genomic DNA downstream (right) of the downstream (right) gRNA target site.

### Example 4: Generation of HDRT

Generation of BEKI HDRT from original *TRAC, CD3ζ*, and *B2M* HDRT-harboring plasmids was performed using multiple fragment In-Fusion cloning according to the manufacturer's protocol (Clontech, Takara). In-fusion cloning strategies were planned with SnapGene (Insightful Science; snapgene.com) before performing PCR reactions resulting in fragments with 15bp overlap PCR fragments (Kapa Hotstart HiFi Polymerase Readymix, Roche). In-fusion reactions were performed in 5 µL reactions at the recommended volume ratios. 2.5µL of In-Fusion reaction mixtures was transformed into 10 µL of Stellar Competent E. coli, plated on ampicillin-containing (LB) broth agar plates, and incubated at 37°C overnight. After performing colony PCR for size validation with universal primers adjacent to the pUC19 insertion site, 5 mL ampicillin-containing LB medium was inoculated with the selected clones and incubated at 37°C overnight. Plasmids were purified using ZymoPURE Plasmid Mini-Prep Kit (Zymo Research). Sequence validation of HDR-donor-template-containing plasmids was performed by Sanger Sequencing (LGC Genomics, Berlin) or whole plasmid sequencing (Eurofins).

### Example 5: HDR-templates with Cas target sequences

dsDNA HDRT without or with tCTS sequences were generated as previously described (Kath et al., 2022). In brief, the HDRT was amplified from the plasmid by PCR using the KAPA HiFi HotStart 2x Readymix (Roche) with a reaction volume of 500 µL. PCR products were purified and concentrated using paramagnetic beads (AMPure XP, Beckman Coulter Genomics), including two washing steps in 70% ethanol. HDRT concentrations were quantified using the Nanodrop ND-1000 spectrophotometer (Thermo Fisher Scientific). For the generation of ssDNA templates via enzymatic digestion (Nitulescu et al., 2024), the "Guide-it Long ssDNA Production System v2" kit was used according to the manufacturer's instructions (Takara Bio). In this process, the PCR products were amplified using a phosphorylated forward primer and a non-phosphorylated reverse primer. After ssDNA production, templates were purified and concentrated, similar to the dsDNA cleanup, using AMPure XP beads. For the generation of double-stranded CTS ends on the ssDNA, complementary oligos were added at a 6:1 molar ratio of oligos to nucleic acid. For the annealing, the mixed solutions were incubated at 94°C for 2 minutes, followed by 70°C for 1 minute, and finally room temperature for 15 minutes.

### Example 6: Gene editing

*T cells*: Primary human T cells were harvested at approximately 48 hours after anti-CD3/CD28 stimulation and washed twice in sterile PBS by centrifugation with 100xg for 10 min at room temperature and resuspended in P3 nucleofection solution (Lonza) at a concentration of 1.0-1.5x106 cells / 20µl.

*gRNA:* Synthesis of synthetic, modified gRNA (sgRNA for Cas9; crRNA for Cas12a) was commissioned at Integrated DNA Technologies (IDT). Upon arrival, they were resuspended to 100µM (in TE buffer), aliquoted, and stored at -20°C prior to use.

*Ribonucleoproteins (RNP) formulation:* RNP were formulated by thoroughly mixing (1) the negatively charged polymer poly-(L)-glutamic acid (PGA), (2) the gRNA, and (3) recombinant Cas protein followed by 15min incubation at room temperature. Per electroporation in 20µl vessels, the following concentrations and volumes were used: 0.5 µL of an aqueous solution of 15- to 50-kDa poly(L-glutamic acid) (PGA) (Sigma-Aldrich, 100 µg/µL), 0.48 µL of modified sgRNA (SpCas9) or modified crRNA (AsCas12a) (Integrated DNA Technologies (IDT), 100µM) and 0.4 µL recombinant SpCas9 protein (Alt-R S.p. Cas9 Nuclease V3; IDT; 10 µg/µL = 61 µ M) orAsCas12a (Alt-R A.s.Cas12a (Cpf1) Ultra; IDT; 10 µg/µL = 63 µM). The molar ratio of Cas9/Cas12a and sgRNA was ~ 1:2.

*Gene editing with recombinant Cas protein:* For RNP-mediated gene editing, 20µl of P3-resuspended T cells were mixed with 1.38µl RNP (per gRNA). For KI conditions, 0.5-2.0µl HDRT (0.5-2µg/µl) was added to the RNP prior to mixing with the cells.

*Gene editing with mRNA-encoded gene editors:* Modified mRNA (2 µg, unless stated otherwise) for *in cellulo* translation of gene editors (S.p.Cas9, S.p. Cas9-nickase (D10A mutant), ABE (8.20-m), CBE (BE4)) was mixed with the respective gRNAs. For ABE- or CBE-mediated knock-in, two sgRNAs were combined, one targeting the sense and the other one targeting the antisense strand of the genomic DNA. For KI experiments, 0.5-2.0µl HDRT (conc.= 0.5-2µg/µl) was added to the mixture. Some conditions involved the simultaneous use of recombinant Cas protein and mRNA-encoded editors. In this case, the reagents were additively combined at the stated volumes/concentrations. Prior to electroporation, 20µl of P3-resuspended T cells were added to the mixture.

*Transfection:* Prior to transfection, the strips or cartridges were gently tapped onto the bench several times to ensure the placement of the liquid on the bottom of the electroporation vessel without any trapped air (bubbles). Transfection of gene editing reagents was performed via electroporation with a Lonza 4D nucleofection device, preferably using the program EH-115. Electroporation was carried out either in 20µl of 100µl reaction units (16-well strip or cartridge, respectively). Immediately after transfection, 5 volumes of pre-warmed T cell medium were added to the cells. The cells were rested for 10 min at 37°C and 5% CO2. Afterward, the cells were transferred to culture plates with a pre-warmed T cell medium. In some experiments, the culture medium was supplemented with DNA-repair modifying ('HDR-enhancing') chemical compounds. The following chemicals were used (final concentration stated): Alt-R HDR-Enhancer V2 (0.5µM, IDT), Nedisertib (1µM, MedChemExpress), AZD7648 (1µM, MedChemExpress), AZD7648 (1µM, MedChemExpress) + Novobiocin (1µM, MedChemExpress), or AZD7648 (1µM, MedChemExpress) + ART588 (3µM, GIpBio), respectively. Half of the medium was replaced with fresh medium the next day to dilute the HDR-enhancing compounds.

### Example 7: Flow cytometry

Assessment of CAR+ / HLA-E KI rate and intracellular cytokine production was performed on a Cytoflex LX device (Beckman Coulter) using Nunc^{™} MicroWell^{™} 96-well U-bottom plates (Thermo Fisher Scientific) for other measurements. The following panels were used:

| | | | | | |
|---|---|---|---|---|---|
| **Knock-in Check / CAR MFI Panel (CD19-CAR; *TRAC*/*CD3ζ*)** | **Name** | **Dilution** | **Clone** | **Catalog number** | **Brand** |
| | LIVE/DEAD Fixable Blue | 1:100 | - | L23105 | Invitrogen |
| | Fc AF647 | 1:50 | polyclonal | 109-605-098 | Jackson ImmunoResearch |
| | CD3 PacBlue | 1:25 | UCHT1 | A93687 | Beckman Coulter |

| | | | | | |
|---|---|---|---|---|---|
| **HLA-E KI B2M + CIITA KO** | **Name** | **Dilution** | **Clone** | **Catalog number** | **Brand** |
| | LIVE/DEAD Fixable Blue | 1:100 | - | L23105 | Invitrogen |
| | HLA-E APC | 1:25 | 3D12 | 342606 | Biolegend |
| | HLA-A,B,C PE-Cy7 | 1:100 | W6/32 | 311429 | Biolegend |
| | HLA-DR,DP,DQ FITC | 1:200 | Tü39 | 361705 | Biolegend |

| | | | | | |
|---|---|---|---|---|---|
| | **Name** | **Dilution** | **Clone** | **Catalog number** | **Brand** |
| | LIVE/DEAD Fixable Blue | 1:100 | - | L23105 | Invitrogen |
| | Fc AF647 | 1:50 | polyclonal | 109-605-098 | Jackson ImmunoResearch |
| **Intracellular Cytokine Panel** | CD3 PacBlue | 1:25 | UCHT1 | A93687 | Beckman Coulter |
| | CD4 PE | 1:25 | 13B8.2 | A07751 | Beckman Coulter |
| | CD8 BV510 | 1:100 | SK1 | 344732 | Blolegend |
| | TNFa AF700 | 1:200 | MAb11 | 557996 | BD Biosciences |
| | IFNy APC-eF780 | 1:100 | 4S.B3 | 47-7319-42 | Ebioscience |
| | IL-2 PE-Cy7 | 1:25 | MQ1-17H12 | 500326 | Biolegend |

Surface staining was performed as previously described (Kath et al., 2022). Due to the potential cross-reactivity of the anti-Fc antibody, which we used for CAR staining, a first extracellular staining step with anti-Fc antibody and a live-dead discriminating dye followed by two washing steps was performed prior to staining with anti-CD3 PacBlue or anti-HLA-A, B, C PE-Cy7 and anti-HLA-DR, DQ, DP FITC.

### Example 8: Intracellular cytokine detection

CD19-CAR T cells were co-cultured in a round-bottom 96-well plate (Sarstedt) either with the CD19+ target cell lines Nalm-6. Prior to the assay, target cells were labeled with carboxyfluorescein diacetate succinimidyl ester (CFDA-SE, Thermo Fisher Scientific). 1 hour after the start of the co-culture, Brefeldin A (Sigma Aldrich) was added (at 10 µg/mL). After 5 hours, cells were washed in PBS and stained with fixable blue dead cell stain dye to discriminate dead cells. After another washing step, the cells were fixed and permeabilized using the Intracellular Fixation & Permeabilization Buffer Set (Thermo Fisher Scientific) prior to intracellular staining.

### Example 9: Digital droplet polymerase chain reaction (ddPCR) for translocation quantification

To demonstrate that BEKI can be used to simultaneously integrate cargo and edit other sites without significant increases in translocations, exemplary ddPCR assays were designed for balanced translocations between the *TRAC, RASA2,* or *FKBP12* genes. Probes were designed to bind to *RASA2* or *FKBP12* in order to prevent background from binding to the homology arms of the DNA template, as previously observed (Glaser et al., 2023). Genomic DNA was isolated on day 14 after electroporation using the Quick-DNA Miniprep Plus Kit (Zymo Research). A ddPCR assay for the RPP30 gene was used as a reference (Oscorbin et al., 2019). 2.5 µL of gDNA (30 ng/µL) was used as a template for a 22 µL PCR mix with 1.25 µl (10µM) of the forward and reverse primers for both the target and reference genes, 1.25 µL (5µM) of target and reference probe, 11µL of 2X ddPCR Supermix for Probes (No dUTP), 0.88 µL DMSO (total 4 %) 0.5 µL Hindlll-HF (NEB) and 5.12 µL nuclease-free water. Droplet generation was performed with the QX200 Droplet Generator, using 20 µL of the PCR mix and 70 µL Droplet Generation Oil inside a DG8 Cartridge covered by a DG8^{™} Gaskets for QX200^{™}/QX100^{™} Droplet Generator. Empty wells were filled with ddPCR Buffer Control. After droplet generation, droplets were gently pipetted into a 96-well PCR plate and sealed using the PX1^{™} PCR Plate Sealer and pierceable foil heat seal for 5s at 185°C. The enzymatic digestion and PCR were performed according to the following program:

| | |
|---|---|
| 37°C | 15 min |
| 95°C | 10 min |
| 94°C | 30 sec |
| 64°C | 1 min 45 |
| 72°C | 3 min |
| 98°C | 10 min |
| 12°C | hold |

ddPCR reaction with samples with less than 15,000 accepted droplets or more than 99% positive droplets for the reference gene were excluded from the analysis. Primers and probes were ordered from IDT, and all ddPCR-specific devices and consumables were acquired from BioRad.

### Example 10: Sanger sequencing - indel and base editing quantification

Genomic DNA was isolated on day 14 after electroporation using the Quick-DNA Miniprep Plus Kit (Zymo Research). Primers were designed to PCR amplify a 500-700 bp fragment of the *RASA2* or *FKBP12* locus from gDNA using COSMID (Cradick et al., 2014). The specificity of the primers was checked *in silico* using primer-Blast (Ye et al., 2012) and on a 1.5% agarose gel. The fragment was purified using the DNA Clean & Concentrator-5 kit (Zymo Research). Sanger sequencing was performed (LGC Genomics), the frequency of insertions and deletions (indel) was determined using ICE analysis (Conant et al., 2022), and the base editing efficiency was quantified using EditR (Kluesner et al., 2018).

### Example 11: Manufacturing of alloreactive T cells

PBMCs were CD8 MACS enriched according to the manufacturer's instructions. 10⁷ obtained CD8+ T cells were mixed in a 1:1 ratio with irradiated PBMCs. After one day, the medium was changed to IL-7 and IL-15 containing T cell medium. The alloreactive T cells were restimulated with 10⁷ freshly irradiated PBMCs on day 7 and cultured for one more week.

### Example 12: Allo-specific cytotoxicity assay set-up

CFSE labeled effector cells were cocultured with different target cells in a ratio of 2:1, 1:1, 0,5:1 and 0:1. HLA-edited target cells were genetically modified two weeks prior to the assay set-up. After 16 hours 100 µl of the cell suspension was resuspended in DAPI-PBS and number of target cells was acquired.

### Example 13: Data analysis, statistics, and presentation

Flow cytometry data were analyzed with FlowJo software v.10 (BD Biosciences). Graphs and statistical analyses were created using Prism 10 (GraphPad). Schemes and graphs in the presented figures were created using www.biorender.com.

### Example 14: Combination of two sgRNAs and a nickase Cas9 base editor enables knock-in of transgenes.

Non-viral CRISPR-Cas9 mediated generation of CAR T cells commonly works by introducing a DNA double-strand break (DSB) into an early exon of the *TRAC* locus followed by homology-directed repair (HDR) using a DNA template for the integration of a full-length CAR (Fig. 1 a left). In contrast, the BEKI method utilizes a nickase Cas9 (nCas9)- base editor with two sgRNAs to target opposing strands of the DNA and introduces single-strand DNA breaks (SSBs) to allow HDR mediated transgene insertion (Fig. 1A right). Cas9 and BEKI-generated CAR T cells express the CAR under the control of the endogenous *TRAC* promotor, while the resulting *TRAC* KO leads to cells deficient of the TCR/CD3 complex (Fig.1 B).

### Example 15: Base editor knock-ins require distinct sgRNA designs for efficient editing with both adenine base editors and cytosine base editors.

We hypothesized that careful selection of the sense and antisense strand targeting sgRNA combination would be crucial for efficient transgene integration during BEKI. In contrast to conventional nickase approaches, base editors may prevent efficient transgene integration by forcing base conversions that (a) reduce the frequency of SSB occurrence by preventing repetitive nCas9-base editor binding (due to forced mismatches between sgRNA and the target sequence) and (b) prevent homology-mediated DNA repair by introducing mismatches to the HDR template. To evaluate the sgRNA design rules for BEKI, we screened sgRNA combinations for the orientation of the PAM (in vs. out), the distance between the sgRNA-base editor forced SSBs, and different numbers of potential target bases in the editing window. For the latter, we hypothesized that converting bases within the editing window should generally reduce knock-in efficacy related to the problem (a). Further, we evaluated different HDR templates for different combinations, designing the DNA templates that would "delete" parts of the sequence between the individual SSBs. In line with previous results, nCas9 can be used for efficient double-nick mediated KI when a 'PAM out' orientation is chosen, which results in 5' overhangs. Most efficient editing was observed for sgRNAs resulting in SSBs with a spatial separation of 35 bp to 170 bp (Fig.1 C, D, E). Compared to nCas9 mediated KI, fewer sgRNA combinations yielded highly efficient ABE BEKI rates as potential base changes of adenines in the editing window could reduce retargeting of the genomic DNA (gDNA) and thus limit the BEKI efficiency (Fig1. F, G, H).

Interestingly, for combinations with 4 or more adenines within the editing window of the two sgRNAs, the KI efficiency was reduced compared to nCas9 editing. Nonetheless, combinations with up to 4 adenines within the editing window of the two sgRNAs were well tolerated for highly efficient integration of a CAR into the *TRAC* locus (Fig1. F, G, H). For CBE BEKI, different sgRNA combinations resulted in efficient HDR, likely due to the distinct pattern of potential cytosine edits (Fig1. I, J, K).

### Example 16: BEKI can be designed for knock-in of different transgenes and target sites and can be optimized to provide simultaneous knock-out of the endogenous gene at the integration locus.

BEKI can reprogram for transgene insertion into different loci, such as *CD3ζ* or *B2M,* showcasing its versatility (Fig.2 A, B). Screening multiple sense and antisense targeting sgRNA combinations revealed promising candidates for efficient KI, but also simultaneously KO for both *CD3ζ* and *B2M* while avoiding sgRNA designs with 'PAM in' orientation or distanced below 30 bp (Fig. 2 C, D, E, F, G, H). Using sgRNA B2 that targets a slice site in the *B2M* gene resulted in high KO frequencies but, interestingly, also enabled efficient BEKI when combined with another sgRNA targeting the antisense strand (Fig. 2 G, H). Retargeting the base editing outcome of sgRNA B2 with sgRNA B3 led to a slight increase in KI frequency when combined with another sgRNA targeting the antisense strand (Fig. 2 G, H). Taken together, the BEKI data for 3 loci *(TRAC, CD3ζ*, and *B2M*) highlight the ideal distance of 30 bp and 250 bp between the sense and antisense strand targeting sgRNAs while emphasizing that more than 4 target bases within the editing window should be avoided.

### Example 17: BEKI rates are increased by exposing cells to DNA repair modulating agents that inhibit non-homologous end joining and microhomology-mediated end joining.

The initially observed BEKI efficiency can be enhanced through inhibitors of DNA-PK (necessary for non-homologous end joining) and PolQ (associated with microhomology-mediated end joining). For BEKI of a truncated CAR into *CD3ζ*, improved HDR rates were detected when using pharmaceutical enhancers like AZD7648 (a DNA-PK inhibitor) and novobiocin (a PolQ inhibitor) (Fig.3 A). The commercially available HDR enhancer V2 (Integrated DNA Technologies Inc) (a NHEJ inhibitor) enhanced BEKI efficiencies of CAR into *CD3ζ* and HLA-E into *B2M* without changing the choice of the most efficient combinations (Fig. 3 B, C, D). The most efficient sgRNA combinations for BEKI at 3 loci *(TRAC, CD3ζ*, and *B2M*) enhanced the gene editing efficiency by 2.7-fold when combining DNA-PK (AZD7648) and PolQ (ART558) inhibitors (Fig. 3 E).

### Example 18: BEKI rates can be enhanced by modification of the non-viral templates with Cas-target sequence motifs.

Cas Target Sequences were previously proposed to enhance knock-in efficacies with different non-viral templates (linear double-stranded DNA: Nguyen et al. 2020 1, plasmid: Jing et al. 2021 2, single-stranded DNA: Shy et al. 2022 3) by enhancing nuclear transport of the template through RNA-reprogrammed Cas-nucleases fused to nuclear localization sequences (NLS). We hypothesized that CTS modification may also enhance BEKI rates due to the presence of NLS on most base editor proteins. To evaluate the compatibility of BEKI with CTS-modified DNA templates, we created double-stranded DNA templates with CTS (dsCTS) and single-stranded DNA with CTS (ssCTS) (Fig. 4 A). DsCTS for CAR into *TRAC* BEKI at low doses and ssCTS at high dose levels show enhanced transgene insertion rated compared to conventional dsDNA templates and can be improved using DNA-PK and PolQ inhibitors (Fig. 4 B, C). In summary, incorporating CTS into the HDR templates improves KI efficiency, particularly when combined with DNA-PK and PolQ inhibitors.

### Example 19: BEKI can be combined with additional base edits to force gene knock-outs or to induce gain-of-function or loss-of-function mutations alongside transgene integration.

Transfecting a base editing enzyme into cells offers the opportunity to facilitate additional single base modifications by adding sgRNAs to redirect the base editor to another target site. Selection of sgRNAs targeting splice donor or acceptor sites can mediate efficient knock-out (KO) of target genes, such as *FKBP12,* surpassing efficiencies observed after conventional Cas9-mediated KO (Fig. 5 A). T cells carrying *FKBP12KO* showed improved cytokine production (IFNγ, TNFα) upon stimulation in the presence of the calcineurin inhibitor Tacrolimus, while *RASA2* KO enhanced the cytokine secretion compared to unedited mock T cells (Fig.5 B, C, D). Three KI strategies (Cas9, Cas12a + ABE, and ABE BEKI) were then used to simultaneously integrate a CD19 CAR transgene and knock out *FKBP12* and RASA2 efficiently to improve cytokine production in the presence of Tacrolimus after CD19+ Nalm6 tumor cell challenge (Fig. 5 E, F).

### Example 20: Combining BEKI with additional simultaneous edits in a single transfection prevents translocations.

Multiplexed knock-in and KO using BEKI resulted in fewer balanced chromosomal translocations between the 3 edited loci when using Cas12a + ABE or BEKI compared to compared to conventional Cas9 mediated multiplexed editing (Fig.6 A, B, C). This indicates that BEKI has a reduced risk for creating translocations, emphasizing its potential for safer therapeutic applications involving targeting multiple genes in a single intervention.

### Example 21: Quintuple BEKI edited CART cells resist allo-specific immune rejection.

BEKI enables efficient multiplex editing for allogeneic off-the-shelf approaches and accommodates editing at 5 locations (or more) simultaneously with high editing degree. BEKI and editing of *B2M* and *CIITA* eliminates HLA-class 1 and -class 2 expression efficiently, which protects T cells from allo-specific T cell lysis (Fig. 7 A, B, C). Editing of HLA for allo-protection can also be combined with other enhancing KOs, such as *FKBP12* and *RASA2,* without loss in editing efficacy. (Fig. 7. B).

### Example 22: BEKI can be used to integrate two transgenes at different loci simultaneously while reducing translocations between the different integration sites.

The simultaneous double knock-in of a truncCAR into *CD3ζ* and HLA-E into *B2M* using BEKI, Cas9, and Cas12a systems is feasible and can be enhanced by DNA-PK and PolQ inhibitors, with BEKI proving more effective than Cas9 in generating cells that carry both transgenes (Fig.8 A, B).

## Claims

1. An *in vitro* method for modifying double stranded DNA (dsDNA) in a eukaryotic cell, the method comprising:
a) introducing a base editor system into the cell, said system comprising
i. a base editor, comprising an RNA guided DNA nickase linked to a single-stranded DNA nucleobase modifying enzyme, or a nucleic acid encoding said base editor, and
ii. at least two guide RNA (sgRNA) molecules, comprising a first sgRNA that hybridizes to a first target sequence in the dsDNA, and a second sgRNA that hybridizes to a second target sequence in an opposing strand of the dsDNA,
b) generating at least two single-strand breaks (SSBs), in opposing strands, of the dsDNA,
iii. wherein a first base editor complex, comprising the first sgRNA, introduces a nick cleavage of one strand of the dsDNA in the first target sequence, and a second base editor complex, comprising the second sgRNA, introduces a nick cleavage of the opposite strand of the dsDNA in the second target sequence, thereby producing a 5' or 3' overhang,
c) inserting an exogenous DNA repair template sequence in proximity to the two single strand breaks (SSBs).

2. The method according to claim 1, wherein the exogenous DNA repair template sequence encodes a recombinant antigen-specific targeting construct, such as a chimeric antigen receptor (CAR) or T cell receptor (TCR), or a construct configured to enhance the persistence and/or potency of cells for therapeutic effects.

3. The method according to any one of the preceding claims, wherein the eukaryotic cell is an immune cell and/or an effector cell, such as a Treg, or a cytotoxic immune effector cell, preferably a T cell, or NK cell.

4. The method according to the preceding claim, wherein the eukaryotic cell is a T or NK cell and the first and second target sequences of the dsDNA are in an endogenous genomic dsDNA encoding a T cell receptor component, such as in the *TRAC* locus, *CD3ζ* locus or *CD3ε* locus, or a major histocompatibility complex component, such as in the *B2M* locus.

5. The method according to any one of the preceding claims, wherein the exogenous DNA repair template sequence comprises at least one sequence (homology arm) of at least 45 bp, preferably 400 bp, with at least 90%, preferably 95%, more preferably 100%, sequence identity to a region of the dsDNA sequence, wherein said homology arm is positioned to hybridize to the dsDNA in proximity to the two single strand breaks (SSBs).

6. The method according to any one of the preceding claims, comprising in step a) introducing one or more additional sgRNA sequences, comprising a third sgRNA that hybridizes to a third target sequence in the dsDNA, and forming a third base editor complex, that introduces a nick cleavage of one strand of the dsDNA and a nucleobase modification in the third target sequence.

7. The method according to the preceding claim, wherein:
the third target sequence of the dsDNA is a component of a gene, such as a regulatory sequence, splice donor, splice acceptor or other sequence associated with expression of said gene, and wherein said modification of the third target sequence leads to disruption of expression of said gene, and/or
wherein the third target sequence of the dsDNA is located in the open reading frame (ORF) of a gene, associated with a functional domain of said gene, and wherein said modification of the third target sequence leads to an amino acid change leading to a functional alteration of said gene.

8. The method according to the preceding claim, wherein the expression of multiple genes is disrupted (multiple gene knockouts) with multiple additional sgRNA sequences, simultaneously with the insertion of an exogenous DNA repair template sequence.

9. The method according to any one of the preceding claims, wherein the single-stranded DNA nucleobase modifying enzyme is an adenosine deaminase, a cytidine deaminase, cytosine-DNA glycolase, thymidine-DNA glycolase or fusion constructs containing deaminases and glycolases, such as a cytidine deaminase with a uracil glycosylase.

10. The method according to any one of the preceding claims, wherein the base editor is a Cytosine Base Editor (CBE) and/or Adenine Base Editor (ABE) and/or a Glycosylase Base Editor (GBE).

11. The method according to any one of the preceding claims, wherein:
the base editor is an ABE, and the first and/or second sgRNA lacks adenine bases in the editing window and/or wherein the base editor is a CBE, and the first and/or second sgRNA lacks cytosine bases in the editing window, or
the base editor is an ABE, and the first and second sgRNA together have a total of less than 6 adenine bases in the editing window and/or wherein the base editor is a CBE, and the first and second sgRNA together have a total of less than 6 cytosine bases in the editing window.

12. The method according to any one of the preceding claims, comprising introducing to said cell one or more DNA repair modulators and/or contacting said cell with one or more DNA-dependent protein kinase PK (DNA-PK) inhibitors or DNA PolQ inhibitors.

13. The method according to any one of the preceding claims, wherein:
the distance between the two single strand breaks on the dsDNA is more than 29 bases, and/or
the distance between the two single strand breaks on the dsDNA is less than 250 bases, preferably less than 200 bases.

14. A genetically modified eukaryotic cell obtained by the method according to any one of the preceding claims.

15. The genetically modified eukaryotic cell according to the preceding claim, comprising at least one insertion of an exogenous DNA donor template sequence and at least one base edit in the dsDNA produced by the base editor system in proximity to said exogenous DNA donor template sequence.

16. A kit for use in the *in vitro* method for modifying double stranded DNA (dsDNA) according to any one of claims 1 to 16, the kit comprising:
a. a base editor system, said system comprising
i. a base editor, comprising an RNA guided DNA nickase linked to a single-stranded DNA nucleobase modifying enzyme, or a nucleic acid encoding said base editor, and
ii. at least two guide RNA (sgRNA) sequences, comprising a first sgRNA that hybridizes to a first target sequence in the dsDNA, and a second sgRNA that hybridizes to a second target sequence in the dsDNA,
wherein a first base editor complex, comprising the first sgRNA, is configured to introduce a nick cleavage of one strand of the dsDNA in the first target sequence, and a second base editor complex, comprising the second sgRNA, is configured to introduce a nick cleavage of the opposite strand of the dsDNA in the second target sequence, thereby producing a 5' or 3' overhang,
and
b. an exogenous DNA repair template that hybridizes to the dsDNA in proximity to one or both single strand breaks (SSBs).
c. and optionally one or more DNA repair modulators.
